# EUROPEAN PATENT SPECIFICATION

(11) **EP 2 744 903 B1**
(45) Date of publication and mention of the grant of the patent: **10.10.2018**
(21) Application number: 12825119.6
(22) Date of filing: 20.08.2012
(51) Int. Cl.: C12N 1/19, C12P 21/08, C12N 1/38, C12N 15/12, C07K 16/00, C12P 21/00, C12N 15/63, C12N 1/16, C12N 15/81, C12P 21/02

(54) **MULTI-COPY STRATEGY FOR HIGH-TITER AND HIGH-PURITY PRODUCTION OF MULTI-SUBUNIT PROTEINS SUCH AS A ANTIBODIES IN TRANSFORMED MICROBES SUCH AS PICHIA PASTORIS**
MEHRFACHKOPIERSTRATEGIE ZUR HERSTELLUNG VON HOCHREINEN UND HOCHTITRIERTEN PROTEINEN MIT MEHREREN UNTEREINHEITEN WIE ANTIKÖRPERN IN TRANSFORMIERTEN MIKROBEN, Z. B. PICHIA PASTORIS
STRATÉGIE À COPIES MULTIPLES POUR UNE PRODUCTION À TITRE ÉLEVÉ ET À PURETÉ ÉLEVÉE DE PROTÉINES À MULTIPLES SOUS-UNITÉS, TELLES QUE DES ANTICORPSA DANS DES MICROBES TRANSFORMÉS TELS QUE PICHIA PASTORIS

(30) Priority: 19.08.2011 US 201161525307 P; 08.05.2012 US 201213466795
(43) Date of publication of application: 25.06.2014
(73) Proprietor: AlderBio Holdings LLC, Las Vegas, NV 89109 (US)
(72) Inventor: MITCHELL, Danielle, Marie, Seattle, Washington 98115 (US); GARCIA-MARTINEZ, Leon, F., Woodinville, Washington 98072 (US); MCNEILL, Patricia, Dianne, Federal Way, Washington 98003 (US); OJALA, Ethan, Wayne, Snohomish, Washington 98290 (US); INAN, Mehmet, 07070 Antalya (TR); LATHAM, John, Seattle, Washington 98119 (US)
(74) Representative: Stephens, Clare Jean
(86) International application number: PCT/US2012/051619
(87) International publication number: WO 2013/028635

(56) References cited:
- WO-A2-2008/063302
- US-A1- 2001 036 647
- US-A1- 2003 027 213
- US-A1- 2006 270 045
- US-A1- 2009 028 784
- SCHLATTER S ET AL: "On the optimal ratio of heavy to light chain genes for efficient recombinant antibody production by CHO cells", BIOTECHNOLOGY PROGRESS, AMERICAN INSTITUTE OF CHEMICAL ENGINEERS, US, vol. 21, no. 1, 1 January 2005 (2005-01-01), pages 122-133, XP008123445, ISSN: 8756-7938, DOI: 10.1021/BP049780W [retrieved on 2004-11-16]
- JIANG ZHOU ET AL: "Regulation of recombinant monoclonal antibody production in Chinese hamster ovary cells: A comparative study of gene copy number, mRNA level, and protein expression", BIOTECHNOLOGY PROGRESS, AMERICAN INSTITUTE OF CHEMICAL ENGINEERS, US, vol. 22, no. 1, 1 January 2006 (2006-01-01), pages 313-318, XP002521023, ISSN: 8756-7938, DOI: 10.1021/BP0501524 [retrieved on 2005-11-15]
- REISINGER H ET AL: "The absence of effect of gene copy number and mRNA level on the amount of mAb secretion from mammalian cells", APPLIED MICROBIOLOGY AND BIOTECHNOLOGY, SPRINGER, DE, vol. 81, no. 4, 23 September 2008 (2008-09-23), pages 701-710, XP002525685, ISSN: 0175-7598, DOI: 10.1007/S00253-008-1701-1 [retrieved on 2008-09-23]
- CHUSAINOW JANET ET AL: "A study of monoclonal antibody-producing CHO cell lines: what makes a stable high producer?", BIOTECHNOLOGY AND BIOENGINEERING, WILEY & SONS, HOBOKEN, NJ, US, vol. 102, no. 4, 8 October 2008 (2008-10-08), pages 1182-1196, XP002525682, ISSN: 0006-3592, DOI: 10.1002/BIT.22158 [retrieved on 2008-10-08]
- WEAVER-FELDHAUS, J. M. ET AL.: 'Yeast mating for combinatorial Fab library generation and surface display' FEBS LETT. vol. 564, 23 March 2004, pages 24 - 34, XP004503587

## Description

### FIELD

The present disclosure generally relates to methods for producing heterologous proteins in transformed cells. In particular, the present disclosure provides improved methods of producing antibodies.

### BACKGROUND

Conventional antibodies are tetrameric proteins composed of two identical light chains and two identical heavy chains. Pure human antibodies of a specific type can be difficult or impossible to purify from natural sources in sufficient amounts for many purposes. As a consequence, biotechnology and pharmaceutical companies have turned to recombinant DNA-based methods to prepare antibodies on a large scale. The production of functional antibodies generally involves not just the synthesis of the two polypeptides but also a number of post-translational events, including proteolytic processing of the N-terminal secretion signal sequence; proper folding and assembly of the polypeptides into tetramers; formation of disulfide bonds; and typically includes a specific N-linked glycosylation. All of these events take place in the eukaryotic cell secretory pathway, an organelle complex unique to eukaryotic cells.

Recombinant synthesis of such complex proteins has typically relied on cultures of higher eukaryotic cells to produce biologically active material, with cultured mammalian cells being very commonly used. However, mammalian tissue culture-based production systems incur significant added expense and complication relative to microbial fermentation methods. Additionally, products derived from mammalian cell culture may require additional safety testing to ensure freedom from mammalian pathogens (including viruses) that might be present in the cultured cells or animal-derived products used in culture, such as serum.

Prior work has help to establish the yeast *Pichia pastoris* as a cost-effective platform for producing functional antibodies that are potentially suitable for research, diagnostic, and therapeutic use. *See* co-owned U.S. Patents 7,935,340 and 7,927,863. Methods are also known in the literature for design and optimization of *P. pastoris* fermentations for expression of recombinant proteins, including optimization of the cell density, broth volume, substrate feed rate, and the length of each phase of the reaction. *See* Zhang et al., "Rational Design and Optimization of Fed-Batch and Continuous Fermentations" in Cregg, J. M., Ed., 2007, Pichia Protocols (2nd edition), Methods in Molecular Biology, vol. 389, Humana Press, Totowa, N.J., pgs. 43-63.

Though recombinant multi-subunit proteins can be produced from cultured cells, undesired side-products may also be produced. For example, the cultured cells may produce the desired multi-subunit protein along with free monomers, complexes having incorrect stoichiometry, or proteins having undesired or aberrant glycosylation. Purification of the desired multi-subunit protein can increase production cost, and the steps involved in purification may decrease total yield of active complexes. Moreover, even after purification, undesired side-products may be present in amounts that cause concern. For example, glycosylated side-products may be present in amounts that increase the risk of an immune reaction after administration, while aberrant complexes or aggregates may decrease specific activity and may also be potentially immunogenic.

Schlatter et al. (Biotechnology Progress, vol. 21, no. 1,1 January 2005, pages 122-133) discusses the optimal ratio of heavy to light chain genes for efficient recombinant antibody production by CHO cells. Jiang et al. (Biotechnology Progress, vol. 22, no. 1,1 January 2006, pages 313-318) discloses regulation of recombinant monoclonal antibody production in Chinese hamster ovary cells: a comparative study of gene copy number, mRNA level, and protein expression. Reisinger et al. (Applied Microbiology and Biotechnology, vol. 81, no. 4, 23 September 2008, pages 701-710) discloses the absence of effect of gene copy number and mRNA level on the amount of mAb secretion from mammalian cells. Chusainow et al. (Biotechnology and Bioengineering, vol. 102, no. 4, 8 October 2008, pages 1182-1196) discusses what makes a stable high producer in a study of monoclonal antibody-producing CHO cell lines.
WO 2008/063302 A2 discloses novel *P. pastoris pastoris* promoters, and the use thereof to direct expression of proteins in yeast, preferably using a haploid mating strategy. US 2006/270045 A1 discloses methods of synthesizing heteromultimeric polypeptides in yeast using a haploid mating strategy. Weaver-Feldhaus et al. (FEBS Lett., vol. 564, 23 March 2004, pages 24-34) discloses yeast mating for combinatorial Fab library generation and surface display. US 2001/036647 A1 discloses functionally assembled antigen-specific intact recombinant antibody and a method for production thereof.

### SUMMARY

The invention is defined in the appended claims and any other aspects or embodiments set forth herein are for information only.

The present invention provides a method of identifying a diploid *Pichia pastoris* host cell that produces a desired antibody with a greater purity than another diploid *Pichia pastoris* host cell, said method comprising: (a) providing a panel of diploid *Pichia pastoris* host cells, said panel comprising at least two diploid *Pichia pastoris* host cells that each comprise differing numbers of copies of one or more of the genes that provide for the expression of the subunits of the desired antibody, wherein said genes that provide for the expression of the subunits of the desired antibody comprise genes encoding the light chain and the heavy chain of the desired antibody; (b) culturing each of said diploid *Pichia pastoris* host cells to express the desired antibody; (c) measuring the purity of the desired antibody produced by each said diploid *Pichia pastoris* host cell; and (d) identifying a diploid *Pichia pastoris* host cell that produces said desired antibody with a greater purity than another diploid *Pichia pastoris* host cell in said panel of diploid *Pichia pastoris* host cells that produce the desired antibody.

The present disclosure provides improved methods and compositions of matter that provide for the recombinant production of multi-subunit proteins such as antibodies, hormones and receptors and other multi-subunit complexes, with a higher yield. These multiunit polypeptides may comprise 2 or more subunits which may be the same or different (i.e., homo- or heteropolymeric polypeptides. In some examples, the secreted or intracellular yield of such multi-subunit proteins may be increased by at least 50%, by at least 100%, or more (relative to conventional methods) using the methods disclosed herein.

The present disclosure also provides improved methods and compositions of matter that provide for the recombinant production of antibodies and other multi-subunit proteins, with decreased production of undesired side-products. In some examples, the undesired side product may be a glycosylated protein, such as a glycosylated antibody heavy chain, whose relative abundance may be decreased by at least 10%, at least 20%, at least 30%, at least 40%, at least 50%, at least 60%, at least 70%, at least 80% , at least 90%, at least 95%, at least 96%, at least 97%, at least 98%, at least 99%, or down to undetectable levels compared to initial abundance levels (relative to conventional methods) using methods disclosed herein. Exemplary undesired side-products whose relative abundance may be so decreased may include one or more species having a different apparent molecular weight than the desired multi-subunit complex. For example, apparent molecular weight may be affected by differences in stoichiometry, folding, complex assembly, and/or glycosylation. For example, such undesired side products may be detected using size exclusion chromatography and/or gel electrophoresis, and may have a higher or lower apparent molecular weight than the desired multi-subunit complex. In some examples, the undesired side-products may be detected under reducing conditions. In other examples, the undesired side-products may be detected under non-reducing conditions.

In some examples, the present disclosure also provides improved methods and compositions of matter that provide for the recombinant production of antibodies and other multi-subunit complexes, with a higher yield. In some examples, the yield may be increased by at least 10%, at least 20%, at least 30%, at least 40%, at least 50%, at least 100%, or more (relative to conventional methods) using the methods disclosed herein.

In some examples, the host cell in which the multi-subunit proteins may be produced may be a yeast, for example in a *Pichia* species such as *P. pastoris* or another methylotrophic yeast, or in a *Saccharomyces* species such as *S. cerevisiae,* or another yeast such as a *Schizosaccharomyces* (e.g., *S. pombe).* Other examples of methylotrophic yeast which may be utilized in the present disclosure include *Pichia angusta* (also known in the art as *Hansenula polymorpha*), *Pichia guillermordii, Pichia methanolica, Pichia inositovera, Ogataea nitratoaversa, and Candida boidnii.*

The present disclosure provides improved methods of identifying a host cell that produces a desired antibody or other desired multi-subunit complex with a greater yield, which may comprise: (a) providing a panel of host cells, said panel comprising at least two host cells that each comprise genes that provide for expression of the subunits of said multi-subunit complex (e.g., the light chain and heavy chain of said desired antibody); (b) culturing each said host cell conditions that permit expression of the multi-subunit complex, wherein the genes in said at least two host cells provide for differing levels of expression of at least one subunit of said desired multi-subunit complex; (c) measuring the yield of the multi-subunit complex produced by each said host cell; and (d) identifying the host cell that produces a greater yield than another host cell in said panel of host cells as a host cell that produces a desired multi-subunit complex with a greater yield.

The present disclosure also provides improved methods of identifying a host cell that produces a desired antibody or other desired multi-subunit complex with a greater purity, which may comprise: (a) providing a panel of host cells, said panel comprising at least two host cells that each comprise genes that provide for expression of the subunits of said multi-subunit complex (e.g., the light chain and heavy chain of said desired antibody); (b) culturing each said host cell conditions that permit expression of the multi-subunit complex, wherein the genes in said at least two host cells provide for differing levels of at least one subunit of said desired multi-subunit complex; (c) measuring the purity of said multi-subunit complex produced by each said host cell; and (d) identifying the host cell that produces a greater purity than another host cell in said panel of host cells as a host cell that produces a desired multi-subunit complex with a greater purity.

The host cell may be a eukaryotic cell, such as a yeast cell, such as a methylotrophic yeast, such as a yeast of the genus Pichia. Exemplary methylotrophic yeasts of the genus Pichia include *Pichia pastoris, Pichia angusta, Pichia guillermordii, Pichia methanolica,* and *Pichia inositovera.* The host cell may be produced by mating, e.g., by mating two haploid yeast cells that each contain one or more copies of at least one gene encoding a subunit of the multi-subunit complex. For example, multiple haploid cells may be produced containing known, differing numbers of copies of one or more subunits of said multi-subunit complex, such that mating between different combinations of haploid cells can rapidly produce a panel of diploid cells, each containing pre-selected numbers of copies of the genes encoding each subunit of the multi-subunit complex. Additionally, multiple diploid cells may be produced containing known, differing numbers of copies of one or more subunits of said multi-subunit complex, such that mating between different combinations of diploid cells can rapidly produce a panel of tetraploid cells, each containing pre-selected numbers of copies of the genes encoding each subunit of the multi-subunit complex.

In one example, the methylotrophic yeasts of the genus Pichia is *Pichia pastoris.* The host cell may be a diploid or tetraploid cell.

At least one of said genes encoding said subunits of the desired multi-subunit complex, such as said desired antibody light chain and / or heavy chain, may be expressed under control of an inducible or constitutive promoter, such as CUP1 (induced by the level of copper in the medium; see Koller et al., Yeast 2000; 16: 651-656.), tetracycline inducible promoters (see, e.g., Staib et al., Antimicrobial Agents And Chemotherapy, Jan. 2008, p. 146-156), thiamine inducible promoters, AOX1, ICL1, glyceraldehyde-3-phosphate dehydrogenase (GAP), FLD1, ADH1, alcohol dehydrogenase II, GAL4, PHO3, PHO5, and Pyk promoters, chimeric promoters derived therefrom, yeast promoters, mammalian promoters, insect promoters, plant promoters, reptile promoters, amphibian promoters, viral promoters, and avian promoters.

At least one of said genes encoding said subunits of the desired multi-subunit complex, such as said desired antibody light chain and / or heavy chain, may be expressed under control of an inducible or constitutive promoter, such as CUP1, AOX1, ICL1, glyceraldehyde-3-phosphate dehydrogenase (GAP), FLD1, ADH1, alcohol dehydrogenase II, GAL4, PHO3, PHO5, and Pyk promoters, tetracycline inducible promoters, thiamine inducible promoters, chimeric promoters derived therefrom, yeast promoters, mammalian promoters, insect promoters, plant promoters, reptile promoters, amphibian promoters, viral promoters, and avian promoters.

The host cell may secrete said desired multi-subunit complex into the culture medium. Alternatively or in addition, said desired multi-subunit complex may be retained in said host cell and may be isolated therefrom.

Said host cell may be a diploid, tetraploid cell, or polyploid.

The method may further comprise purifying said multi-subunit complex from said host cells or from the culture medium.

Said multi-subunit complex may be purified from an intracellular component, cytoplasm, nucleoplasm, or a membrane of said host cells.

The desired multi-subunit complex may comprise an antibody, such as a monospecific or bispecific antibody. The antibody may be an antibody that specifically binds any antigen.

Said multi-subunit complex may comprise a human antibody or a humanized antibody or fragment thereof.

Said humanized antibody may be of mouse, rat, rabbit, goat, sheep, or cow origin.

Said humanized antibody may be of rabbit origin.

Said multi-subunit complex may comprise a monovalent, bivalent, or multivalent antibody.

Said antibody may be purified from said culture by protein A and/or protein G affinity.

At least one of the genes that provide for expression of a subunit of said multi-subunit complex in at least one of said eukaryotic cells in said panel may be optimized for expression in said eukaryotic cell.

The at least two host cells in said panel may comprise differing numbers of copies of a gene encoding the subunits of said multi-subunit complex, e.g., differing numbers of copies of a gene encoding a desired antibody heavy chain and / or said desired antibody light chain.

At least one host cell in said panel may comprise at least two copies of a gene encoding a subunit of said multi-subunit complex, e.g., at least two copies of a gene encoding a desired antibody heavy chain and / or said desired antibody light chain.

At least one host cell in said panel may comprise a gene encoding a subunit of said desired multi-subunit complex (such as a desired antibody heavy chain or a desired antibody light chain) whose expression may be driven by a different promoter than the promoter that drives the expression of the corresponding gene in a different host cell in said panel.

At least one host cell in said panel may comprise a polycistronic gene comprising more than one sequence encoding one or more subunits of said desired multi-subunit complex.

The desired multi-subunit complex may comprise a desired antibody, which may specifically bind to any antigen. Exemplary non-limiting examples include IL-6, TNF-alpha, CGRP, PCSK9, or NGF.

The desired multi-subunit complex may comprise an antibody of any type. Exemplary antibody types include antibodies of any mammalian species, e.g., human, mouse, rat, rabbit, goat, sheep, cow, etc. Preferably, the antibody is a human antibody or a humanized antibody that may be of rabbit origin. The desired antibody may be a monovalent, bivalent, or multivalent antibody.

At least one of said genes that provide for expression of a subunit of the desired multi-subunit complex, such as the light chain and/or heavy chain of a desired antibody, in at least one of said host cells in said panel may be optimized for expression in said host cell (e.g., by selecting preferred codons and/or altering the percentage AT through codon selection).

As shown in the working examples, in some examples, the yield and/or purity of the antibody is further optimized by the use of host cells for expression that have more copies of the heavy chain relative to the light chain, more copies of the light chain relative to the heavy chain, or equal numbers of copies of the light and heavy chains.

The purity of said desired multi-subunit complex, such as a desired antibody, may be assessed by measuring the fraction of the desired multi-subunit complex produced by said host cell that is non-glycosylated, is contained in complexes having the expected apparent hydrodynamic radius and/or apparent molecular weight (e.g., measured by size exclusion chromatography), has the expected electrophoretic mobility (e.g., detected by gel electrophoresis, such as SDS-PAGE, and optionally Western blotting), and / or by measuring the specific activity of the multi-subunit complex (e.g., specific binding a target of a desired antibody).

The desired multi-subunit complex may be an antibody, and yield of said antibody may be assessed by determining the amount of desired antibody produced by said host cell discounting any product-associated variants that are glycosylated, contained in antibody complexes other than complexes having the expected apparent molecular weight or hydrodynamic radius, and / or that fail to specifically bind to the target of said desired antibody.

The present disclosure also provides a method of recombinantly producing a desired multi-subunit complex, such as a desired antibody, comprising: (a) providing a host cell comprising a gene encoding the light and heavy chains of said desired antibody, wherein said host cell is identified by any of the methods described herein as a host cell that produces a desired antibody with a greater yield and / or purity; and (b) culturing said host cell under conditions that permit expression of said light and heavy chain genes. The method may further comprise purification of said desired antibody.

The present disclosure further provides a method of recombinantly producing a desired multi-subunit complex, such as a desired antibody, comprising: (a) providing a host cell comprising multiple copies of the genes encoding the light and heavy chains of said desired antibody, which host cell produces a desired antibody with a greater yield and / or purity relative to an isogenic host cell containing only a single copy of said genes encoding the light and heavy chains of said desired antibody; and (b) culturing said host cell under conditions that permit expression of said light and heavy chain genes. The method may further comprise purification of said desired antibody.

Said methods may further comprise culturing using methods and/or conditions as described in co-owned U.S. application ser. no. 13/466,795 (Atty. Docket No. 75820.711001), entitled "HIGH-PURITY PRODUCTION OF MULTI-SUBUNIT PROTEINS SUCH AS ANTIBODIES IN TRANSFORMED MICROBES SUCH AS PICHIA PASTORIS" and filed May 8, 2012, published as US 2012/0277408 A1. For example, said culturing may include addition of an ethanol bolus to the culture, e.g., to a final concentration of about 1% w/w.

For example, the disclosure provides a method of producing a multi-subunit complex, comprising: (a) providing a culture comprising a eukaryotic cells comprising multiple copies of the genes that provide for the expression of the subunits of said multi-subunit complex; (b) adding a bolus of ethanol to said culture; and (c) culturing said culture to produce said multi-subunit complex.

The ethanol bolus may enhance the formation of stable disulfide bonds relative to the same method effected in the absence of the bolus of ethanol.

Said multi-subunit complex may contain one or more polypeptides comprising at least one disulfide bond.

Said multi-subunit complex may comprise an antibody.

The method may decrease the relative abundance of one or more product-associated variants relative to the same method effected in the absence of the bolus of ethanol.

The method may decrease the relative abundance of product-associated variants having a higher or lower apparent molecular weight than said desired multi-subunit complex as detected by size exclusion chromatography or gel electrophoresis relative to the same method effected in the absence of the bolus of ethanol.

The method may decrease the relative abundance of complexes having aberrant stoichiometry relative to the same method effected in the absence of the bolus of ethanol.

The method may decrease the relative abundance of complexes having aberrant disulfide bonds relative to the same method effected in the absence of the bolus of ethanol.

The method may decrease the relative abundance of complexes having reduced cysteines relative to the same method effected in the absence of the bolus of ethanol.

The method may decrease the relative abundance of complexes having aberrant glycosylation relative to the same method effected in the absence of the bolus of ethanol.

The method may modulate the formation or stability of inter-heavy chain disulfide bonds.

The method may modulate the formation or stability of disulfide bonds linking the light and heavy chains.

The method may decrease the relative abundance of one or more product-associated variants relative to the same method effected in the absence of the bolus of ethanol.

Said product-associated variants may comprise one or more of the H1L1, H2L1, and H4L4 product-associate variants.

The method increase the purity of said antibody relative to said method effected in the absence of said bolus of ethanol.

Step (b) may be effected prior to step (c).

Step (b) may be effected subsequent to step (c).

Step (b) may be effected concurrently with step (c).

Step (b) may result in a concentration of ethanol in said culture of between about 0.01% and about 4% (w/v).

Step (b) may result in a concentration of ethanol in said culture of between about 0.01% and about 4%, between about 0.02% and about 3.75%, between about 0.04% and about 3.5%, between about 0.08% and about 3.25%, between about 0.1% and about 3%, between about 0.2% and about 2.75%, between about 0.3% and about 2.5%, between about 0.4% and about 2.25%, between about 0.5% and about 1.5%, between about 0.5% and about 2%, between about 0.6% and about 1.75%, between about 0.7% and about 1.5%, or between about 0.8% and about 1.25%.

Step (b) may result in a concentration of ethanol in said culture that may be at least about 0.01%, 0.02%, 0.03%, 0.04%, 0.05%, 0.06%, 0.07%, 0.08%, 0.09%, 0.10%, 0.2%, 0.3%, 0.4%, 0.6%, 0.6%, 0.7%, 0.8% or 0.9% (w/v).

Step (b) may result in a concentration of ethanol in said culture that may be at most about 4%, 3.5%, 3%, 2.5%, 2%, 1.8%, 1.6%, 1.5%, 1.4%, 1.3%, 1.2%, 1.1%, 1.0%, 0.9%, 0.8%, 0.7%, 0.6%, 0.5%, 0.4%, 0.35%, 0.3%, 0.25%, 0.2%, or 0.15% (w/v).

Step (b) may comprise adding ethanol to said culture, adding a carrier comprising ethanol to said culture, adding said cells to a medium or carrier comprising ethanol, or replacing part of the culture medium.

Said bolus of ethanol may be added to the culture medium over a period of time between 1 and 20 minutes.

Step (c) may comprise providing oxygen to said cells.

Said providing oxygen may comprise agitating said culture.

Said providing oxygen may comprise contacting said culture with a gas mixture comprising oxygen.

Step (c) may comprise adding a feed comprising a carbon source to said culture.

Said feed may comprise at least one fermentable carbon source.

Said feed may comprise one or more of glucose, ethanol, citrate, sorbitol, xylose, trehalose, arabinose, galactose, fructose, melibiose, lactose, maltose, rhamnose, ribose, mannose, mannitol, and raffinose.

The method may further comprise maintaining the concentration of ethanol between an upper set point and a lower set point during step (c).

Said lower set point may be about 0.01%, 0.02%, 0.03%, 0.04%, 0.05%, 0.06%, 0.07%, 0.08%, 0.09%, 0.10%, 0.2%, 0.3%, 0.4%, 0.6%, 0.6%, 0.7%, 0.8% or 0.9% (w/v).

Said upper set point may be about 4%, 3.5%, 3%, 2.5%, 2%, 1.8%, 1.6%, 1.5%, 1.4%, 1.3%, 1.2%, 1.1%, 1.0%, 0.9%, 0.8%, 0.7%, 0.6%, 0.5%, 0.4%, 0.35%, 0.3%, 0.25%, 0.2%, or 0.15% (w/v).

Said upper set point may be at most about 1.5%, 1.4%, 1.3, 1.2%, or 1.1% (w/v).

The method may further comprise maintaining the concentration of ethanol at a set point during step (c).

Said set point may be about 0.1%, 0.2%, 0. 3%, 0. 4%, 0.5%, 0.6%, 0.7%, 0.8%, 0.9%, 01.%, 01.1%, 01.2%, 01.3%, 01.4%, or 01.5% (w/v).

Step (c) may comprise maintaining the concentration of ethanol in said culture between about 0.01% and about 4%, between about 0.02% and about 3.75%, between about 0.04% and about 3.5%, between about 0.08% and about 3.25%, between about 0.1% and about 3%, between about 0.2% and about 2.75%, between about 0.3% and about 2.5%, between about 0.4% and about 2.25%, between about 0.5% and about 2%, between about 0.6% and about 1.75%, between about 0.7% and about 1.5%, or between about 0.8% and about 1.25%.

The concentration of ethanol in said culture may be maintained by controlling production of ethanol by said cells or by addition of ethanol to said culture.

The step of controlling production of ethanol may comprise controlling one or more of the concentration of glucose, availability of oxygen, intensity of agitation, gas pressure, flow rate of supplied air or other gas mixture, viscosity of the culture, culture density, concentration of oxygen in the supplied air or other gas mixture, and temperature.

The time between step (a) and step (b) may be less than about 72 hours, less than about 48 hours, less than about 24 hours, less than about 12 hours, less than about 9 hours, less than about 6 hours, less than about 5 hours, less than about 4 hours, less than about 3 hours, less than about 90 minutes, less than about 30 minutes, less than about 5 minutes, or less than about 1 minute.

The time between step (b) and step (c) may be less than about 10 hours, less than about 9 hours, less than about 8 hours, less than about 7 hours, less than about 6 hours, less than about 5 hours, less than about 4 hours, less than about 3 hours, less than about 2 hours, less than about 90 minutes, less than about 80 minutes, less than about 70 minutes, less than about 60 minutes, less than about 50 minutes, less than about 40 minutes, less than about 30 minutes, less than about 20 minutes, less than about 10 minutes, less than about 5 minutes, or less than about 1 minute.

The culture of step (a) may be produced by adding a carbon source to said culture, and culturing said culture until the carbon source may be depleted.

Said carbon source may comprise one or more of: glycerol, glucose, ethanol, citrate, sorbitol, xylose, trehalose, arabinose, galactose, fructose, melibiose, lactose, maltose, rhamnose, ribose, mannose, mannitol, and raffinose.

The depletion of the carbon source may be determined by detecting a decrease in the metabolic activity of said eukaryotic cells.

Said decrease in the metabolic activity of said eukaryotic cells may be identified by detecting a decrease in the consumption of oxygen by said eukaryotic cells, by detecting an increase in pH in the culture, by detecting stabilization of the wet cell mass, or by detecting an increase in the concentration of ammonia in the culture.

Said decrease in the consumption of oxygen by said eukaryotic cells may be identified by detecting an increase in the concentration of dissolved oxygen in said culture.

Said eukaryotic cells may comprise yeast cells.

Said yeast cells may comprise methylotrophic yeast.

Said methylotrophic yeast may be of the genus Pichia.

Said methylotrophic yeast of the genus Pichia may be Pichia pastoris.

Said methylotrophic yeast of the genus Pichia may be selected from the group consisting of: Pichia angusta, Pichia guillermordii, Pichia methanolica, and Pichia inositovera.

The genes that provide for expression of said multi-subunit complex may be integrated into one or more genomic loci.

At least one of said genomic loci may be selected from the group consisting of the pGAP locus, 3' AOX TT locus; PpURA5; OCH1; AOX1; HIS4; GAP; pGAP; 3' AOX TT; ARG; and the HIS4 TT locus.

At least one of the genes encoding said subunits of the multi-subunit complex may be expressed under control of an inducible or constitutive promoter.

Said inducible promoter may be selected from the group consisting of the AOX1, CUP1, tetracycline inducible, thiamine inducible, and FLD1 promoters.

At least one of the genes encoding said subunits of the multi-subunit complex may be expressed under control of a promoter selected from the group consisting of: the CUP1, AOX1, ICL1, glyceraldehyde-3-phosphate dehydrogenase (GAP), FLD1, ADH1, alcohol dehydrogenase II, GAL4, PHO3, PHO5, and Pyk promoters, tetracycline inducible promoters, thiamine inducible promoters, chimeric promoters derived therefrom, yeast promoters, mammalian promoters, insect promoters, plant promoters, reptile promoters, amphibian promoters, viral promoters, and avian promoters.

Said eukaryotic cell may be a diploid, tetraploid cell, or polyploid.

The method may further comprise purifying said multi-subunit complex from said eukaryotic cells or from the culture medium.

Said multi-subunit complex may be purified from an intracellular component, cytoplasm, nucleoplasm, or a membrane of said eukaryotic cells.

Said eukaryotic cells secrete said multi-subunit complex into the culture medium.

Said multi-subunit complex may be purified from said culture medium.

Said multi-subunit complex may comprise a monospecific or bispecific antibody.

Said multi-subunit complex may comprise a human antibody or a humanized antibody or fragment thereof.

Said humanized antibody may be of mouse, rat, rabbit, goat, sheep, or cow origin.

Said humanized antibody may be of rabbit origin.

Said multi-subunit complex may comprise a monovalent, bivalent, or multivalent antibody.

Said antibody may be purified from said culture by protein A and/or protein G affinity.

At least one of the genes that provide for expression of a subunit of said multi-subunit complex in at least one of said eukaryotic cells in said panel may be optimized for expression in said eukaryotic cell.

Said multi-subunit complex may comprise an antibody and the purity of said antibody may be assessed by measuring the fraction of the antibody produced by said eukaryotic cell that may be contained in antibody complexes having the expected apparent hydrodynamic radius, may be contained in antibody complexes having the expected molecular weight, and / or specifically binds a target of said antibody.

Said multi-subunit complex may comprise an antibody and the yield of said antibody may be assessed by determining the amount of antibody produced by said eukaryotic cell discounting any product-associated variants that may be abnormally glycosylated, contained in antibody complexes other than complexes having the expected apparent hydrodynamic radius, contained in antibody complexes having the expected molecular weight, and / or that fail to specifically bind to the target of said antibody.

The molecular weight of said antibody complexes may be determined by non-reducing SDS-PAGE.

Said multi-subunit complex may comprise an antibody, said method may further comprise purifying said antibody.

Said culture cell may produce a supernatant antibody titer of at least 100 mg / L, at least 150 mg / L, at least 200 mg / L, at least 250 mg / L, at least 300 mg / L, between 100 and 300 mg / L, between 100 and 500 mg / L, between 100 and 1000 mg / L, at least 1000 mg / L, at least 1250 mg/liter, at least 1500 mg/liter, at least about 1750 mg/liter, at least about 2000 mg/liter, at least about 10000 mg/liter, or more.

One or more subunits of said multi-subunit complex may be expressed from more than one gene copy.

Said multi-subunit complex may comprise an antibody which may be expressed from between 1-10 copies of a gene encoding the light chain of said antibody and from 1-10 copies of a gene encoding the heavy chain of said antibody.

The genes that provide for expression of said multi-subunit complex may be integrated into genome of said cells.

The genes that provide for expression of said multi-subunit complex may be contained on an extrachromosomal element, plasmid, or artificial chromosome.

Said cells may comprise more copies of the gene that provide for the expression of the light chain of said antibody than copies of the gene that provide for expression of the heavy chain of said antibody.

The respective number of copies of the gene encoding the heavy chain of said antibody and the number of copies of the gene encoding the light chain of said antibody in said cells may be: 2 and 2, 2 and 3, 3 and 3, 3 and 4, 3 and 5, 4 and 3, 4 and 4, 4 and 5, 4 and 6, 5 and 4, 5 and 5, 5 and 6, or 5 and 7.

The respective number of copies of the gene encoding the heavy chain of said antibody and the number of copies of the gene encoding the light chain of said antibody in said cells may be: 2 and 1, 3 and 1, 4 and 1, 5 and 1, 6 and 1, 7 and 1, 8 and 1, 9 and 1, 10 and 1, 1 and 2, 2 and 2, 3 and 2, 4 and 2, 5 and 2, 6 and 2, 7 and 2, 8 and 2, 9 and 2, 10 and 2, 1 and 3, 2 and 3, 3 and 3, 4 and 3, 5 and 3, 6 and 3, 7 and 3, 8 and 3, 9 and 3, 10 and 3, 1 and 4, 2 and 4, 3 and 4, 4 and 4, 5 and 4, 6 and 4, 7 and 4, 8 and 4, 9 and 4, 10 and 4, 1 and 5, 2 and 5, 3 and 5, 4 and 5, 5 and 5, 6 and 5, 7 and 5, 8 and 5, 9 and 5, 10 and 5, 1 and 6, 2 and 6, 3 and 6, 4 and 6, 5 and 6, 6 and 6, 7 and 6, 8 and 6, 9 and 6, 10 and 6, 1 and 7, 2 and 7, 3 and 7, 4 and 7, 5 and 7, 6 and 7, 7 and 7, 8 and 7, 9 and 7, 10 and 7, 1 and 8, 2 and 8, 3 and 8, 4 and 8, 5 and 8, 6 and 8, 7 and 8, 8 and 8, 9 and 8, 10 and 8, 1 and 9, 2 and 9, 3 and 9, 4 and 9, 5 and 9, 6 and 9, 7 and 9, 8 and 9, 9 and 9, 10 and 9, 1 and 10, 2 and 10, 3 and 10, 4 and 10, 5 and 10, 6 and 10, 7 and 10, 8 and 10, 9 and 10, 10 and 10.

The culture of step (c) may be grown in a production medium.

Said production medium may be a minimal medium.

Said minimal medium lacks selective agents.

Said minimal medium lacks pre-formed amino acids or other complex biomolecules.

The production medium may be a complex medium.

The complex medium may comprise one or more of yeast extract, soy peptones, and other plant peptones.

The culture of step (c) may be grown to a high cell density.

Said high cell density may be at least 50 g/L.

Said high cell density may be at least 100 g/L.

Said high cell density may be at least 300 g/L.

Said high cell density may be at least 400 g/L.

Said high cell density may be at least 500 g/L.

Said high cell density may be at least 750 g/L.

The yeast cells may be cultured for at least 20 doublings and maintain high levels of expression of said multi-subunit complex after said at least 20 doublings.

The cells of step (c) may be cultured for at least 50 doublings and maintain high levels of expression of said multi-subunit complex after said at least 50 doublings.

The cells of step (c) may be cultured for at least 100 doublings and maintain high levels of expression of said multi-subunit complex after said at least 100 doublings.

At least one subunit of said multi-subunit complex may comprise a secretion signal.

Said multi-subunit complex may comprise an antibody.

The subject methods may produce a supernatant antibody titer of at least 100 mg / L, at least 150 mg / L, at least 200 mg / L, at least 250 mg / L, at least 300 mg / L, between 100 and 300 mg / L, between 100 and 500 mg / L, between 100 and 1000 mg / L or in excess of 1000 mg/L e.g., as high as 1200 mg/L, as high as 10,000 mg / L, or higher.

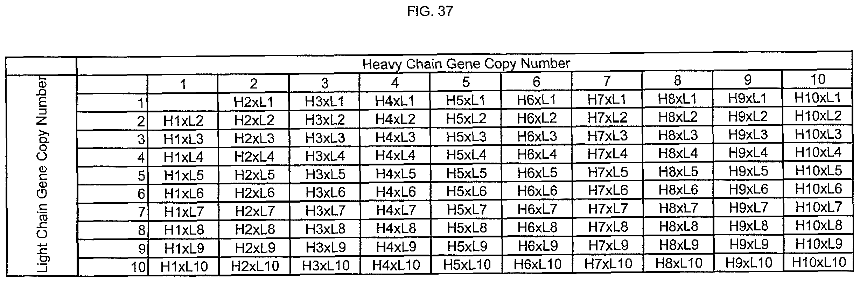

The present disclosure also provides a host cell identified by any of the foregoing methods as a host cell that produces a desired multi-subunit complex, such as a desired antibody, with a greater yield and / or purity,. The host cell may be a diploid or tetraploid cell of the genus Pichia, such as a *Pichia pastoris* cell. The present disclosure also provides a diploid or tetraploid yeast culture derived from the aforementioned host cell. The genes that provide for expression of the subunits of said desired multi-subunit complex, such as the light chain and heavy chain of a desired antibody, may be integrated into genome of said host cell. The genes that provide for expression of the subunits of said desired multi-subunit complex, such as the light chain and heavy chain of a desired antibody, may be contained on an extrachromosomal element, plasmid, or artificial chromosome. Where the desired multi-subunit complex is an antibody, the host cell may comprise more copies of the gene that provide for the expression of the light chain than copies of the gene that provide for expression of the heavy chain. In some examples, the host cell may comprise from 1-10 copies of a gene encoding the light chain and from 1-10 copies of a gene encoding the heavy chain. The respective number of copies of the gene encoding the heavy chain and the number of copies of the gene encoding the light chain in said host cell may be: 2 and 2, 2 and 3, 3 and 3, 3 and 4, 3 and 5, 4 and 3, 4 and 4, 4 and 5, 4 and 6, 5 and 4, 5 and 5, 5 and 6, or 5 and 7, respectively. Additional exemplary combinations of heavy and light chain gene copy numbers are enumerated in FIG. 37, which enumerates combinations having up to ten copies of the heavy and/or light chain gene, including strains having the identifiers H2xL1, H3xL1, H4xL1, H5xL1, H6xL1, H7xL1, H8xL1, H9xL1, H10xL1, H1xL2, H2xL2, H3xL2, H4xL2, H5xL2, H6xL2, H7xL2, H8xL2, H9xL2, H10xL2, H1xL3, H2xL3, H3xL3, H4xL3, H5xL3, H6xL3, H7xL3, H8xL3, H9xL3, H10xL3, H1xL4, H2xL4, H3xL4, H4xL4, H5xL4, H6xL4, H7xL4, H8xL4, H9xL4, H10xL4, H1xL5, H2xL5, H3xL5, H4xL5, H5xL5, H6xL5, H7xL5, H8xL5, H9xL5, H10xL5, H1xL6, H2xL6, H3xL6, H4xL6, H5xL6, H6xL6, H7xL6, H8xL6, H9xL6, H10xL6, H1xL7, H2xL7, H3xL7, H4xL7, H5xL7, H6xL7, H7xL7, H8xL7, H9xL7, H10xL7, H1xL8, H2xL8, H3xL8, H4xL8, H5xL8, H6xL8, H7xL8, H8xL8, H9xL8, H10xL8, H1xL9, H2xL9, H3xL9, H4xL9, H5xL9, H6xL9, H7xL9, H8xL9, H9xL9, H10xL9, H1xL10, H2xL10, H3xL10, H4xL10, H5xL10, H6xL10, H7xL10, H8xL10, H9xL10, H10xL10. For example, the specified number of heavy and light chain gene copies may be tandemly integrated into a single locus, or into multiple loci (any or all of which may contain more than one copy). Optionally, each genomic locus may contain no more than three or four tandemly integrated gene copies, thereby promoting copy number stability during propagation and/or antibody production.

Culturing most typically involves proving cells with an energy source, oxygen, and nutrients. Methods are also known in the literature for design and optimization of *P. pastoris* fermentations for expression of recombinant proteins, including optimization of the cell density, broth volume, substrate feed rate, and the length of each phase of the reaction. *See* Zhang et al., "Rational Design and Optimization of Fed-Batch and Continuous Fermentations" in Cregg, J. M., Ed., 2007, Pichia Protocols (2nd edition), Methods in Molecular Biology, vol. 389, Humana Press, Totowa, N.J., pgs. 43-63. The culture may be provided with a gas mixture comprising oxygen, such as air with or without oxygen supplementation. The yeast culture may be cultured in a culture medium which may be a minimal medium, may lack selective agents, and / or may lack pre-formed amino acids or other complex biomolecules. The culture medium may also be a complex medium (e.g., containing yeast extract and/or plant peptone(s)). The medium may include a nitrogen source (e.g., methylamine chloride, NH4SO4, yeast extract, soy peptone, other plant peptones, etc.). Exemplary minimal media include minimal dextrose medium (MD) (1.34% yeast nitrogen base (YNB) (w/o amino acids), 4 × 10⁻⁵% biotin, and 2% glucose.), buffered minimal glycerol complex medium (BMGY) (1% yeast extract, 2% peptone, 1% glycerol, 1.34% YNB (w/o amino acids), 4 × 10-5% biotin and 100 mM potassium phosphate (pH 6.0)). Media may include one or more salts (such as sodium chloride, calcium, magnesium, and phosphate), buffers (such as potassium phosphate, Tris, or HEPES), nucleosides (such as adenosine and thymidine), antibiotics (e.g., added to inhibit growth of contaminants and/or for maintenance of a selectable marker), trace elements, and glucose or another energy source. Any supplements and substitutions may also be included at appropriate concentrations that would be known to those skilled in the art.

The culture may be grown to a high cell density, such as at least 50 g/L, at least 100 g/L, at least 300 g/L, at least 400 g/L, at least 500 g/L, or at least 700 g/L. These culture densities are illustrative rather than limiting, and suitable culture densities may be readily determined by those of ordinary skill in the art.

The yeast cells may be cultured for at least 20 doublings and maintain high levels of expression of said antibody after said at least 20 doublings.

The yeast cells may be cultured for at least 50 doublings and maintain high levels of expression of said antibody after said at least 50 doublings.

The yeast cells may be cultured for at least 100 doublings and maintain high levels of expression of said antibody after said at least 100 doublings.

The present disclosure also provides a culture medium containing a stable diploid *Pichia* yeast culture produced according to any of the foregoing methods, wherein the culture medium may comprise expression levels of said desired antibody which may be at least about 50 mg/liter, 100 mg/liter, 500 mg/liter, 750 mg/liter, 1000 mg/liter, 1250 mg/liter, 1500 mg/liter, 1750 mg/liter, 2000 mg/liter, or more. These yield values are illustrative rather than limiting. Optionally, yield may be optimized, for example using the methods and general approach described in Zhang et al. (2007), *supra.* For example, yield may be optimized by varying temperature, pH, media composition (e.g., carbon source, carbon source concentration, mixture of two or more carbon sources, nitrogen source and concentration, concentration of salts and nutrients including KH₂PO₄, K₂HPO₄, MgSO₄, potassium sulfate, sodium citrate, potassium sulfate, sodium citrate, trace metals such as cobalt chloride, cupric sulfate, sodium iodide, manganese sulfate, sodium molybdate, boric acid, zinc chloride, ferrous sulfate, vitamins such as biotin, inositol, thiamine, peptone, yeast extract, casamino acids, urea, ammonium phosphate or other ammonium ions, L-arginine-hydrochloride), time, culture density, oxygenation, and other factors that influence yield. For example, yield, expression, and/or purity of the desired multi-subunit complex may in some instances be improved by maintaining the temperature at a desired set point, e.g., a set point between about 15°C and about 30 °C, such as between about 17 °C and about 25 °C). Without intent to be limited by theory, it is hypothesized that controlling the temperature may assist intracellular trafficking through the folding and post-translational processing pathways, and/or may decrease the activity of cellular proteases. Likewise, yield, expression, and/or purity of the desired multi-subunit complex may in some instances be improved by maintaining the pH of the culture medium at a desired set point, e.g., a set point between pH 3 to pH 8, such as between pH 4 and pH 7.

The present disclosure further provides a culture medium containing a stable diploid Pichia pastoris yeast culture derived from a cell produced according to any of the foregoing methods that expresses said desired antibody into a culture medium wherein the cell density of said diploid cells in said culture may be at least about 50 g/L, 100 g/L, 300 g/L, 400 g/L, 500 g/L, 700 g/L or more. These culture densities are illustrative rather than limiting, and suitable culture densities may be readily determined by those of ordinary skill in the art.

At least one subunit of said antibody or other multi-subunit protein may comprise a secretion signal, such as the S. chicken lysozyme (CLY) signal peptide; CLY-L8; S. cerevisiae invertase (SUC2) signal peptide; MF-alpha (Prepro); MF-alpha (Pre)-apv; MF-alpha (Pre)-apvSLEKR; MF-alpha (Prepro)-(EA)3; αF signal peptide; KILM1 signal peptide; repressible acid phosphatase (PHO1) signal peptide; A. niger GOX signal peptide; Schwanniomyces occidentalis glucoamylase gene (GAM1) signal peptide; human serum albumin (HSA) signal peptide without pro-sequence; human serum albumin (HSA) signal peptide with pro-sequence; ISN signal peptide; IFN signal peptide; HGH signal peptide; phytohaemagglutinin (PHA); Silkworm lysozyme; Human lysozyme (LYZ1); activin receptor type-1; activin type II receptor; P. pastoris immunoglobulin binding protein (PpBiP); human antibody 3D6 light chain leader; and any combination thereof.

The host cell may be produced by mating two haploid yeast cells that each contain one or more copies of a gene encoding one or more subunits of said antibody or other multi-subunit protein.

### BRIEF DESCRIPTION OF THE DRAWINGS

**FIG. 1** provides an overview of an exemplary methodology for obtaining haploid strains containing a specifically targeted number of copies of genes encoding a desired antibody light chain and / or heavy chain, and of mating haploid strains to obtain a panel of diploid strains that express the desired antibody from a specifically targeted number of copies of the light and heavy chain genes.
**FIG. 2** graphically illustrates relative whole antibody yield in comparison to the H3xL3 strain from selected diploid strains containing increasing numbers of copies of genes encoding the light and heavy chains of Ab-A. Setting the H3xL3 yield at 100%, the relative whole broth antibody titer generally increased with increasing antibody total copy number, in the order H3xL4, H3xL3, H4xL4, H4xL6, H5xL4, H5xL5, and H5xL7.
**FIG. 3** graphically illustrates relative whole broth antibody yield in comparison to the H3xL3 strain from strains containing increasing numbers of copies of genes encoding the light and heavy chains of Ab-B. Setting the H3xL3 antibody yield at 100%, the relative whole broth antibody titer generally increased with increasing antibody copy number, in the order H3xL3, H3xL4, H4xL3, H4xL5, and H4xL6.
**FIG. 4** graphically illustrates relative whole broth antibody yield in comparison to the H3xL3 strain from strains containing increasing numbers of copies of genes encoding the light and heavy chains of Ab-C. Setting the H3xL3 antibody yield at 100%, the relative whole broth antibody titer generally increased with increasing antibody copy number, in the order Ab-C-H3xL4, Ab-C-H4xL3, Ab-C-H4xL4, Ab-C-H4xL5, Ab-C-H5xL5, Ab-C-H5xL4, Ab-C-H5xL6, and Ab-C-H6xL5.
**FIG. 5A-E** shows the purity of protein-A capture eluate of Ab-A produced from H4xL4 and H4xL6 strains determined by HPLC. The level of the product-associated variant (measured by the percentage of total integrated area) migrating at 15.5 min, was decreased by more than five-fold (from 8.81 in H4xL4 down to 1.58% in H4xL6).
**FIG. 6A-E** shows the purity of protein-A capture eluate of Ab-B produced from H4xL3 and H4xL5 strains determined by HPLC. The level of the product-associated variant (measured by the percentage of total integrated area) migrating at 15.5 min, was decreased by about 59% (from 6.26% in H4xL3 down to 2.54% in H4xL5).
**FIG. 7A-E** shows the purity of protein-A capture eluate of Ab-C produced from HexL3 and H5xL5 strains determined by HPLC. The level of the product-associated variant (measured by the percentage of total integrated area) migrating at 15.2 to 16.1 min, was decreased by about 39% (from 6.55% in H3xL3 down to 4.00% in H5xL5).
**FIG. 8** shows a stained SDS-PAGE gel of Ab-A produced from H4xL4 and H4xL6 strains. An observed "low-mobility product-associated variant" (arrow) was less abundant in the preparation from the strain with the higher light chain copy number.
**FIG. 9** shows a stained SDS-PAGE gel of Protein-A purified Ab-B produced from strains H4xL5 and H4xL6. As with Ab-A, an observed "low-mobility product-associated variant" (arrow) was less abundant in the preparation from the strain with the higher light chain copy number.
**FIG. 10** shows a stained SDS-PAGE gel of Protein-A purified Ab-C produced from strains H3xL3 and H5xL5. As with Ab-A and Ab-B, an observed "low-mobility product-associated variant" (arrow) was less abundant in the preparation from the strain with the higher antibody chain copy number.

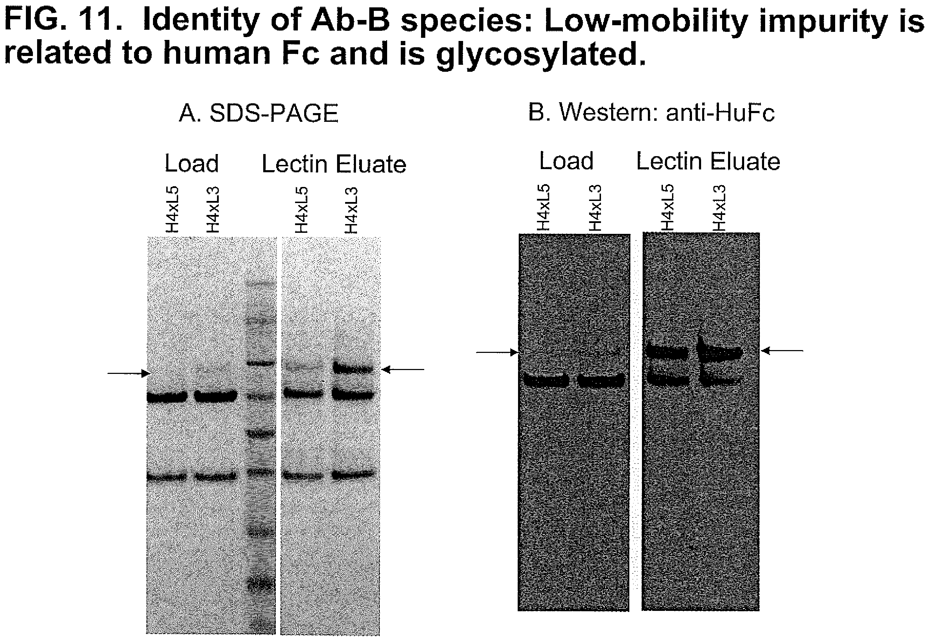

**FIG. 11** shows identification of the low-mobility product-associated variant as a glycosylated protein related to human Fc (demonstrated by its selective enrichment by a lectin column and specific recognition by an anti-Fc antibody). An antibody preparation ("Load") was bound to a lectin resin and eluted ("Lectin Eluate"). SDS-PAGE **(****FIG. 11A****)** demonstrated selective enrichment of the low-mobility product-associated variant by the lectin column. Western blotting with an anti-HuFc antibody **(****FIG. 11A****)** detected the low-mobility product-associated variant, indicating that it contained at least a partial human Fc sequence. This product-associated variant is referred to herein as the "glyco-heavy variant." Additionally, the amount of this product-associated variant was visibly reduced in the antibody preparation from strain H4xL5 relative to strain H4xL3.
**FIGS. 12A-D** **and** **13A-D** demonstrate that a product-associated variant observed by HPLC (having a retention time of approximately 15.5 minutes) was selectively enriched in the lectin column eluate, indicating that the glyco-heavy variant was a constituent of this product-associated variant. Antibody Ab-B was prepared from H4xL3 and H4xL5 strains.
**FIG. 14** shows a map of a construct used for targeted integration of an antibody heavy chain sequence for Ab-A or Ab-B into the pGAP locus (Locus # 1).
**FIG. 15** shows a map of a construct used for targeted integration of an antibody light chain sequence for Ab-A or Ab-B into the pGAP locus (Locus # 1).
**FIG. 16** shows a map of a construct used for targeted integration of an antibody heavy chain sequence for Ab-A or Ab-B into the HIS4 TT locus (Locus # 2).
**FIG. 17** shows a map of a construct used for targeted integration of an antibody light chain sequence for Ab-A or Ab-B into the HIS4 TT locus (Locus # 2).
**FIG. 18** shows a map of a construct used for targeted integration of an antibody heavy chain sequence for Ab-C into the AOX1 TT locus (Locus # 1).
**FIG. 19** shows a map of a construct used for targeted integration of an antibody light chain sequence for Ab-C into the AOX1 TT locus (Locus # 1).
**FIG. 20** shows a map of a construct used for targeted integration of an antibody heavy chain sequence for Ab-C into the HIS4 TT locus (Locus # 2).
**FIG. 21** shows a map of a construct used for targeted integration of an antibody light chain sequence for Ab-C into the HIS4 TT locus (Locus # 2).
**FIG. 22** illustrates the relationship between antibody copy number integrated at a single locus and the expected fragment sizes detectable by Southern blot.
**FIGS. 23** **and** **24** show Southern blots used to detect the number of copies of an antibody heavy chain gene and light chain gene, respectively, in multiple isolates transformed with genes encoding Ab-A chains.
**FIGS. 25-27** show Southern blots used to confirm the number of copies of the genes encoding the Ab-A heavy and light chains present at the pGAP **(****FIGS. 25-26****)** and HIS4 TT **(****FIG. 27****)** loci in a panel of diploid strains produced by mating transformed haploid strains.
**FIG. 28A-B** shows Southern blots used to detect the number of copies of the antibody heavy chain gene and light chain genes, respectively, in multiple isolates transformed with genes encoding Ab-B chains.
**FIGS. 29-31** show Southern blots that confirmed the number of copies of the genes encoding the Ab-B heavy and light chains present at the pGAP **(****FIGS. 29-30****)** and HIS4 TT **(****FIG. 31****)** loci in a panel of diploid strains produced by mating transformed haploid strains.
**FIGS. 32-33** show Southern blots used to detect the number of copies of the antibody heavy chain and light chain genes, respectively, in multiple isolates transformed with genes encoding the Ab-C chains.
**FIGS. 34-36** show Southern blots that confirmed the number of copies of the genes encoding the Ab-C heavy and light chains present at the 3' AOX TT **(****FIGS. 34-35****)** and HIS4 TT **(****FIG. 36****)** loci in a panel of diploid strains produced by mating transformed haploid strains.
**FIG. 37** illustrates exemplary, non-limiting combinations of light and heavy chain gene copy numbers that may be used in accordance with embodiments of the present disclosure.
**FIG. 38** shows the sequence of polynucleotides encoding the Ab-A light and heavy chains and the polypeptides they encode, as well as CDR sequences contained therein.
**FIG. 39** shows the sequence of polynucleotides encoding the Ab-B light and heavy chains and the polypeptides they encode, as well as CDR sequences contained therein.
**FIG. 40** shows the sequence of polynucleotides encoding the Ab-C light and heavy chains and the polypeptides they encode.

### DETAILED DESCRIPTION

The present disclosure provides methods of generating and identifying host cells able to produce an increased yield of a desired heterologous multi-subunit complex and / or produce a desired heterologous multi-subunit complex having improved purity. In a preferred example, the heterologous multi-subunit complex is an antibody or antibody fragment, such as a humanized antibody, comprised of two heavy chain subunits and two light chain subunits. Preferred host cells include yeasts, and particularly preferred yeasts include methylotrophic yeast strains, *e.g., Pichia pastoris, Hansenula polymorpha* (*Pichia angusta*)*, Pichia guillermordii, Pichia methanolica, Pichia inositovera,* and others (*see, e.g.,* U.S. Patent 4,812,405, 4,818,700, 4,929,555, 5,736,383, 5,955,349, 5,888,768, and 6,258,559). The host cell may be produced by methods known in the art. For example, a panel of diploid or tetraploid yeast cells containing differing combinations of gene copy numbers may be generated by mating cells containing varying numbers of copies of the individual subunit genes (which numbers of copies preferably are known in advance of mating).

Applicants have unexpectedly discovered that cultures can maintain a high stable copy number of the genes encoding the desired multi-subunit complex. The working examples demonstrate cells which maintain up to six or seven copies of antibody heavy and light chain-encoding genes. These cells can stably express the desired antibody even over a prolonged culture duration. Additionally, the cells can maintain high yield and expression of the desired multi-subunit complex even after a prolonged culture duration.

In a preferred example, the host cell may comprise more than one copy of one or more of the genes encoding the heterologous protein subunits. For example, multiple copies of a subunit gene may be integrated in tandem into one or more chromosomal loci. Tandemly integrated gene copies are preferably retained in a stable number of copies during culture for the production of the multi-subunit complex. For example, in the examples described below, gene copy numbers were generally stable for *P. pastoris* strains containing three to four tandemly integrated copies of light and heavy chain antibody genes.

One or more of the genes encoding the heterologous protein subunits are preferably integrated into one or more chromosomal loci of a host cell. Any suitable chromosomal locus may be utilized for integration, including intergenic sequences, promoters sequences, coding sequences, termination sequences, regulatory sequences, etc.. Exemplary chromosomal loci that may be used in *P. pastoris* include PpURA5; *OCH1; AOX1; HIS4;* and *GAP.* The encoding genes may also be integrated into one or more random chromosomal loci rather than being targeted. In preferred example, the chromosomal loci are selected from the group consisting of the pGAP locus, the 3'AOX TT locus and the HIS4 TT locus. In additional examples, the genes encoding the heterologous protein subunits may be contained in one or more extrachromosomal elements, for example one or more plasmids or artificial chromosomes.

In some examples, the multi-subunit protein may comprise two, three, four, five, six, or more non-identical subunits. Additionally, each subunit may be present one or more times in each multi-subunit protein. For example, the multi-subunit protein may be a multi-specific antibody such as a bi-specific antibody comprising two non-identical light chains and two non-identical heavy chains. A panel of diploid or tetraploid yeast cells containing differing combinations of gene copy numbers may be quickly generated by mating cells containing varying copy numbers of the individual subunit genes. Antibody production from each strain in the panel may then be assessed to identify a strain for further use based on a characteristic such as yield of the desired multi-subunit protein or purity of the desired multi-subunit protein relative to undesired side-products.

The subunits may be expressed from monocistronic genes, polycistronic genes, or any combination thereof. Each polycistronic gene may comprise multiple copies of the same subunit, or may comprise one or more copies of each different subunit.

Exemplary methods that may be used for manipulation of *Pichia pastoris* (including methods of culturing, transforming, and mating) are disclosed in Published Applications including U.S. 20080003643, U.S. 20070298500, and U.S. 20060270045, and in Higgins, D. R., and Cregg, J. M., Eds. 1998. Pichia Protocols. Methods in Molecular Biology. Humana Press, Totowa, N.J., and Cregg, J. M., Ed., 2007, Pichia Protocols (2nd edition), Methods in Molecular Biology. Humana Press, Totowa, N.J.

An exemplary expression cassette that may be utilized is composed of the glyceraldehyde dehydrogenase gene (GAP gene) promoter, fused to sequences encoding a secretion signal, followed by the sequence of the gene to be expressed, followed by sequences encoding a *P. pastoris* transcriptional termination signal from the *P. pastoris* alcohol oxidase I gene (AOX1). The Zeocin resistance marker gene may provide a means of enrichment for strains that contain multiple integrated copies of an expression vector in a strain by selecting for transformants that are resistant to higher levels of Zeocin. Similarly, G418 or Kanamycin resistance marker genes may be used to provide a means of enrichment for strains that contain multiple integrated copies of an expression vector in a strain by selecting for transformants that are resistant to higher levels of Geneticin or Kanamycin.

Host strains that may be utilized include auxotrophic *P. pastoris* or other Pichia strains, for example, strains having mutations in met1, lys3, ura3 and ade1 or other auxotrophy-associated genes. Preferred mutations are incapable of giving rise to revertants at any appreciable frequency and are preferably partial or even more preferably full deletion mutants. Preferably, prototrophic diploid or tetraploid strains are produced by mating a complementing sets of auxotrophic strains.

Transformation of haploid *P. pastoris* strains and genetic manipulation of the *P. pastoris* sexual cycle may be performed as described in Pichia Protocols (1998, 2007), *supra.*

Prior to transformation, each expression vector may be linearized by restriction enzyme cleavage within a region homologous to the target genomic locus (e.g., the GAP promoter sequence) to direct the integration of the vectors into the target locus in the host cell. Samples of each vector may then be individually transformed into cultures of the desired strains by electroporation or other methods, and successful transformants may be selected by means of a selectable marker, *e.g.,* antibiotic resistance or complementation of an auxotrophy. Isolates may be picked, streaked for single colonies under selective conditions and then examined to confirm the number of copies of the gene encoding the subunit of the multi-subunit complex (e.g., a desired antibody) by Southern Blot or PCR assay on genomic DNA extracted from each strain. Optionally, expression of the expected subunit gene product may be confirmed, e.g., by FACS, Western Blot, colony lift and immunoblot, and other means known in the art. Optionally, haploid isolates are transformed additional times to introduce additional heterologous genes, e.g., additional copies of the same subunit integrated at a different locus, and / or copies of a different subunit. The haploid strains are then mated to generate diploid strains (or strains of higher ploidy) able to synthesize the multi-protein complex. Presence of each expected subunit gene may be confirmed by Southern blotting, PCR, and other detection means known in the art. Where the desired multi-protein complex is an antibody, its expression may also be confirmed by a colony lift/immunoblot method (Wung et al. Biotechniques 21 808-812 (1996) and / or by FACS.

This transformation protocol is optionally repeated to target a heterologous gene into a second locus, which may be the same gene or a different gene than was targeted into the first locus. When the construct to be integrated into the second locus encodes a protein that is the same as or highly similar to the sequence encoded by the first locus, its sequence may be varied to decrease the likelihood of undesired integration into the first locus. For example, the sequence to be integrated into the second locus may have differences in the promoter sequence, termination sequence, codon usage, and/or other tolerable sequence differences relative to the sequence integrated into the first locus.

To mate *P. pastoris* haploid strains, each strain to be crossed can be patched together onto mating plates. For example, multiple matings can be conveniently performed at the same time by streaking each strain to be mated across a plate suitable for its growth, and the mating partners may be streaked across a second plate (preferably the plates are rich media such as YPD). Typically, after one or two days incubation at 30° C., cells from the two plates can be replica plated in a crisscross fashion onto a mating plate, resulting in a cross-hatched pattern with each pair of strains being co-plated and having the opportunity to mate at the intersection of a pair of the original streak lines. The mating plate can then be incubated (e.g., at 30° C.) to stimulate the initiation of mating between strains. After about two days, the cells on the mating plates can be streaked, patched, or replica plated onto media selective for the desired diploid strains (e.g., where the mated strains have complementary autotrophies, drop-out or minimal medium plates may be used). These plates can be incubated (e.g., at 30° C.) for a suitable duration (e.g., about three days) to allow for the selective growth of the desired diploid strains. Colonies that arise can be picked and streaked for single colonies to isolate and purify each diploid strain.

Expression vectors for use in the methods described herein may further include yeast specific sequences, including a selectable auxotrophic or drug marker for identifying transformed yeast strains. A drug marker may further be used to amplify copy number of the vector in a yeast host cell, e.g., by culturing a population of cells in an elevated concentration of the drug, thereby selecting transformants that express elevated levels of the resistance gene.

In one example, one or more of the genes encoding the heterologous protein subunits are coupled to an inducible promoter. Suitable exemplary promoters include the alcohol oxidase 1 gene promoter, formaldehyde dehydrogenase genes (FLD; see U.S. Pub. No. 2007/0298500), and other inducible promoters known in the art. The alcohol oxidase 1 gene promoter, is tightly repressed during growth of the yeast on most common carbon sources, such as glucose, glycerol, or ethanol, but is highly induced during growth on methanol (Tschopp et al., 1987; U.S. Pat. No. 4,855,231 to Stroman, D. W., et al). For production of foreign proteins, strains may be initially grown on a repressing carbon source to generate biomass and then shifted to methanol as the sole (or main) carbon and energy source to induce expression of the foreign gene. One advantage of this regulatory system is that *P. pastoris* strains transformed with foreign genes whose expression products are toxic to the cells can be maintained by growing under repressing conditions.

In another example, one or more of the heterologous genes may be coupled to a regulated promoter, whose expression level can be upregulated under appropriate conditions. Exemplary regulated promoters include the CUP1 promoter (induced by the level of copper in the medium), tetracycline inducible promoters, thiamine inducible promoters, the AOX1 promoter, and the FLD1 promoter.

Though much of the present disclosure describes production of antibodies, the methods described herein are readily adapted to other multi-subunit complexes as well. Without intent to be limited by theory, it is believed that the yield and purity of multi-subunit complexes can be greatly influenced by the concentration and stoichiometry of the subunits, which are in turn influenced by the level of expression of the genes responsible for production of each subunit. The methods disclosed herein may readily be utilized to improve the yield and / or purity of any recombinant multi-subunit complex comprising two or more different subunits. Additionally, the present methods are not limited to production of multi-protein complexes but may also be readily adapted for use with ribonucleoprotein (RNP) complexes including telomerase, hnRNPs, Ribosomes, snRNPs, signal recognition particles, prokaryotic and eukaryotic RNase P complexes, and any other complexes that contain multiple distinct protein and / or RNA subunits. The host cell that expresses the multi-subunit complex may be produced by methods known in the art. For example, a panel of diploid or tetraploid yeast cells containing differing combinations of gene copy numbers may be generated by mating cells containing varying numbers of copies of the individual subunit genes (which numbers of copies preferably are known in advance of mating).

### Definitions

It is to be understood that this invention is not limited to the particular methodology, protocols, cell lines, animal species or genera, and reagents described, as such may vary. It is also to be understood that the terminology used herein is for the purpose of describing particular embodiments only, and is not intended to limit the scope of the present invention which is defined by the claims.

As used herein the singular forms "a", "and", and "the" include plural referents unless the context clearly dictates otherwise. Thus, for example, reference to "a cell" includes a plurality of such cells and reference to "the protein" includes reference to one or more proteins and equivalents thereof known to those skilled in the art, and so forth. All technical and scientific terms used herein have the same meaning as commonly understood to one of ordinary skill in the art to which this invention belongs unless clearly indicated otherwise.

*Bolus addition: In the present disclosure, "bolus addition" generally refers to rapid change in concentration of a substance (such as ethanol) in contact with cultured cells (for example, in a culture medium). For example, the substance may be added to the cultured cells in a single addition, a succession of more than one addition, and*/*or infused over a period of time (e.g., over about 1, 2, 3, 4, 5, 6, 7, 8, 9, 10, 15, 20, 25, 30, 40, 50, 60, 90, or 120 minutes). The substance may also be added by replacing the culture medium in part or in full, for example by concentrating the cells (using centrifugation, filtration, settling, or other methods), removing part or all of the medium, and adding the substance, or by adding the cells to a medium containing the substance. The substance may be admixed with a carrier (e.g., culture media, water, saline, etc.). For example, a bolus addition of ethanol may comprise the addition of pure or concentrated ethanol (e.g., 100%, 95%, 70%, 50%, 60%, 40%, 30%, 20%, etc.) to the culture medium in an amount sufficient to produce the desired concentration. As another example, the cells may be added to a medium containing ethanol, e.g., by adding an inoculum containing the cells to a medium containing ethanol.*

*Bolus concentration: In the present disclosure, "bolus concentration* " *generally refers to the concentration that results from a bolus addition of a substance (e.g., ethanol).*

*Mating competent yeast species:* In the present disclosure this is intended to broadly encompass any diploid or tetraploid yeast which can be grown in culture. Such species of yeast may exist in a haploid, diploid, or other polyploid form. The cells of a given ploidy may, under appropriate conditions, proliferate for an indefinite number of generations in that form. Diploid cells can also sporulate to form haploid cells. Sequential mating can result in tetraploid strains through further mating or fusion of diploid strains. The present disclosure contemplates the use of haploid yeast, as well as diploid or other polyploid yeast cells produced, for example, by mating or fusion (e.g., spheroplast fusion).

In one example, the mating competent yeast is a member of the *Saccharomycetaceae* family, which includes the genera *Arxiozyma; Ascobotryozyma; Citeromyces; Debaryomyces; Dekkera; Eremothecium; Issatchenkia; Kazachstania; Kluyveromyces; Kodamaea; Lodderomyces; Pachysolen; Pichia; Saccharomyces; Saturnispora; Tetrapisispora; Torulaspora; Williopsis;* and *Zygosaccharomyces.* Other types of yeast that are potentially useful include *Yarrowia; Rhodosporidium; Candida; Hansenula; Filobasium; Sporidiobolus; Bullera; Leucosporidium* and *Filobasidella.*

In a preferred example, the mating competent yeast is a member of the genus *Pichia or is another methylotroph.* In a further preferred example, the mating competent yeast of the genus *Pichia* is one of the following species: *Pichia pastoris, Pichia methanolica,* and *Hansenula polymorpha (Pichia angusta*)*.* In a particularly preferred example, the mating competent yeast of the genus *Pichia* is the species *Pichia pastoris.*

*Haploid Yeast Cell:* A cell having a single copy of each gene of its normal genomic (chromosomal) complement.

*Polyploid Yeast Cell:* A cell having more than one copy of its normal genomic (chromosomal) complement.

*Diploid Yeast Cell:* A cell having two copies (alleles) of essentially every gene of its normal genomic complement, typically formed by the process of fusion (mating) of two haploid cells.

*Tetraploid Yeast Cell:* A cell having four copies (alleles) of essentially every gene of its normal genomic complement, typically formed by the process of fusion (mating) of two diploid cells. Tetraploids may carry two, three, four, or more different expression cassettes. Such tetraploids might be obtained in *S. cerevisiae* by selective mating homozygotic heterothallic a/a and alpha/alpha diploids and in Pichia by sequential mating of haploids to obtain auxotrophic diploids. For example, a [met his] haploid can be mated with [ade his] haploid to obtain diploid [his]; and a [met arg] haploid can be mated with [ade arg] haploid to obtain diploid [arg]; then the diploid [his] can be mated with the diploid [arg] to obtain a tetraploid prototroph. It will be understood by those of skill in the art that reference to the benefits and uses of diploid cells may also apply to tetraploid cells.

*Yeast Mating:* The process by which two yeast cells fuse to form a single yeast cell. The fused cells may be haploid cells or cells of higher ploidy (e.g., mating two diploid cells to produce a tetraploid cell).

*Meiosis:* The process by which a diploid yeast cell undergoes reductive division to form four haploid spore products. Each spore may then germinate and form a haploid vegetatively growing cell line.

*Selectable Marker:* A selectable marker is a gene or gene fragment that confers a growth phenotype (physical growth characteristic) on a cell receiving that gene as, for example through a transformation event. The selectable marker allows that cell to survive and grow in a selective growth medium under conditions in which cells that do not receive that selectable marker gene cannot grow. Selectable marker genes generally fall into several types, including positive selectable marker genes such as a gene that confers on a cell resistance to an antibiotic or other drug, temperature when two temperature sensitive ("ts") mutants are crossed or a ts mutant is transformed; negative selectable marker genes such as a biosynthetic gene that confers on a cell the ability to grow in a medium without a specific nutrient needed by all cells that do not have that biosynthetic gene, or a mutagenized biosynthetic gene that confers on a cell inability to grow by cells that do not have the wild type gene; and the like. Suitable markers include but are not limited to: ZEO; NEO (G418); LYS3; MET1; MET3a; ADE1; ADE3; URA3; and the like.

*Integrated:* A genetic element (typically a heterologous genetic element) that are covalently joined into a chromosome of an organism.

*Tandemly integrated:* Two or more copies of a genetic element that are integrated in adjacent locations in a chromosome. The two or more copies do not necessarily have the orientation; e.g., for transcribed genes, some copies may be transcribed from the Watson strand and others from the Crick strand.

*Host cell:* In the context of the present disclosure, the term host cell refers to a cell (e.g., a eukaryotic cell, such as a Pichia cell) which contains a heterologous gene. For example, the heterologous gene may provide for the expression of a subunit of a desired multi-subunit complex, a gene involved in protein folding (e.g., a chaperone), expression, or secretion, and/or another desired gene. The heterologous gene may be integrated into the genome of the eukaryotic cell or contained in extrachromosomal element such as a plasmid or artificial chromosome.

*Expression Vector:* These DNA vectors contain elements that facilitate manipulation for the expression of a foreign protein within the target host cell. Conveniently, manipulation of sequences and production of DNA for transformation is first performed in a bacterial host, e.g. E. coli, and usually vectors will include sequences to facilitate such manipulations, including a bacterial origin of replication and appropriate bacterial selection marker. Selection markers encode proteins necessary for the survival or growth of transformed host cells grown in a selective culture medium. Host cells not transformed with the vector containing the selection gene will not survive in the culture medium. Typical selection genes encode proteins that (a) confer resistance to antibiotics or other toxins, (b) complement auxotrophic deficiencies, or (c) supply critical nutrients not available from complex media. Exemplary vectors and methods for transformation of yeast are described, for example, in Burke, D., Dawson, D., & Stearns, T. (2000). Methods in yeast genetics: a Cold Spring Harbor Laboratory course manual. Plainview, N.Y.: Cold Spring Harbor Laboratory Press.

Expression vectors for use in the methods of the disclosure may further include yeast specific sequences, including a selectable auxotrophic or drug marker for identifying transformed yeast strains. A drug marker may further be used to select for amplification of copy number of the vector in a yeast host cell.

The polypeptide coding sequence of interest is typically operably linked to transcriptional and translational regulatory sequences that provide for expression of the polypeptide in yeast cells. These vector components may include, but are not limited to, one or more of the following: an enhancer element, a promoter, and a transcription termination sequence. Sequences for the secretion of the polypeptide may also be included, e.g. a signal sequence, and the like. A yeast origin of replication is optional, as expression vectors are often integrated into the yeast genome.

Though optional, in one example, one or more subunit of the multi-subunit complex is operably linked, or fused, to a secretion sequence that provides for secretion of the expressed polypeptide into the culture media, which can facilitate harvesting and purification of the heterologous multi-subunit complex. Even more preferably, the secretion sequences provide for optimized secretion of the polypeptide from the host cells (e.g., yeast diploid cells), such as through selecting preferred codons and/or altering the percentage AT through codon selection. It is known in the art that secretion efficiency and / or stability can be affected by the choice of secretion sequence and the optimal secretion sequence can vary between different proteins (*see,* e.g., Koganesawa et al., Protein Eng. 2001 Sep;14(9):705-10). Many potentially suitable secretion signals are known in the art and can readily be tested for their effect upon yield and/or purity of a particular heterologous multi-subunit complex. Any secretion sequences may potentially be used, including those present in secreted proteins of yeasts and other species, as well as engineered secretion sequences. Exemplary secretion sequences that may be utilized include: chicken lysozyme (CLY) signal peptide (MRSLLILVLCFLPLAALG (SEQ ID NO:31)), CLY-L8 (MRLLLLLLLLPLAALG (SEQ ID NO:32)), S. cerevisiae invertase (SUC2) signal peptide (MLLQAFLFLLAGFAAKISA (SEQ ID NO:33)), MF-alpha (Prepro) (MRFPSIFTAVLFAASSALA-APVNTTTE-EGVSLEKR (SEQ ID NO:34)), MF-alpha (Pre)-apv (MRFPSIFTAVLFAASSALA-APV (SEQ ID NO:35)), MF-alpha (Pre)-apv-SLEKR (MRFPSIFTAVLFAASSALA-APVSLEKR (SEQ ID NO:36)), MF-alpha (Prepro)-(EA)3 (MRFPSIFTAVLFAASSALA-APVNTTTE-EGVSLEKR-EAEAEA (SEQ ID NO:37)), αF signal peptide (MRFPSIFTAVLFAASSALA-APVNTTTE-DETAQIPAEAVIGYSDLEGDFDVAVLPFSNSTNNGLLFINTTIASIAAKE-EGVSLEKR (SEQ ID NO:38)), KILM1 signal peptide (MTKPTQVLVRSVSILFFITLLHLVVALNDVAGPAETAPVSLLPR (SEQ ID NO:39)), repressible acid phosphatase (PHO1) signal peptide (MFSPILSLEIILALATLQSVFA (SEQ ID NO:40)), A. niger GOX signal peptide (MQTLLVSSLWSLAAALPHYIR (SEQ ID NO:41)), Schwanniomyces occidentalis glucoamylase gene (GAM1) signal peptide (MIFLKLIKSIVIGLGLVSAIQA (SEQ ID NO:42)), human serum albumin (HSA) signal peptide with pro-sequence (MKWVTFISLLFLFSSAYSRGVFRR (SEQ ID NO:43)), human serum albumin (HSA) signal peptide without pro-sequence (MKWVTFISLLFLFSSAYS (SEQ ID NO:44)), ISN signal peptide (MALWMRLLPLLALLALWGPDPAAA (SEQ ID NO:45)), IFN signal peptide (MKYTSYILAFQLCIVLGSLGCDLP (SEQ ID NO:46)), HGH signal peptide (MAADSQTPWLLTFSLLCLLWPQEPGA (SEQ ID NO:47)), phytohaemagglutinin (PHA) (MKKNRMMMMIWSVGWWMLLLVGGSYG (SEQ ID NO:48)), Silkworm lysozyme (MQKLIIFALWLCVGSEA (SEQ ID NO:49)), Human lysozyme (LYZ1) (MKALIVLGLVLLSVTVQG (SEQ ID NO:50)), activin receptor type-1 (MVDGVMILPVLIMIALPSPS (SEQ ID NO:51)), activin type II receptor (MGAAAKLAFAVFLISCSSG (SEQ ID NO:52)), P. pastoris immunoglobulin binding protein (PpBiP) (MLSLKPSWLTLAALMYAMLLVWPFAKPVRA (SEQ ID NO:53)), and human antibody 3D6 light chain leader (MDMRVPAQLLGLLLLWLPGAKC (SEQ ID NO:54)). See Hashimoto et al., Protein Engineering vol. 11 no. 2 pp.75-77, 1998; Oka et al., Biosci Biotechnol Biochem. 1999 Nov; 63(11):1977-83; Gellissen et al., FEMS Yeast Research 5 (2005) 1079-1096; Ma et al., Hepatology. 2005 Dec;42(6):1355-63; Raemaekers et al., Eur J Biochem. 1999 Oct 1;265(1):394-403; Koganesawa et al., Protein Eng. (2001) 14 (9): 705-710; Daly et al., Protein Expr Purif. 2006 Apr;46(2):456-67 ; Damasceno et al., Appl Microbiol Biotechnol (2007) 74:381-389; and Felgenhauer et al., Nucleic Acids Res. 1990 Aug 25;18(16):4927). The multi-subunit complex may also be secreted into the culture media without being operably linked or fused to a secretion signal. For example, it has been demonstrated that some heterologous polypeptides are secreted into the culture media when expressed in *P. pastoris* even without being linked or fused to a secretion signal. Additionally, the multi-subunit complex may be purified from host cells (which, for example, may be preferable if the complex is poorly secreted) using methods known in the art.

Media or cells comprising a desired multi-subunit complex may be recovered from the culture. Optionally, the secreted proteins may be purified. For example, cells comprising a desired multi-subunit complex may be lysed using mechanical, chemical, enzymatic, and/or osmotic methods (e.g., freezing with liquid nitrogen, using a homogenizer, spheroplasting, sonication, agitation in the presence of glass beads, using detergents, etc.). The desired multi-subunit complex may be concentrated, filtered, dialyzed, etc., using methods known in the art. The desired multi-subunit complex may be purified based on, for example, its molecular mass (e.g., size exclusion chromatography), isoelectric point (e.g., isoelectric focusing), electrophoretic mobility (e.g., gel electrophoresis), hydrophobic interaction chromatography (e.g., HPLC), charge (e.g., ion exchange chromatography), affinity (e.g., in the case of an antibody, binding to protein A, protein G, and/or an epitope to which the desired antibody binds), and/or glycosylation state (e.g., detected by lectin binding affinity). Multiple purification steps may be performed to obtain the desired level of purity. In one example, the desired multi-subunit complex may be comprise an immunoglobulin constant domain and may be purified using protein A or protein G affinity, size exclusion chromatography, and lack of binding to lectin (to remove glycosylated forms). Optionally the A protease inhibitor, such as phenyl methyl sulfonyl fluoride (PMSF) may be added to inhibit proteolytic degradation during purification.

Nucleic acids are "operably linked" when placed into a functional relationship with another nucleic acid sequence. For example, DNA for a signal sequence is operably linked to DNA for a polypeptide if it is expressed as a preprotein that participates in the secretion of the polypeptide; a promoter or enhancer is operably linked to a coding sequence if it affects the transcription of the sequence. Generally, "operably linked" means that the DNA sequences being linked are contiguous, and, in the case of a secretory leader, contiguous and in reading frame. However, enhancers do not have to be contiguous. Linking may be accomplished by ligation at convenient restriction sites or alternatively via a PCR/recombination method familiar to those skilled in the art (Gateway® Technology; Invitrogen, Carlsbad Calif.). If such sites do not exist, the synthetic oligonucleotide adapters or linkers may be used in accordance with conventional practice. Desired nucleic acids (including nucleic acids comprising operably linked sequences) may also be produced by chemical synthesis.

Promoters are untranslated sequences located upstream (5') to the start codon of a structural gene (generally within about 100 to 1000 bp) that control the transcription and translation of particular nucleic acid sequences to which they are operably linked. Such promoters fall into several classes: inducible, constitutive, and repressible promoters (that increase levels of transcription in response to absence of a repressor). Inducible promoters may initiate increased levels of transcription from DNA under their control in response to some change in culture conditions, e.g., the presence or absence of a nutrient or a change in temperature.

The yeast promoter fragment may also serve as the site for homologous recombination and integration of the expression vector into the same site in the yeast genome; alternatively a selectable marker is used as the site for homologous recombination. Pichia transformation is described in Cregg et al. (1985) Mol. Cell. Biol. 5:3376-3385.

Examples of suitable promoters from *Pichia* include the CUP1 (induced by the level of copper in the medium), tetracycline inducible promoters, thiamine inducible promoters, AOX1 promoter (Cregg et al. (1989) Mol. Cell. Biol. 9:1316-1323); ICL1 promoter (Menendez et al. (2003) Yeast 20(13):1097-108); glyceraldehyde-3-phosphate dehydrogenase promoter (GAP) (Waterham et al. (1997) Gene 186(1):37-44); and FLD1 promoter (Shen et al. (1998) Gene 216(1):93-102). The GAP promoter is a strong constitutive promoter and the CUP1, AOX and FLD1 promoters are inducible.

Other yeast promoters include ADH1, alcohol dehydrogenase II, GAL4, PHO3, PHO5, Pyk, and chimeric promoters derived therefrom. Additionally, non-yeast promoters may be used such as mammalian, insect, plant, reptile, amphibian, viral, and avian promoters. Most typically the promoter will comprise a mammalian promoter (potentially endogenous to the expressed genes) or will comprise a yeast or viral promoter that provides for efficient transcription in yeast systems.

The polypeptides of interest may be produced recombinantly not only directly, but also as a fusion polypeptide with a heterologous polypeptide, e.g. a signal sequence or other polypeptide having a specific cleavage site at the N-terminus of the mature protein or polypeptide. In general, the signal sequence may be a component of the vector, or it may be a part of the polypeptide coding sequence that is inserted into the vector. The heterologous signal sequence selected preferably is one that is recognized and processed through one of the standard pathways available within the host cell. The *S. cerevisiae* alpha factor pre-pro signal has proven effective in the secretion of a variety of recombinant proteins from *P. pastoris.* Other yeast signal sequences include the alpha mating factor signal sequence, the invertase signal sequence, and signal sequences derived from other secreted yeast polypeptides. Additionally, these signal peptide sequences may be engineered to provide for enhanced secretion in diploid yeast expression systems. Other secretion signals of interest also include mammalian signal sequences, which may be heterologous to the protein being secreted, or may be a native sequence for the protein being secreted. Signal sequences include pre-peptide sequences, and in some instances may include propeptide sequences. Many such signal sequences are known in the art, including the signal sequences found on immunoglobulin chains, e.g., K28 preprotoxin sequence, PHA-E, FACE, human MCP-1, human serum albumin signal sequences, human Ig heavy chain, human Ig light chain, and the like. For example, see Hashimoto et. al. Protein Eng 11(2) 75 (1998); and Kobayashi et. al. Therapeutic Apheresis 2(4) 257 (1998).

Transcription may be increased by inserting a transcriptional activator sequence into the vector. These activators are cis-acting elements of DNA, usually about from 10 to 300 bp, which act on a promoter to increase its transcription. Transcriptional enhancers are relatively orientation and position independent, having been found 5' and 3' to the transcription unit, within an intron, as well as within the coding sequence itself. The enhancer may be spliced into the expression vector at a position 5' or 3' to the coding sequence, but is preferably located at a site 5' from the promoter.

Expression vectors used in eukaryotic host cells may also contain sequences necessary for the termination of transcription and for stabilizing the mRNA. Such sequences are commonly available from 3' to the translation termination codon, in untranslated regions of eukaryotic or viral DNAs or cDNAs. These regions contain nucleotide segments transcribed as polyadenylated fragments in the untranslated portion of the mRNA.

Construction of suitable vectors containing one or more of the above-listed components employs standard ligation techniques or PCR/recombination methods. Isolated plasmids or DNA fragments are cleaved, tailored, and re-ligated in the form desired to generate the plasmids required or via recombination methods. For analysis to confirm correct sequences in plasmids constructed, the ligation mixtures are used to transform host cells, and successful transformants selected by antibiotic resistance (e.g. ampicillin or Zeocin) where appropriate. Plasmids from the transformants are prepared, analyzed by restriction endonuclease digestion and/or sequenced.

As an alternative to restriction and ligation of fragments, recombination methods based on att sites and recombination enzymes may be used to insert DNA sequences into a vector. Such methods are described, for example, by Landy (1989) Ann. Rev. Biochem. 58:913-949; and are known to those of skill in the art. Such methods utilize intermolecular DNA recombination that is mediated by a mixture of lambda and E. coli-encoded recombination proteins. Recombination occurs between specific attachment (att) sites on the interacting DNA molecules. For a description of att sites see Weisberg and Landy (1983) Site-Specific Recombination in Phage Lambda, in Lambda II, Weisberg, ed. (Cold Spring Harbor, N.Y.: Cold Spring Harbor Press), pp. 211-250. The DNA segments flanking the recombination sites are switched, such that after recombination, the att sites are hybrid sequences comprised of sequences donated by each parental vector. The recombination can occur between DNAs of any topology.

Att sites may be introduced into a sequence of interest by ligating the sequence of interest into an appropriate vector; generating a PCR product containing att B sites through the use of specific primers; generating a cDNA library cloned into an appropriate vector containing att sites; and the like.

*Monocistronic and polycistronic genes.* A monocistronic gene encodes an RNA that contains the genetic information to translate only a single protein. A polycistronic gene encodes an mRNA that contains the genetic information to translate more than one protein. The proteins encoded in a polycistronic gene may have the same or different sequences or a combination thereof. Dicistronic or bicistronic refers to a polycistronic gene that encodes two proteins. Polycistronic genes optionally include one or more internal ribosome entry site (IRES) elements to facilitate cap-independent initiation of translation, which may be situated at a location that can drive translation of the downstream protein coding region independently of the 5'-cap structure bound to the 5' end of the mRNA molecule. Any known IRES sequence (e.g., viral, eukaryotic, or artificial in origin) may be used. For example, the cricket paralysis virus IRES sequence in the intergenic region (IGR) may be used, as described in Thompson et al. (2001) PNAS 98:12972-12977. Optionally, IRES function may be potentiated by genetic alteration, e.g., by causing constitutive expression of eIF2 kinase GCN2 or disrupting two initiator tRNA(met) genes disrupted (*id.*).

Folding, as used herein, refers to the three-dimensional structure of polypeptides and proteins, where interactions between amino acid residues act to stabilize the structure. While non-covalent interactions are important in determining structure, usually the proteins of interest will have intra- and/or intermolecular covalent disulfide bonds formed by two cysteine residues. For naturally occurring proteins and polypeptides or derivatives and variants thereof, the proper folding is typically the arrangement that results in optimal biological activity, and can conveniently be monitored by assays for activity, e.g. ligand binding, enzymatic activity, etc.

In some instances, for example where the desired product is of synthetic origin, assays based on biological activity will be less meaningful. The proper folding of such molecules may be determined on the basis of physical properties, energetic considerations, modeling studies, and the like.

The expression host may be further modified by the introduction of sequences encoding one or more enzymes that enhance folding and disulfide bond formation, i.e. foldases, chaperoning, etc. Such sequences may be constitutively or inducibly expressed in the yeast host cell, using vectors, markers, etc. as known in the art. Preferably the sequences, including transcriptional regulatory elements sufficient for the desired pattern of expression, are stably integrated in the yeast genome through a targeted methodology.

For example, the eukaryotic PDI is not only an efficient catalyst of protein cysteine oxidation and disulfide bond isomerization, but also exhibits chaperone activity. Co-expression of PDI can facilitate the production of active proteins having multiple disulfide bonds. Also of interest is the expression of BIP (immunoglobulin heavy chain binding protein); cyclophilin; and the like. In one example, the multi-subunit complex may be expressed from a yeast strain produced by mating, wherein each of the haploid parental strains expresses a distinct folding enzyme, e.g. one strain may express BIP, and the other strain may express PDI or combinations thereof.

The terms "desired protein" or "target protein" are used interchangeably and refer generally to a heterologous multi-subunit protein such as a humanized antibody or a binding portion thereof described herein.

The term "antibody" includes any polypeptide chain-containing molecular structure with a specific shape that fits to and recognizes an epitope, where one or more non-covalent binding interactions stabilize the complex between the molecular structure and the epitope. The archetypal antibody molecule is the immunoglobulin, and all types of immunoglobulins, IgG, IgM, IgA, IgE, IgD, etc., from all sources, e.g. human, rodent, rabbit, cow, sheep, pig, dog, other mammals, chicken, other avians, etc., are considered to be "antibodies." A preferred source for producing antibodies useful as starting material is rabbits. Numerous antibody coding sequences have been described; and others may be raised by methods well-known in the art. Examples thereof include chimeric antibodies, human antibodies and other non-human mammalian antibodies, humanized antibodies, single chain antibodies such as scFvs, camelbodies, nanobodies, IgNAR (single-chain antibodies derived from sharks), small-modular immunopharmaceuticals (SMIPs), and antibody fragments such as Fabs, Fab', F(ab')₂ and the like. See Streltsov V A, et al., Structure of a shark IgNAR antibody variable domain and modeling of an early-developmental isotype, Protein Sci. 2005 November; 14(11):2901-9. Epub 2005 Sep. 30; Greenberg A S, et al., A new antigen receptor gene family that undergoes rearrangement and extensive somatic diversification in sharks, Nature. 1995 Mar. 9; 374(6518):168-73; Nuttall S D, et al., Isolation of the new antigen receptor from wobbegong sharks, and use as a scaffold for the display of protein loop libraries, Mol Immunol. 2001 August; 38(4):313-26; Hamers-Casterman C, et al., Naturally occurring antibodies devoid of light chains, Nature. 1993 Jun. 3; 363(6428):446-8; Gill D S, et al., Biopharmaceutical drug discovery using novel protein scaffolds, Curr Opin Biotechnol. 2006 December; 17(6):653-8. Epub 2006 Oct. 19.

For example, antibodies or antigen binding fragments may be produced by genetic engineering. In this technique, as with other methods, antibody-producing cells are sensitized to the desired antigen or immunogen. The messenger RNA isolated from antibody producing cells is used as a template to make cDNA using PCR amplification. A library of vectors, each containing one heavy chain gene and one light chain gene retaining the initial antigen specificity, is produced by insertion of appropriate sections of the amplified immunoglobulin cDNA into the expression vectors. A combinatorial library is constructed by combining the heavy chain gene library with the light chain gene library. This results in a library of clones which co-express a heavy and light chain (resembling the Fab fragment or antigen binding fragment of an antibody molecule). The vectors that carry these genes are co-transfected into a host cell. When antibody gene synthesis is induced in the transfected host, the heavy and light chain proteins self-assemble to produce active antibodies that can be detected by screening with the antigen or immunogen.

Antibody coding sequences of interest include those encoded by native sequences, as well as nucleic acids that, by virtue of the degeneracy of the genetic code, are not identical in sequence to the disclosed nucleic acids, and variants thereof. Variant polypeptides can include amino acid (aa) substitutions, additions or deletions. The amino acid substitutions can be conservative amino acid substitutions or substitutions to eliminate non-essential amino acids, such as to alter a glycosylation site, or to minimize misfolding by substitution or deletion of one or more cysteine residues that are not necessary for function. Variants can be designed so as to retain or have enhanced biological activity of a particular region of the protein (e.g., a functional domain, catalytic amino acid residues, etc). Variants also include fragments of the polypeptides disclosed herein, particularly biologically active fragments and/or fragments corresponding to functional domains. Techniques for in vitro mutagenesis of cloned genes are known. Also included in the present disclosure are polypeptides that have been modified using ordinary molecular biological techniques so as to improve their resistance to proteolytic degradation or to optimize solubility properties or to render them more suitable as a therapeutic agent.

Chimeric antibodies may be made by recombinant means by combining the variable light and heavy chain regions (V_{L} and V_{H}), obtained from antibody producing cells of one species with the constant light and heavy chain regions from another. Typically chimeric antibodies utilize rodent or rabbit variable regions and human constant regions, in order to produce an antibody with predominantly human domains. The production of such chimeric antibodies is well known in the art, and may be achieved by standard means (as described, e.g., in U.S. Pat. No. 5,624,659). It is further contemplated that the human constant regions of chimeric antibodies may be selected from IgG1, IgG2, IgG3, or IgG4, constant regions.

Humanized antibodies are engineered to contain even more human-like immunoglobulin domains, and incorporate only the complementarity-determining regions of the animal-derived antibody. This is accomplished by carefully examining the sequence of the hyper-variable loops of the variable regions of the monoclonal antibody, and fitting them to the structure of the human antibody chains. Although facially complex, the process is straightforward in practice. See, e.g., U.S. Pat. No. 6,187,287. Methods of humanizing antibodies have been described previously in issued U.S. Patent No. 7935340. In some instances, a determination of whether additional rabbit framework residues are required to maintain activity is necessary. In some instances the humanized antibodies still requires some critical rabbit framework residues to be retained to minimize loss of affinity or activity. In these cases, it is necessary to change single or multiple framework amino acids from human germline sequences back to the original rabbit amino acids in order to have desired activity. These changes are determined experimentally to identify which rabbit residues are necessary to preserve affinity and activity.

In addition to entire immunoglobulins (or their recombinant counterparts), immunoglobulin fragments comprising the epitope binding site (e.g., Fab', F(ab')₂, or other fragments) may be synthesized. "Fragment," or minimal immunoglobulins may be designed utilizing recombinant immunoglobulin techniques. For instance "Fv" immunoglobulins for use in the present disclosure may be produced by synthesizing a fused variable light chain region and a variable heavy chain region. Combinations of antibodies are also of interest, e.g. diabodies, which comprise two distinct Fv specificities. In another example, SMIPs (small molecule immunopharmaceuticals), camelbodies, nanobodies, and IgNAR are encompassed by immunoglobulin fragments.

Immunoglobulins and fragments thereof may be modified post-translationally, e.g. to add effector moieties such as chemical linkers, detectable moieties, such as fluorescent dyes, enzymes, toxins, substrates, bioluminescent materials, radioactive materials, chemiluminescent moieties and the like, or specific binding moieties, such as streptavidin, avidin, or biotin, and the like may be utilized in the methods and compositions of the present description. Examples of additional effector molecules are provided infra.

*Product-associated variant*: a product other than the desired product (e.g., the desired multi-subunit complex) which is present in a preparation of the desired product and related to the desired product. Exemplary product-associated variants include truncated or elongated peptides, products having different glycosylation than the desired glycosylation (e.g., if an aglycosylated product is desired then any glycosylated product would be considered to be a product-associated variant), complexes having abnormal stoichiometry, improper assembly, abnormal disulfide linkages, abnormal or incomplete folding, aggregation, protease cleavage, or other abnormalities. Exemplary product-associated variants may exhibit alterations in one or more of molecular mass (e.g., detected by size exclusion chromatography), isoelectric point (e.g., detected by isoelectric focusing), electrophoretic mobility (e.g., detected by gel electrophoresis), phosphorylation state (e.g., detected by mass spectrometry), charge to mass ratio (e.g., detected by mass spectrometry), mass or identity of proteolytic fragments (e.g., detected by mass spectrometry or gel electrophoresis), hydrophobicity (e.g., detected by HPLC), charge (e.g., detected by ion exchange chromatography), affinity (*e.g.,* in the case of an antibody, detected by binding to protein A, protein G, and/or an epitope to which the desired antibody binds), and glycosylation state (e.g., detected by lectin binding affinity). Where the desired protein is an antibody, the term product-associate variant may include a glyco-heavy variant and/or half antibody species (described below).

Exemplary product-associated variants include variant forms that contain aberrant disulfide bonds. For example, most IgG1 antibody molecules are stabilized by a total of 16 intra-chain and inter-chain disulfide bridges, which stabilize the folding of the IgG domains in both heavy and light chains, while the inter-chain disulfide bridges stabilize the association between heavy and light chains. Other antibody types likewise contain characteristic stabilizing intra-chain and inter-chain disulfide bonds. Further, some antibodies (including Ab-A and Ab-B disclosed herein) contain additional disulfide bonds referred to as non-canonical disulfide bonds. Thus, aberrant inter-chain disulfide bonds may result in abnormal complex stoichiometry, due to the absence of a stabilizing covalent linkage, and/or disulfide linkages to additional subunits. Additionally, aberrant disulfide bonds (whether inter-chain or intra-chain) may decrease structural stability of the antibody, which may result in decreased activity, decreased stability, increased propensity to form aggregates, and/or increased immunogenicity. Product-associated variants containing aberrant disulfide bonds may be detected in a variety of ways, including non-reduced denaturing SDS-PAGE, capillary electrophoresis, cIEX, mass spectrometry (optionally with chemical modification to produce a mass shift in free cysteines), size exclusion chromatography, HPLC, changes in light scattering, and any other suitable methods known in the art. *See, e.g.,* The Protein Protocols Handbook 2002, Part V, 581-583, DOI: 10.1385/1-59259-169-8:581.

*Half antibody, half-antibody species,* or *H1L1* refer to a protein complex that includes a single heavy and single light antibody chain, but lacks a covalent linkage to a second heavy and light antibody chain. Two half antibodies may remain non-covalently associated under some conditions (which may give behavior similar to a full antibody, e.g., apparent molecular weight determined by size exclusion chromatography). Similarly, *H2L1* refers to a protein complex that includes two heavy antibody chains and single light antibody chain, but lacks a covalent linkage to a second light antibody chain; these complexes may also non-covalently associate with another light antibody chain (and likewise give similar behavior to a full antibody). Like full antibodies, half antibody species and H2L1 species can dissociate under reducing conditions into individual heavy and light chains. Half antibody species and H2L1 species can be detected on a non-reduced SDS-PAGE gel as a species migrating at a lower apparent molecular weight than the full antibody, e.g., H1L1 migrates at approximately half the apparent molecular weight of the full antibody (e.g., about 75 kDa).

*Glyco-heavy variant* refers to a glycosylated product-associated variant sometimes present in antibody preparations and which contains at least a partial Fc sequence. The glyco-heavy variant is characterized by decreased electrophoretic mobility observable by SDS-PAGE (relative to a normal heavy chain), lectin binding affinity, binding to an anti-Fc antibody, and apparent higher molecular weight of antibody complexes containing the glyco-heavy variant as determined by size exclusion chromatography.

The term "polyploid yeast that stably expresses or expresses a desired secreted heterologous polypeptide for prolonged time" refers to a yeast culture that secretes said polypeptide for at least several days to a week, more preferably at least a month, still more preferably at least 1-6 months, and even more preferably for more than a year at threshold expression levels, typically at least 50-500 mg/liter (after about 90 hours in culture) and preferably substantially greater.

The term "polyploidal yeast culture that secretes desired amounts of recombinant polypeptide" refers to cultures that stably or for prolonged periods secrete at least at least 50-500 mg/liter, and most preferably 500-1000 mg/liter or more.

A polynucleotide sequence "corresponds" to a polypeptide sequence if translation of the polynucleotide sequence in accordance with the genetic code yields the polypeptide sequence (i.e., the polynucleotide sequence "encodes" the polypeptide sequence), one polynucleotide sequence "corresponds" to another polynucleotide sequence if the two sequences encode the same polypeptide sequence.

A "heterologous" region or domain of a DNA construct is an identifiable segment of DNA within a larger DNA molecule that is not found in association with the larger molecule in nature. Thus, when the heterologous region encodes a mammalian gene, the gene will usually be flanked by DNA that does not flank the mammalian genomic DNA in the genome of the source organism. Another example of a heterologous region is a construct where the coding sequence itself is not found in nature (e.g., a cDNA where the genomic coding sequence contains introns, or synthetic sequences having codons different than the native gene). Allelic variations or naturally-occurring mutational events do not give rise to a heterologous region of DNA as defined herein.

A "coding sequence" is an in-frame sequence of codons that (in view of the genetic code) correspond to or encode a protein or peptide sequence. Two coding sequences correspond to each other if the sequences or their complementary sequences encode the same amino acid sequences. A coding sequence in association with appropriate regulatory sequences may be transcribed and translated into a polypeptide. A polyadenylation signal and transcription termination sequence will usually be located 3' to the coding sequence. A "promoter sequence" is a DNA regulatory region capable of binding RNA polymerase in a cell and initiating transcription of a downstream (3' direction) coding sequence. Promoter sequences typically contain additional sites for binding of regulatory molecules (e.g., transcription factors) which affect the transcription of the coding sequence. A coding sequence is "under the control" of the promoter sequence or "operatively linked" to the promoter when RNA polymerase binds the promoter sequence in a cell and transcribes the coding sequence into mRNA, which is then in turn translated into the protein encoded by the coding sequence.

Vectors are used to introduce a foreign substance, such as DNA, RNA or protein, into an organism or host cell. Typical vectors include recombinant viruses (for polynucleotides) and liposomes (for polypeptides). A "DNA vector" is a replicon, such as plasmid, phage or cosmid, to which another polynucleotide segment may be attached so as to bring about the replication of the attached segment. An "expression vector" is a DNA vector which contains regulatory sequences which will direct polypeptide synthesis by an appropriate host cell. This usually means a promoter to bind RNA polymerase and initiate transcription of mRNA, as well as ribosome binding sites and initiation signals to direct translation of the mRNA into a polypeptide(s). Incorporation of a polynucleotide sequence into an expression vector at the proper site and in correct reading frame, followed by transformation of an appropriate host cell by the vector, enables the production of a polypeptide encoded by said polynucleotide sequence.

"Amplification" of polynucleotide sequences is the in vitro production of multiple copies of a particular nucleic acid sequence. The amplified sequence is usually in the form of DNA. A variety of techniques for carrying out such amplification are described in the following review articles: Van Brunt 1990, Bio/Technol., 8(4):291-294; and Gill and Ghaemi, Nucleosides Nucleotides Nucleic Acids. 2008 Mar;27(3):224-43. Polymerase chain reaction or PCR is a prototype of nucleic acid amplification, and use of PCR herein should be considered exemplary of other suitable amplification techniques.

The general structure of antibodies in most vertebrates (including mammals) is now well understood (Edelman, G. M., Ann. N.Y. Acad. Sci., 190: 5 (1971)). Conventional antibodies consist of two identical light polypeptide chains of molecular weight approximately 23,000 daltons (the "light chain"), and two identical heavy chains of molecular weight 53,000-70,000 (the "heavy chain"). The four chains are joined by disulfide bonds in a "Y" configuration wherein the light chains bracket the heavy chains starting at the mouth of the "Y" configuration. The "branch" portion of the "Y" configuration is designated the F_{ab} region; the stem portion of the "Y" configuration is designated the Fc region. The amino acid sequence orientation runs from the N-terminal end at the top of the "Y" configuration to the C-terminal end at the bottom of each chain. The N-terminal end possesses the variable region having specificity for the antigen that elicited it, and is approximately 100 amino acids in length, there being slight variations between light and heavy chain and from antibody to antibody.

The variable region is linked in each chain to a constant region that extends the remaining length of the chain and that within a particular class of antibody does not vary with the specificity of the antibody (i.e., the antigen eliciting it). There are five known major classes of constant regions that determine the class of the immunoglobulin molecule (IgG, IgM, IgA, IgD, and IgE corresponding to gamma, mu, alpha, delta, and epsilon heavy chain constant regions). The constant region or class determines subsequent effector function of the antibody, including activation of complement (Kabat, E. A., Structural Concepts in Immunology and Immunochemistry, 2nd Ed., p. 413-436, Holt, Rinehart, Winston (1976)), and other cellular responses (Andrews, D. W., et al., Clinical Immunobiology, pp 1-18, W. B. Sanders (1980); Kohl, S., et al., Immunology, 48: 187 (1983)); while the variable region determines the antigen with which it will react. Light chains are classified as either kappa or lambda. Each heavy chain class can be paired with either kappa or lambda light chain. The light and heavy chains are covalently bonded to each other, and the "tail" portions of the two heavy chains are bonded to each other by covalent disulfide linkages when the immunoglobulins are generated either by hybridomas or by B cells.

The expression "variable region" or "VR" refers to the domains within each pair of light and heavy chains in an antibody that are involved directly in binding the antibody to the antigen. Each heavy chain has at one end a variable domain (V_{H}) followed by a number of constant domains. Each light chain has a variable domain (V_{L}) at one end and a constant domain at its other end; the constant domain of the light chain is aligned with the first constant domain of the heavy chain, and the light chain variable domain is aligned with the variable domain of the heavy chain.

The expressions "complementarity determining region," "hypervariable region," or "CDR" refer to one or more of the hyper-variable or complementarity determining regions (CDRs) found in the variable regions of light or heavy chains of an antibody (See Kabat, E. A. et al., Sequences of Proteins of Immunological Interest, National Institutes of Health, Bethesda, Md., (1987)). These expressions include the hypervariable regions as defined by Kabat et al. ("Sequences of Proteins of Immunological Interest," Kabat E., et al., US Dept. of Health and Human Services, 1983) or the hypervariable loops in 3-dimensional structures of antibodies (Chothia and Lesk, J Mol. Biol. 196 901-917 (1987)). The CDRs in each chain are held in close proximity by framework regions and, with the CDRs from the other chain, contribute to the formation of the antigen binding site. Within the CDRs there are select amino acids that have been described as the selectivity determining regions (SDRs) which represent the critical contact residues used by the CDR in the antibody-antigen interaction (Kashmiri, S., Methods, 36:25-34 (2005)).

The expressions "framework region" or "FR" refer to one or more of the framework regions within the variable regions of the light and heavy chains of an antibody (See Kabat, E. A. et al., Sequences of Proteins of Immunological Interest, National Institutes of Health, Bethesda, Md., (1987)). These expressions include those amino acid sequence regions interposed between the CDRs within the variable regions of the light and heavy chains of an antibody.

The expression "stable copy number" refers to a host cell that substantially maintains the number of copies of a gene (such as an antibody chain gene) over a prolonged period of time (such as at least a day, at least a week, or at least a month, or more) or over a prolonged number of generations of propagation (e.g., at least 30, 40, 50, 75, 100, 200, 500, or 1000 generations, or more). For example, at a given time point or number of generations, at least 50%, and preferably at least 70%, 75%, 85%, 90%, 95%, or more of cells in the culture may maintain the same number of copies of the gene as in the starting cell. In a preferred example, the host cell contains a stable copy number of each subunit of the desired multi-subunit complex (e.g., antibody).

The expression "stably expresses" refers to a host cell that maintains similar levels of expression of a gene or protein (such as an antibody) over a prolonged period of time (such as at least a day, at least a week, or at least a month, or more) or over a prolonged number of generations of propagation (e.g., at least 30, 40, 50, 75, 100, 200, 500, or 1000 generations, or more). For example, at a given time point or number of generations, the rate of production or yield of the gene or protein may be at least 50%, and preferably at least 70%, 75%, 85%, 90%, 95%, or more of the initial rate of production. In a preferred example, the host cell stably expresses the desired multi-subunit complex (e.g., antibody).

### EXAMPLES

The following examples are put forth so as to provide those of ordinary skill in the art with a complete disclosure and description of how to make and use the subject invention, and are not intended to limit the scope of what is regarded as the invention. The scope of the invention is defined by the claims. Efforts have been made to ensure accuracy with respect to the numbers used (e.g. amounts, temperature, concentrations, etc.) but some experimental errors and deviations should be allowed for. Unless otherwise indicated, parts are parts by weight, molecular weight is average molecular weight, temperature is in degrees centigrade; and pressure is at or near atmospheric.

### EXAMPLE 1 - Reference Example

### Increased Antibody Yield by Varying Heavy and Light Chain Gene Copy Number

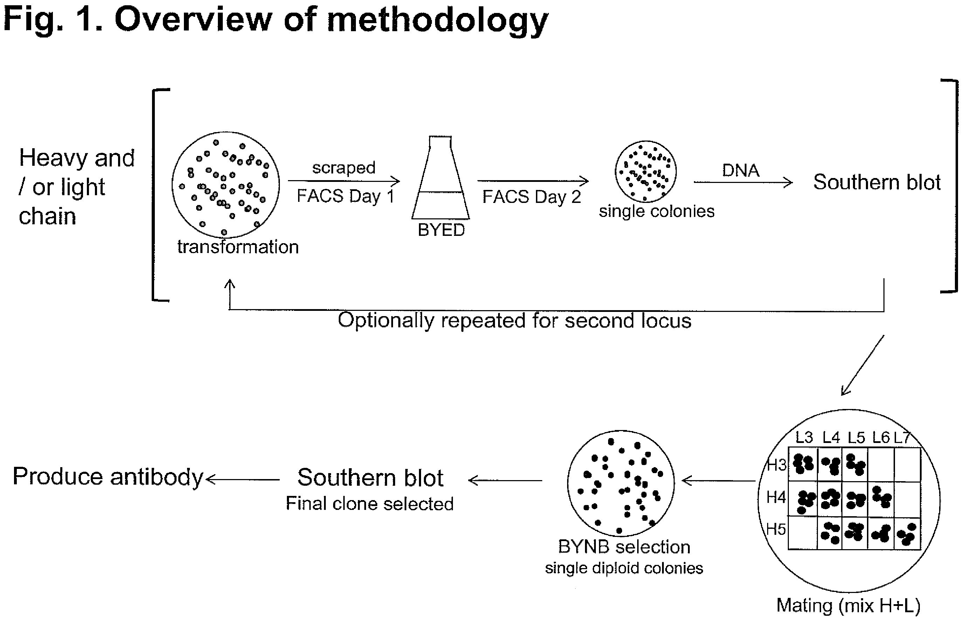

This example demonstrates that yield of recombinant antibodies produced in *P. pastoris* can be greatly increased by varying the number of copies of the heavy and light chain genes. In particular, this example demonstrates that the present methods permit targeted matings that yield such strains. A panel of diploid *P. pastoris* strains each expressing humanized antibodies from a differing number of copies of the heavy and light chain genes was generated and tested to identify combinations of gene copy numbers that produced a greater antibody yield. **FIG. 1** provides an overview of the methods used to efficiently generate the panel of diploid strains (detailed methods are provided in Example 4 below). In brief, haploid strains were transformed with genes encoding either the heavy or light chain genes under the control of a promoter fused to a secretion signal. To direct integration into a specific genetic locus within the *P. pastoris* genome, the plasmid was linearized within the homologous sequence of the targeted locus. In this example, the constructs were integrated into the pGAP, 3'AOX TT and HIS4 TT loci, though other loci could also be used. Transformants containing multiple tandemly integrated copies of the antibody chain gene were identified by Southern blot, and haploid strains containing defined numbers of copies of the light or heavy chain genes were selected for further use. Optionally, additional copies of the same antibody chain gene were integrated into a second locus in the haploid strain. Mating of these haploid strains efficiently produced diploid strains containing defined numbers of light and heavy chain genes in varying combinations. After mating, gene copy numbers in the diploid strains were verified by Southern blotting.

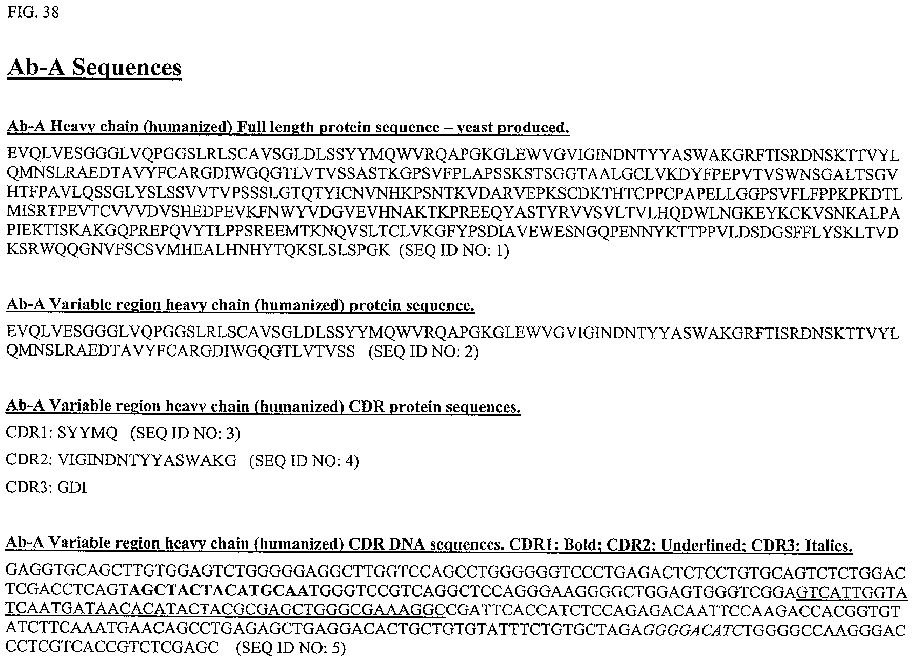

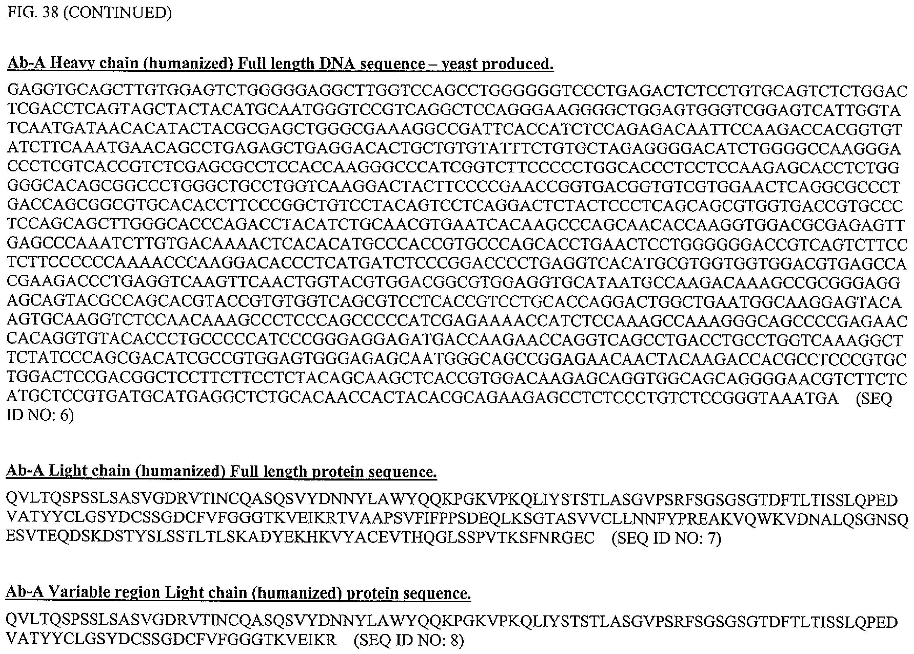

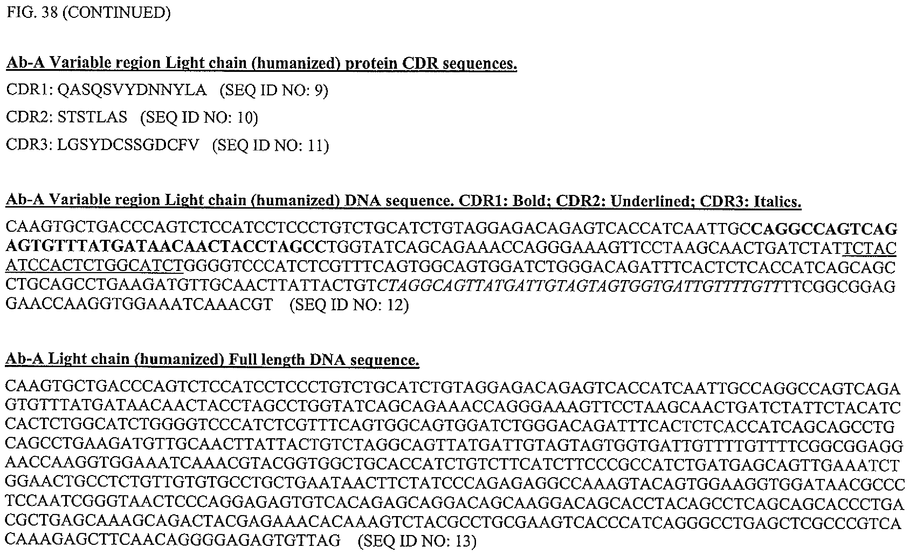

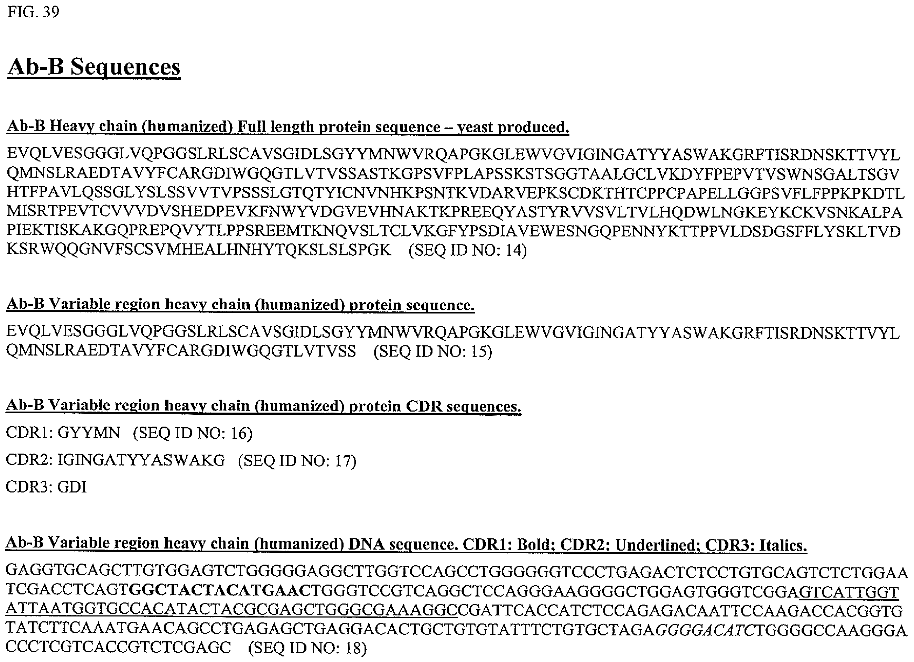

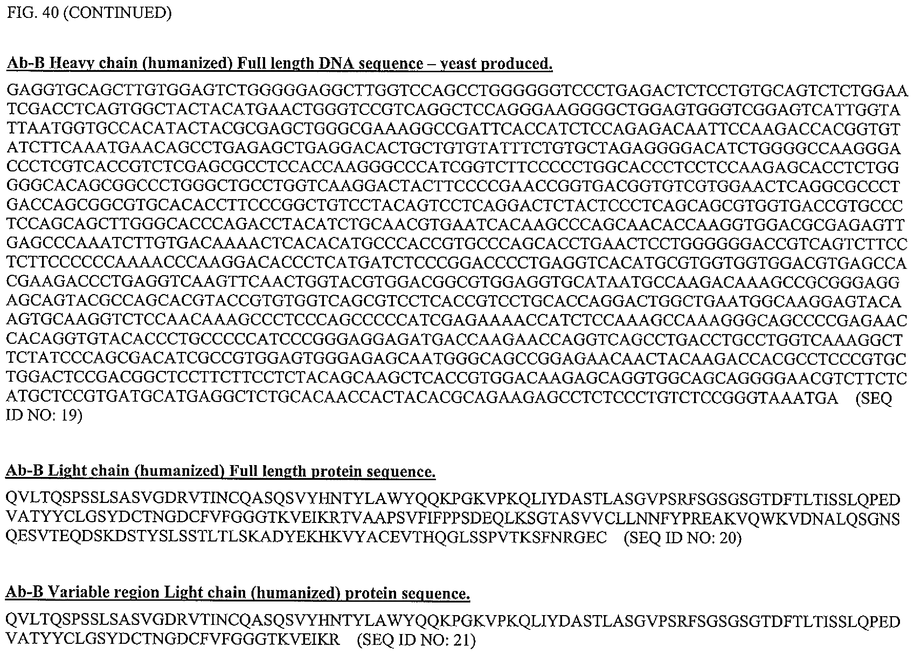

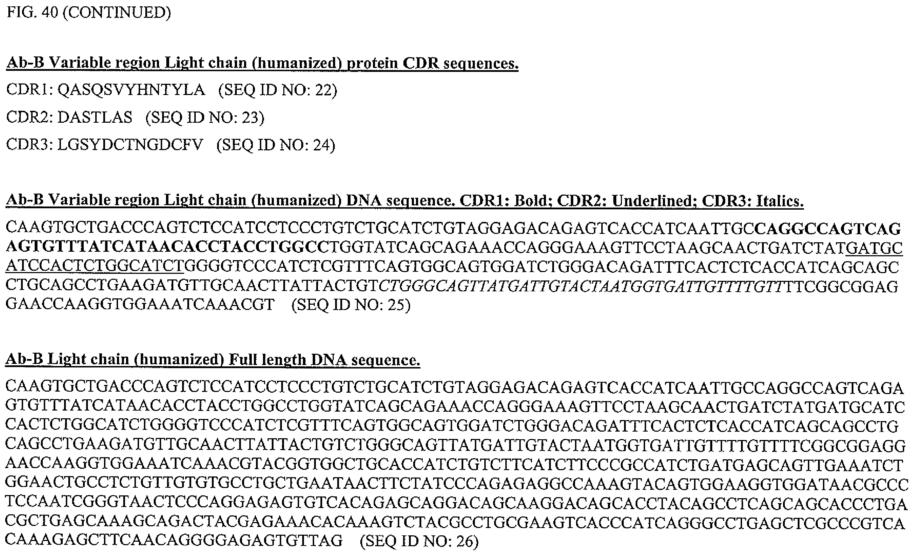

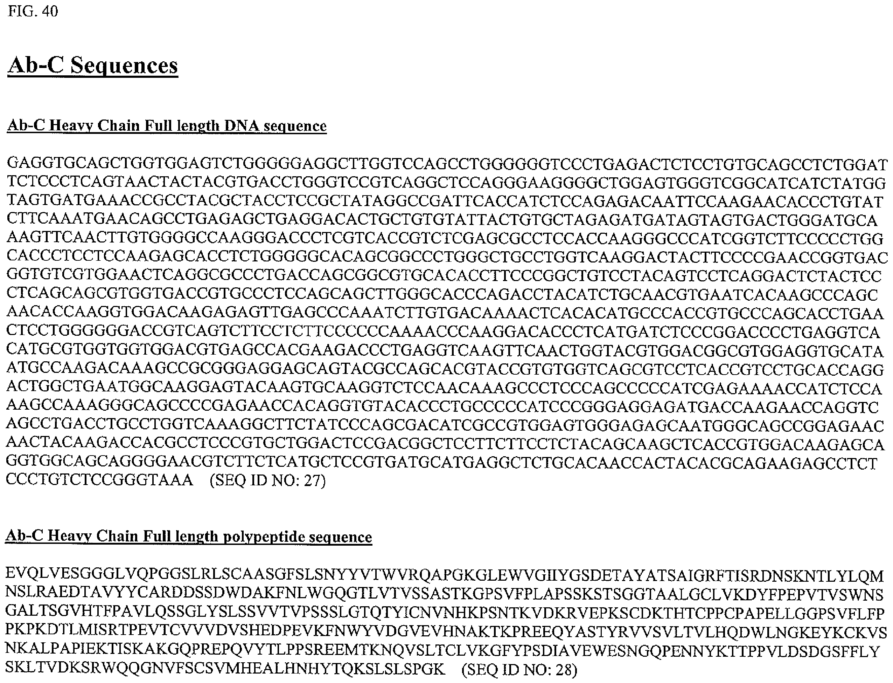

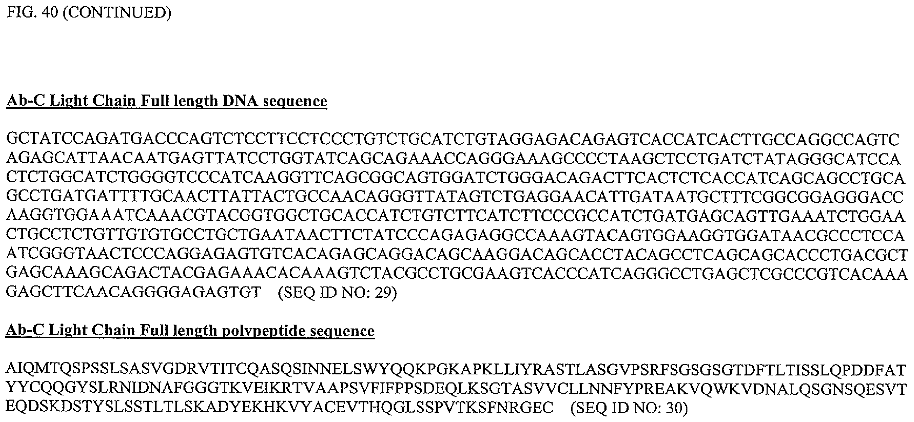

Using these methods, diploid *P. pastoris* strains were produced containing heavy and light chain genes encoding three humanized antibodies, Ab-A and Ab-B, and Ab-C. The antibody polypeptide and polynucleotide sequences are shown in **FIG. 38** (Ab-A), **FIG. 39** (Ab-B, and **FIG. 40** (Ab-C)). Ab-A, Ab-B and Ab-C are humanized antibodies that were derived from three different rabbit antibodies. Ab-C is specific for a different antigen than Ab-A and Ab-B.

The diploid strains are summarized in Tables 1, 2, and 3 below. The prefix of each strain identifier indicates the antibody produced, and the numbers following H and L refer to the number of integrated copies of the heavy and light chain genes, respectively. For example, the strain designator Ab-A-H3xL4 identifies a strain that expresses Ab-A and contains three copies of the heavy chain gene and four copies of the light chain gene. Columns labeled pGAP, 3'AOX TT and HIS4 TT indicate the number of gene copies integrated at each respective locus. Each locus is listed twice to reflect integration into homologous chromosomes (one originating from each parental haploid strain).

Selected diploid strains were cultured in a bioreactor under conditions that generated antibody production and secretion as described in Example 5. Each antibody chain was under control of the GAP promoter, whose expression was upregulated by switching from a glycerol carbon source to a glucose carbon source under conditions that converted some of the glucose to ethanol (low oxygen availability). An in-frame fusion of each antibody chain gene to a secretion sequence caused secretion of the expressed antibodies into the culture media. Culture media was harvested at approximately 90 hours of growth (T90) and antibody yield was determined by high performance liquid chromatography (HPLC) as described in Example 6.

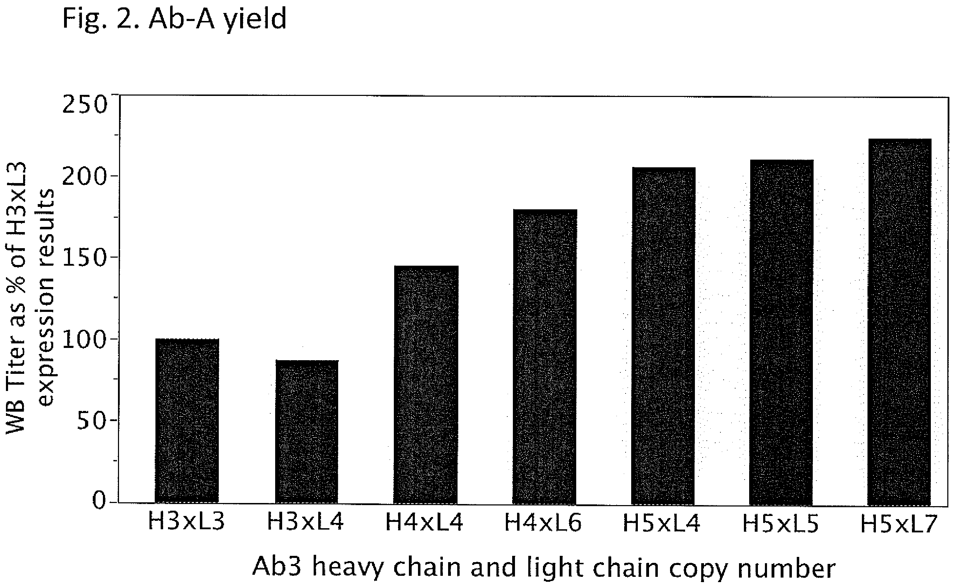

Relative antibody yields from selected Ab-A-expressing strains at T90 are shown in the rightmost column of Table 1, below, and illustrated graphically in **FIG. 2****.** The H3xL3 strain was used as a reference and its expression yield was set to 100%. Whole broth antibody titer generally increased with antibody copy number, in the order Ab-A-H3xL4, Ab-A-H3xL3, Ab-A-H4xL4, Ab-A-H4xL6, Ab-A-H5xL4, Ab-A-H5xL5, and Ab-A-H5xL7. Yield from all three Ab-A-H5 strains exceeded that from both Ab-A-H4 strains, which exceeded that from the two Ab-A-H3 strains. For a given heavy chain copy number, total yield also increased with increasing light chain copy number, with the exception that the yield from H3xL4 was about 13% lower than the yield from H3xL3.

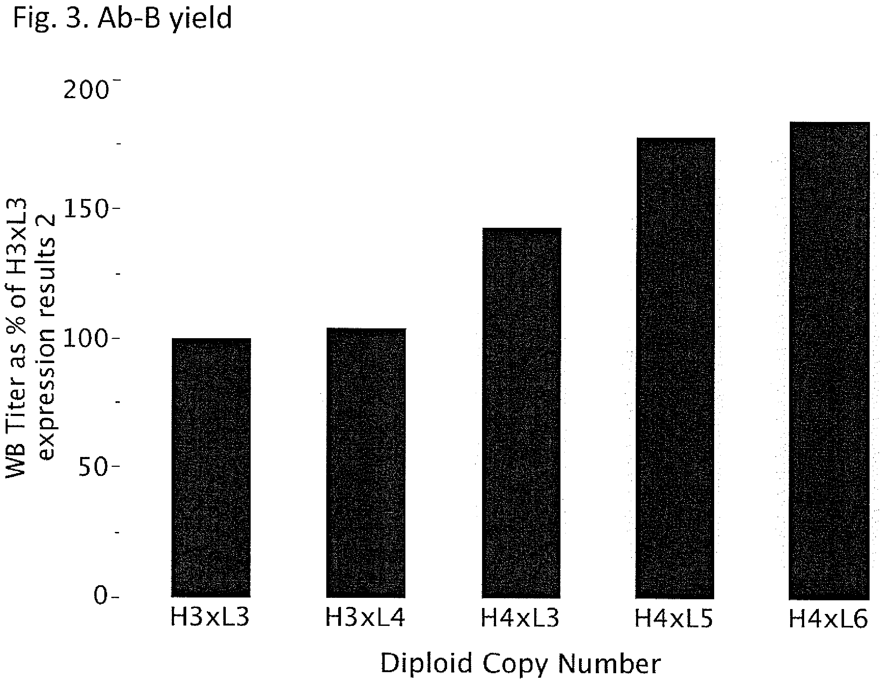

Similar yield results were obtained from strains expressing Ab-B, which are shown in the rightmost column of Table 2, below, and illustrated graphically in **FIG. 3****.** The H3xL3 strain was used as a reference and its expression yield was set to 100%. As with Ab-A, yield of Ab-B generally increased with antibody copy number, in the order Ab-B-H3xL3, Ab-B-H3xL4, Ab-B-H4xL3, Ab-B-H4xL5, and Ab-B-H4xL6. Yield from all three Ab-B-H4 strains exceeded that from both Ab-B-H3 strains.

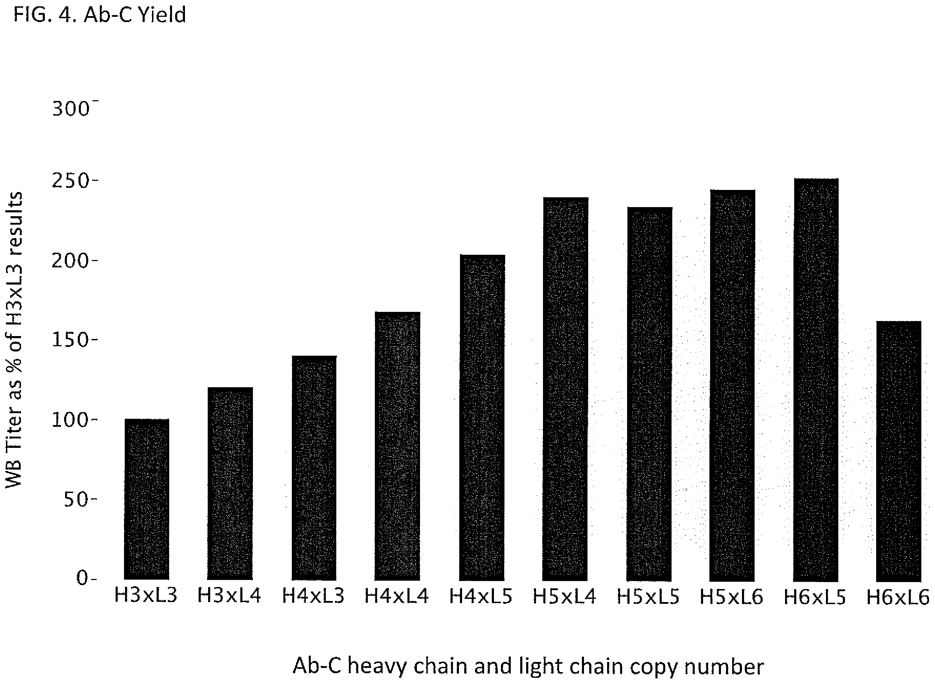

Increasing antibody copy number likewise generally increased the yield of antibody Ab-C, as shown in the rightmost column of Table 3 below and illustrated graphically in **FIG. 4****.** Antibody yields generally increased with antibody copy number in the order Ab-C-H3xL4, Ab-C-H4xL3, Ab-C-H4xL4, Ab-C-H4xL5, Ab-C-H5xL5, Ab-C-H5xL4, Ab-C-H5xL6, and Ab-C-H6xL5. Yield increases were relatively modest between strains having 5 heavy chain copies or more. Additionally, the Ab-C-H6xL6 strain exhibited a substantial decrease in yield relative to the Ab-C-H6xL5 and Ab-C-H5xL6 strains, such that the yield from this strain was comparable to the Ab-C-H4xL4 strain.

**TABLE 1. Summary of diploid P. pastoris strains expressing Ab-A. Defined numbers of copies of genes encoding the heavy chain (Hc) and light chain (Lc) of Ab-A were integrated into the identified chromosomal loci. Column headings that identify same genomic locus refer to homologous chromosomes Selected strains were cultured and assayed to determine yield of antibody secreted into the culture media (rightmost column). These results are graphically illustrated in FIG. 2.**

| **Diploid Strain** | **Hc copies (pGAP locus)** | **Hc copies (HIS4 TT locus)** | **Lc copies (pGAP locus)** | **Lc copies (HIS4 TT locus)** | **Whole Broth Antibody Yield (% lowest copy number)** |
|---|---|---|---|---|---|
| Ab-A-H2xL2 | 2 | 0 | 2 | 0 | NT |
| Ab-A-H2xL3 | 2 | 0 | 3 | 0 | NT |
| Ab-A-H3xL3 | 3 | 0 | 3 | 0 | 100% |
| Ab-A-H3xL4 | 3 | 0 | 4 | 0 | 87% |
| Ab-A-H3xL5 | 3 | 0 | 3 | 2 | NT |
| Ab-A-H4xL3 | 3 | 1 | 3 | 0 | NT |
| Ab-A-H4xL4 | 3 | 1 | 3 | 1 | 145% |
| Ab-A-H4xL5 | 3 | 1 | 3 | 2 | NT |
| Ab-A-H4xL6 | 3 | 1 | 3 | 3 | 180% |
| Ab-A-H5xL4 | 3 | 2 | 3 | 1 | 206% |
| Ab-A-H5xL5 | 3 | 2 | 3 | 2 | 211% |
| Ab-A-H5xL6 | 3 | 2 | 3 | 3 | NT |
| Ab-A-H5xL7 | 3 | 2 | 3 | 4 | 224% |

| | | | | | |
|---|---|---|---|---|---|
| NT: Not tested in bioreactors | | | | | |

**TABLE 2. Summary of diploid P. pastoris strains expressing Ab-B. Defined numbers of copies of genes encoding the heavy chain (Hc) and light chain (Lc) of Ab-B were integrated into the identified chromosomal loci. Column headings that identify same genomic locus refer to homologous chromosomes Selected strains were cultured and assayed to determine yield of antibody secreted into the culture media (rightmost column). These results are graphically illustrated in FIG. 3.**

| **Diploid Strain** | **Hc copies (pGAP)** | **Hc copies (HIS4 TT)** | **Lc copies (pGAP)** | **Lc copies (HIS4 TT)** | **Whole Broth Antibody Yield (% lowest copy number)** |
|---|---|---|---|---|---|
| Ab-B-H2xL2 | 2 | 0 | 2 | 0 | NT |
| Ab-B-H2xL3 | 2 | 0 | 3 | 0 | NT |
| Ab-B-H2xL4 | 2 | 0 | 4 | 0 | NT |
| Ab-B-H3xL3 | 3 | 0 | 3 | 0 | 100% |
| Ab-B-H3xL4 | 3 | 0 | 4 | 0 | 104% |
| Ab-B-H3xL5 | 3 | 0 | 5 | 0 | NT |
| Ab-B-H4xL3 | 3 | 1 | 3 | 0 | 143% |
| Ab-B-H4xL4 | 3 | 1 | 3 | 1 | NT |
| Ab-B-H4xL5 | 3 | 1 | 3 | 2 | 178% |
| Ab-B-H4xL6 | 3 | 1 | 3 | 3 | 184% |
| Ab-B-H5xL4 | 3 | 2 | 3 | 1 | NT |
| Ab-B-H5xL5 | 3 | 2 | 3 | 2 | NT |
| Ab-B-H5xL6 | 3 | 2 | 3 | 3 | NT |
| Ab-B-H5xL7 | 3 | 2 | 3 | 4 | NT |

| | | | | | |
|---|---|---|---|---|---|
| NT: Not tested | | | | | |

**TABLE 3. Summary of diploid P. pastoris strains expressing Ab-C. Defined numbers of copies of genes encoding the heavy chain (Hc) and light chain (Lc) of Ab-C were integrated into the identified chromosomal loci. Column headings that identify same genomic locus refer to homologous chromosomes Selected strains were cultured and assayed to determine yield of antibody secreted into the culture media (rightmost column). These results are graphically illustrated in FIG. 4.**

| **Diploid Strain** | **Hc copies (3'AOX TT)** | **Hc copies (HIS4 TT)** | **Lc copies (3'AOX TT)** | **Lc copies (HIS4 TT)** | **Whole Broth Antibody Yield (% H3xL3)** |
|---|---|---|---|---|---|
| Ab-C-H3xL4 | 3 | 0 | 4 | 0 | 120 |
| Ab-C-H4xL3 | 4 | 0 | 3 | 0 | 140 |
| Ab-C-H4xL4 | 4 | 0 | 4 | 0 | 168 |
| Ab-C-H4xL5 | 4 | 0 | 5 | 0 | 204 |
| Ab-C-H5xL4 | 3 | 2 | 4 | 0 | 240 |
| Ab-C-H5xL5 | 3 | 2 | 3 | 2 | 234 |
| Ab-C-H5xL6 | 3 | 2 | 4 | 2 | 245 |
| Ab-C-H6xL5 | 4 | 2 | 3 | 2 | 252 |
| Ab-C-H6xL6 | 4 | 2 | 4 | 2 | 163 |

These results demonstrated that yield of three different antibodies can be greatly increased by altering the number of copies of the heavy and light chain genes. Moreover, the results demonstrate that a panel of strains containing varying (defined) numbers of copies of heavy and light chain genes can be generated by mating haploid strains containing defined numbers of copies of the heavy chain genes with haploid strains containing defined numbers of copies of the light chain genes. Among the strains shown, yield could be more than doubled (e.g., compare **FIG. 2****,** strains H3xL4 and H5xL7). Though strains containing lower numbers of gene copies were not included in this example, it is expected that the magnitude of improvement is even greater relative such strains. Moreover, further improvement might be attainable by further increasing the number of gene copies beyond those exemplified, though yield may decrease for copy numbers exceeding an optimum value.

### EXAMPLE 2

### Increased Antibody Purity by Varying Heavy and Light Chain Gene Copy Number

This example demonstrates that the purity of recombinant antibodies generated in *P. pastoris* was greatly increased by varying the number of copies of the light and heavy chain genes. Strains containing varying (defined) numbers of copies of heavy and light chain genes were generated by mating haploid strains containing defined numbers of copies of the heavy chain genes with haploid strains containing defined numbers of copies of the light chain genes. Between the strains compared, overall production of undesired side-products was decreased by approximately 20%. Additionally, production of the single most abundant side-product was demonstrated to be decreased by up to about 82% between the strains compared.

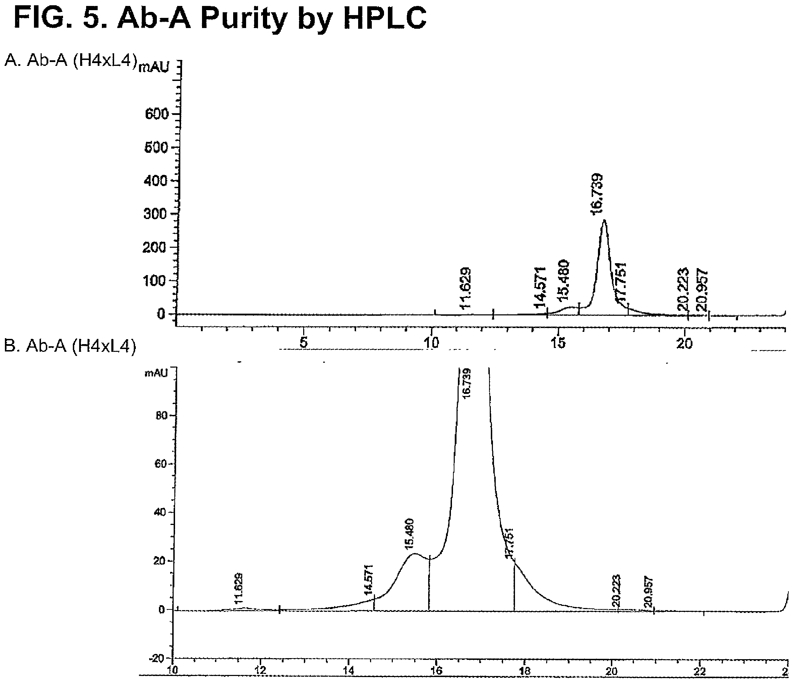

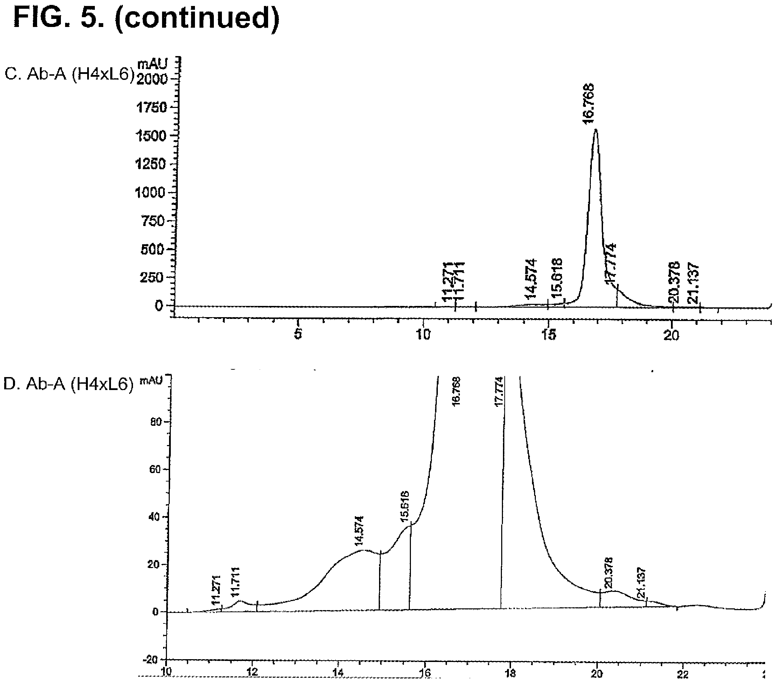

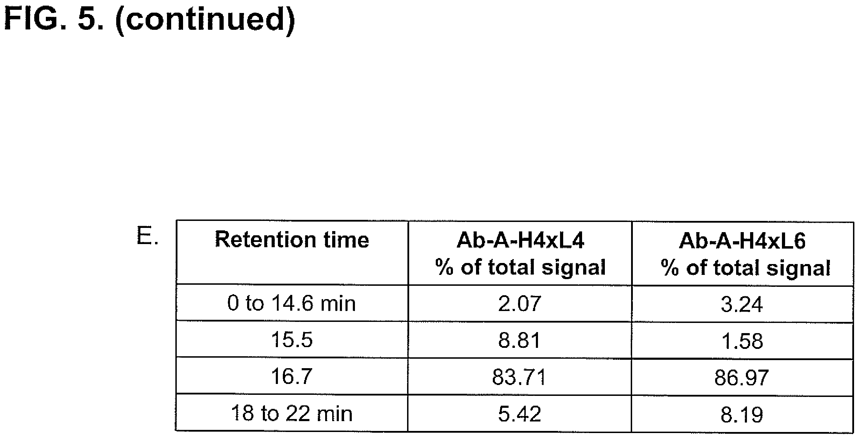

Antibody purity was determined by protein-A purification of secreted antibody from the culture media, followed by HPLC using the methods described in Example 6. The samples were maintained in native conditions expected to preserve assembled antibody complexes, permitting detection of abnormalities affecting the assembled complexes (such as incorrect stoichiometry, improper assembly, aggregation, protease cleavage, and other aberrations). Overall purity was determined by measuring the proportion of total signal observed in the peak corresponding to the expected antibody (about 16.7 minutes retention time). Exemplary HPLC traces are shown for Ab-A preparations from strains Ab-A-H4xL4 (**FIG. 5A** and enlarged view in **FIG. 5B**) and Ab-A-H4xL6 (**FIG. 5C** and enlarged view in **FIG. 5D**). Total detected signal is quantified for four regions of each trace, corresponding to the elution prior to the major peaks (0 to 14.6 minutes retention time), the main product variant peak (15.5 minutes retention time), the expected antibody peak (16.7 minutes retention time), and elution after the expected antibody peak (18 to 22 minutes) (**FIG. 5E**). The purity of the antibody preparation from Ab-A-H4xL4 was about 83.7%, and the purity of the antibody preparation from strain Ab-A-H4xL6 was about 87.0%. By this measure, the overall level of impurities was reduced by about 20% (from 16.3% to 13%) by increasing the number of copies of the light chain gene. The abundance of the main product variant peak (15.5 minutes retention time) was dramatically decreased from 8.81% to 1.58% (82% decreased) by increasing the number of copies of the light chain gene.

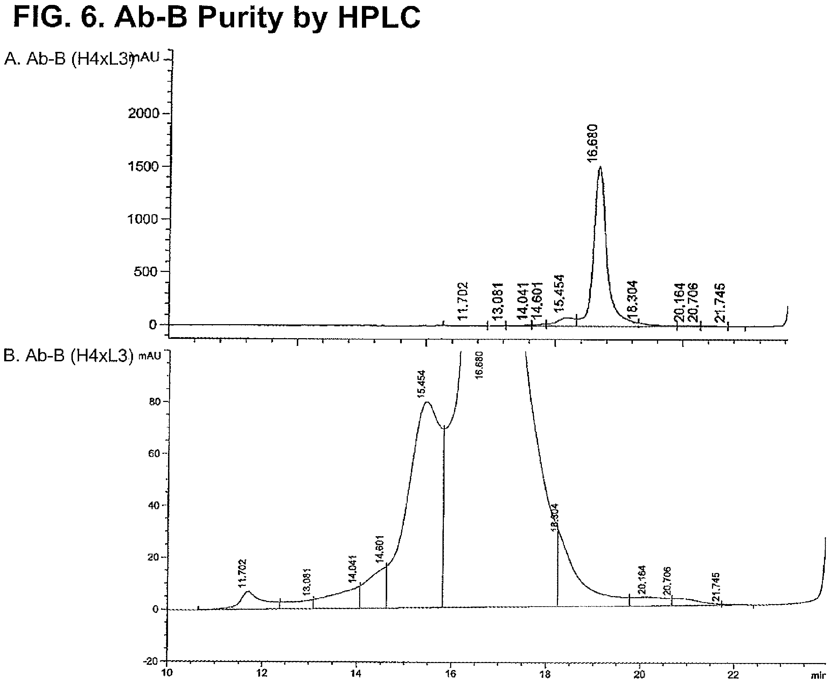

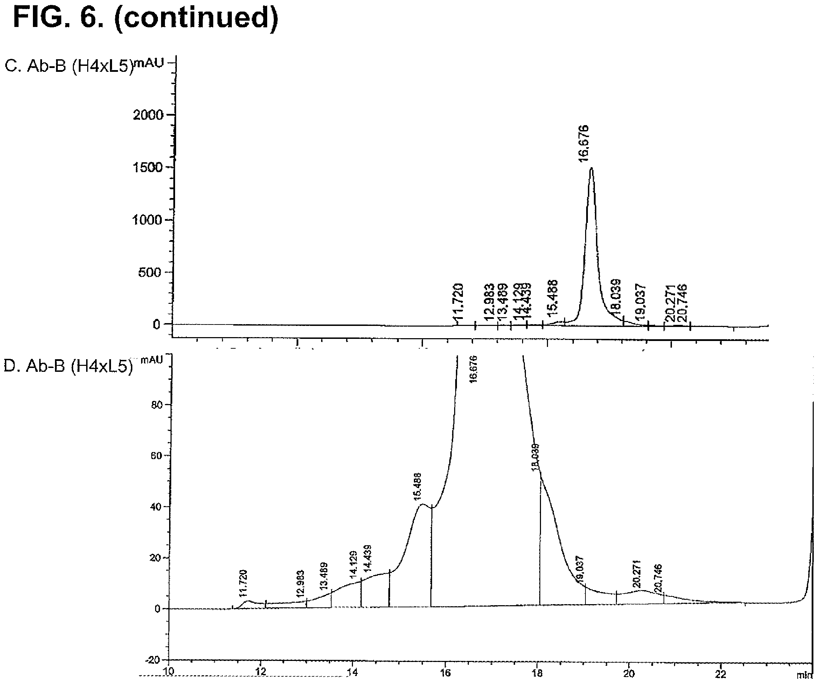

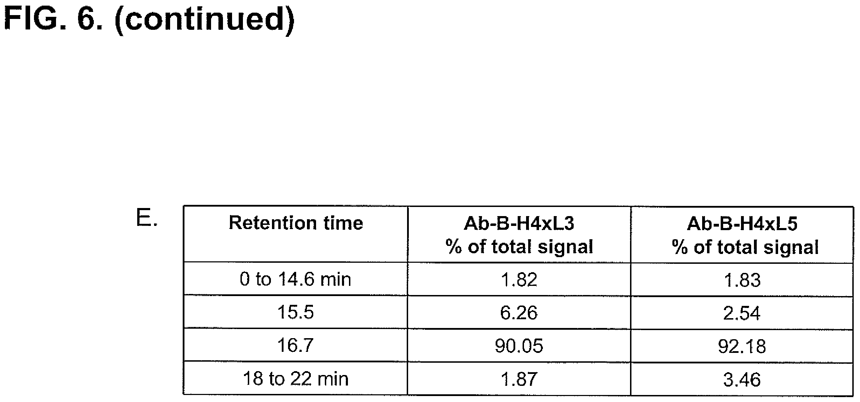

Similar results were obtained by HPLC analysis of Ab-B preparations. Exemplary HPLC traces are shown for the antibody preparation from strains Ab-B-H4xL3 (**FIG**. **6A** and enlarged view in **FIG. 6B**) and Ab-B-H4xL5 (**FIG**. **6C** and enlarged view in **FIG. 6D**). The purity of the antibody preparation from Ab-B-H4xL3 was about 90.05%, and the purity of the antibody preparation from strain Ab-B-H4xL5 was about 92.18%. By this measure, the level of impurities was reduced by about 21% (from about 10% to about 7.8%) by increasing the number of copies of the light chain gene. As with Ab-A, at the main product variant had a peak with a retention time of about 15.5 minutes. The abundance of this predominant variant was decreased by about 59% (from 6.26% to 2.54%) by increasing the number of copies of the light chain gene (**FIG. 6E**).

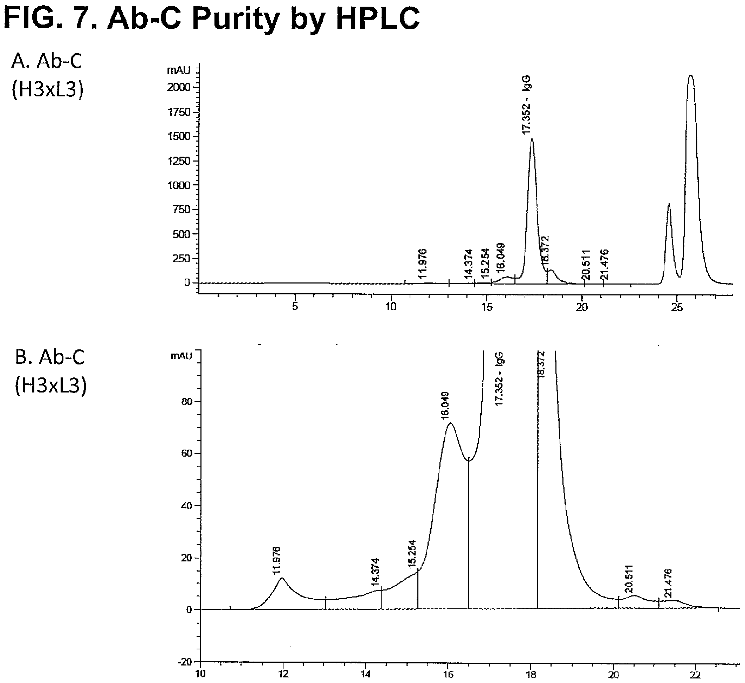

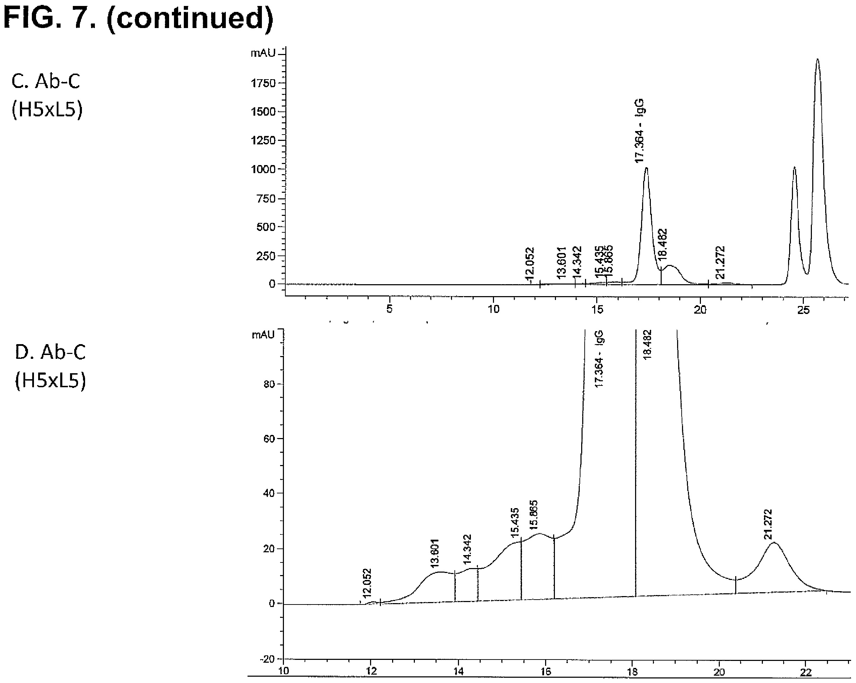

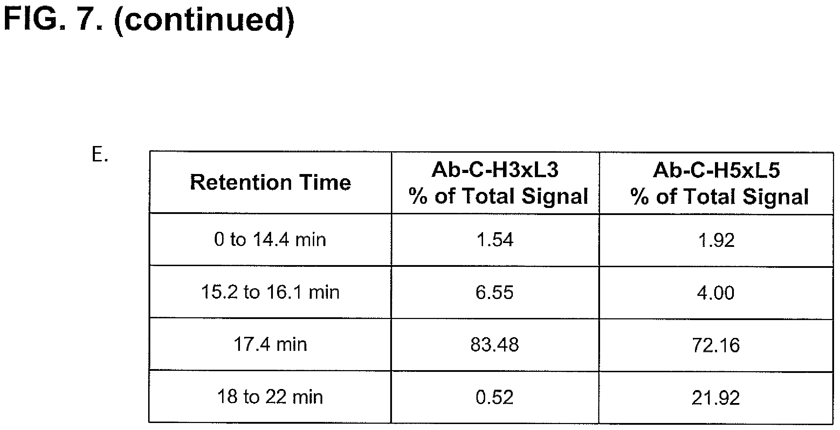

Purity of the Ab-C preparations were also analyzed by HPLC. Exemplary HPLC traces are shown for the antibody preparation from strains Ab-C-H3xL3 (**FIG. 7A** and enlarged view in **FIG**. **7B**) and Ab-C-H5xL5 (**FIG. 7C** and enlarged view in **FIG. 7D**). The main product variant had a peak with a retention time of 15.2 to 16.1 minutes. The abundance of this predominant variant was decreased by about 39% (from 6.55% to 4.00%) by increasing the number of copies of the light and heavy chain genes (**FIG. 7E**).

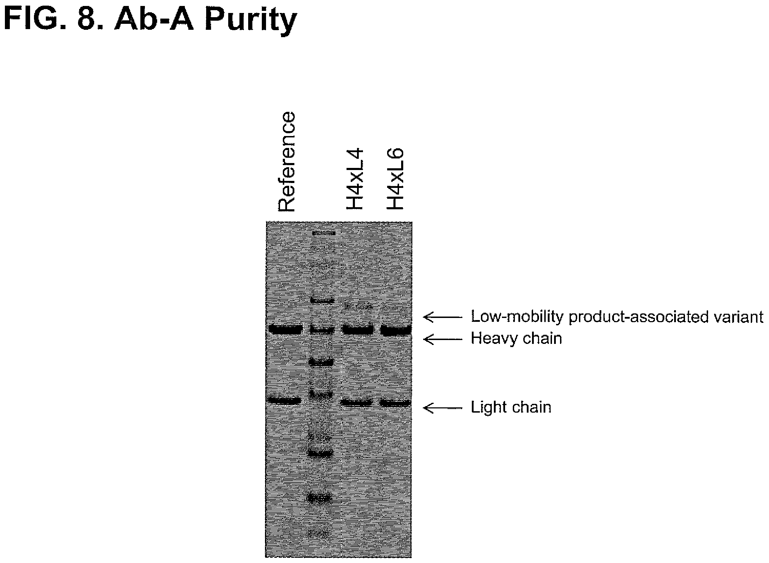

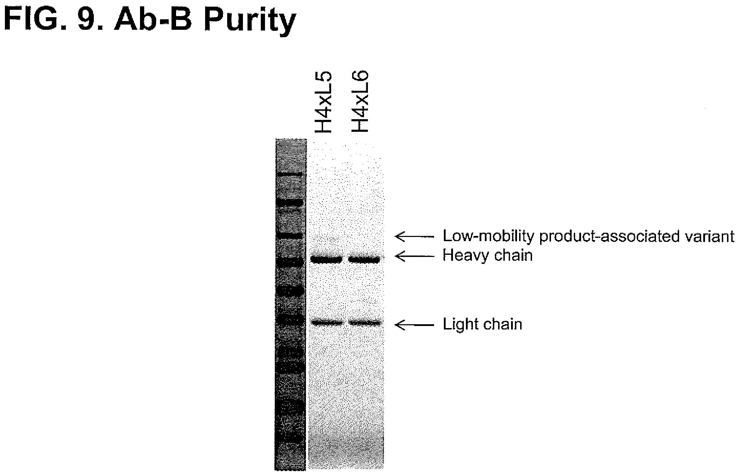

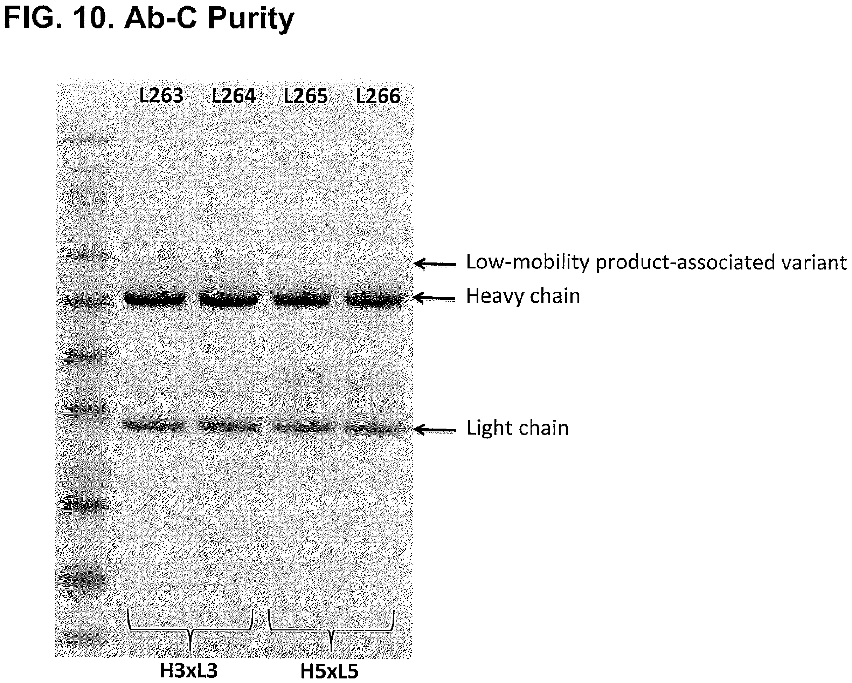

Product variants in Ab-A (**FIG. 8**), Ab-B (**FIG. 9**), and Ab-C (**FIG. 10**) preparations were also visualized on protein gels. Because the samples were subjected to denaturing and reducing conditions, this method can detect abnormalities affecting the constitution of individual antibody chains but would not be expected to detect other types of abnormalities (such as complexes having improper stoichiometry, aggregation, protease cleavage, improper disulfide linkages, or other assembly errors). The antibodies were purified by Protein-A affinity chromatography as described in Example 7, then resolved by SDS-PAGE and stained with Coomassie Blue staining. As expected, the major bands corresponded to the predicted molecular weight of the heavy and light chains (confirmed in **FIG. 8** by the lane labeled "Reference" which was loaded with a pure reference antibody). A single predominant product-associated variant was readily observable in each sample (**FIGS. 8****,** **9****, and** **10****,** arrow labeled "Low-mobility product-associated variant"). The low-mobility product-associated variant had decreased electrophoretic mobility relative to the heavy chain. The amount of this product-associated variant was visibly reduced in the antibody preparation from the strains containing higher numbers of copies of the light chain, specifically in the Ab-A preparation from the Ab-A-H4xL6 strain compared to the Ab-A-H4xL4 strain (**FIG. 8**), in the Ab-B preparation from the Ab-B-H4xL6 strain compared to the Ab-B-H4xL5 strain (**FIG. 9**), and in the Ab-C preparation from the Ab-C-H5xL5 strain compared to the Ab-C-H3xL3 strain.

Thus, for three different antibodies, two complementary methods of detecting impurities (HPLC and SDS-PAGE) both demonstrated that increased copy number of the antibody light chain resulted in improved antibody purity. Further experiments (described in Example 3 below) demonstrated a glycosylated heavy chain variant ("glyco-heavy variant") was a constituent of the predominant product-associated variant detected by both methods.

### EXAMPLE 3

### Decreased Production of Glycosylated Heavy Chain Variant by Varying Heavy and Light Chain Gene Copy Number

This example characterizes the most abundant product-associated variant that was observed in preparations of recombinant antibodies in the preceding example. Specifically, the product-associated variant was shown to be a glycosylated polypeptide containing at least part of human Fc ("glyco-heavy variant"). Production of the glyco-heavy variant was shown to be decreased in strains having an increased number of copies of the light chain gene. Because glycoproteins are potentially more immunogenic than aglycosylated forms, manipulation of the host cell to decrease their production may be particularly beneficial for some intended uses.

The low-mobility product-associated variant described in the preceding example was demonstrated to be a glycoprotein by its specific binding to a lectin-containing resin. Glycoproteins were purified from two Ab-B preparations (from H4xL5 and H4xL3 strains) and analyzed by SDS-PAGE and Western blotting using the methods described in Example 8. **FIG. 11A** shows analysis of the loaded material (left panel, "Load") and lectin column eluate (right panel, "Lectin Eluate) by SDS-PAGE with Coomassie Blue staining. Three predominant bands were detected in the lectin column eluate: the low-mobility product-associated variant (arrows), and the light and heavy chain polypeptides. Compared to the loaded material (**FIG. 11A****,** left panel), the low-mobility product-associated variant (arrow) was greatly enriched in the lectin column eluate (**FIG. 11A****,** right panel). These results indicated that the low-mobility product-associated variant was a glycoprotein. Additionally, the co-purification of light- and heavy-chain polypeptides strongly suggests that the low-mobility product-associated variant was physically associated with these polypeptides. **FIG. 11B** shows further characterization of the loaded material (left panel, "Load") and lectin column eluate (right panel, "Lectin Eluate") by Western blotting with an anti-HuFc antibody conjugated to horseradish peroxidase (GoatAntiHuFC-HRP at 1:10,000). This antibody specifically bound the Fc sequence contained in the human heavy chain polypeptide as expected (**FIG. 11B****,** lower band). The low-mobility product-associated variant was specifically detected (**FIG. 11B****,** arrow), indicating that it contained at least part of Fc sequence of the heavy chain. Moreover, Western signal from the low-mobility product-associated variant was greatly enriched in the lectin column eluate, confirming that the lectin-enriched band and Fc-containing band were the same. Therefore, we concluded that the low-mobility product-associated variant was a glycoprotein containing at least the Fc portion of the heavy chain, possibly associated with a complex containing light- and heavy-chains, which is referred to herein as the "glyco-heavy variant."

Relative abundance of the glyco-heavy variant was also shown to be decreased in the preparation from strain Ab-B-H4xL5 relative to Ab-B-H4xL3. Protein-A purified antibody prepared from each strain was compared on a Coomassie stained gel (**FIG. 11A**, left panel) and the abundance of the glyco-heavy variant was visibly decreased in the H4xL5 preparation. Consistent with these results, the abundance of the glyco-heavy variant in the H4xL5 preparation was also visibly decreased relative to the (H4xL3 preparation) in the lectin column eluate (**FIG. 11A**, right panel). The same results were observed when the glyco-heavy variant was detected with an anti-HuFc antibody (**FIG. 11B**). These results indicate that the production of the glyco-heavy variant can be modulated by varying the number of copies of antibody chain genes.

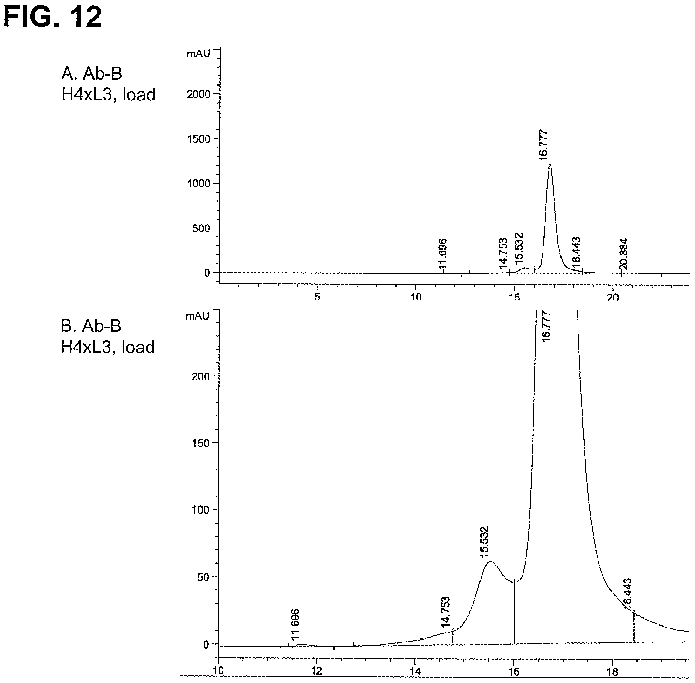

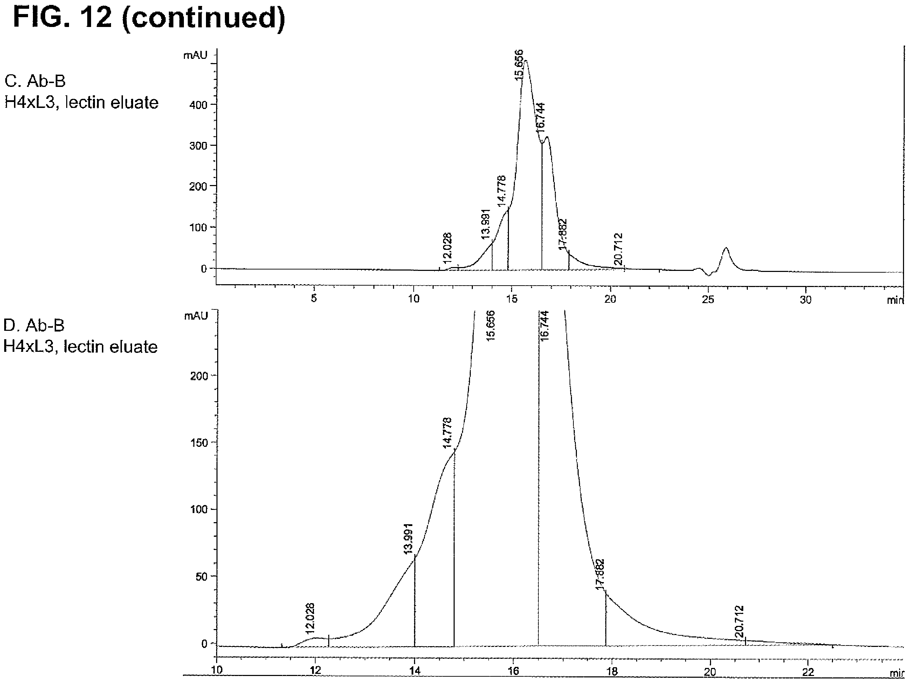

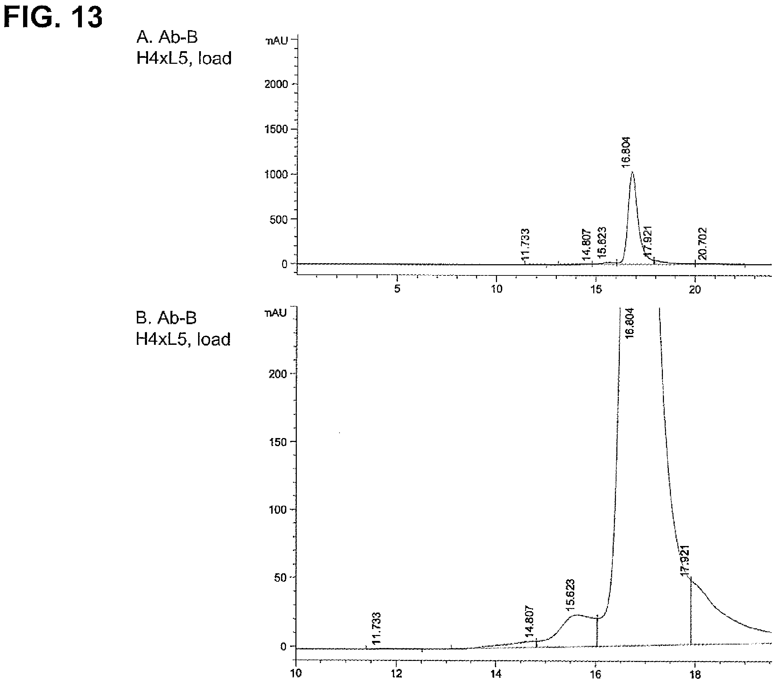

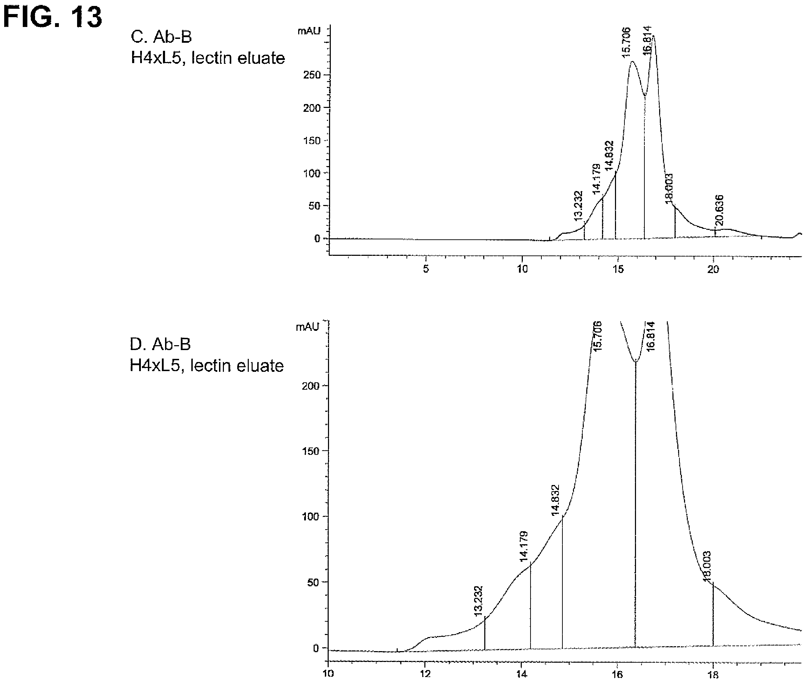

Lectin-purified Ab-B preparations were then analyzed by HPLC using the methods described in Example 8 below. Prior to lectin purification, the main peak corresponded to the expected antibody (about 16.7 minutes retention time) for the preparations from both H4xL3 (**FIG. 12A** and enlarged view in **FIG. 12B**) and H4xL5 (**FIG. 13A** and enlarged view in **FIG. 13B**) strains. In both preparations, the most abundant product-associated variant observed had a retention time of about 15.5 minutes. After lectin purification, that product-associated variant was greatly enriched and became predominant in the preparations from both H4xL3 (**FIG. 12C** and enlarged view in **FIG. 12D**) and H4xL5 (**FIG. 13C** and enlarged view in **FIG. 13D**) strains. Because it was greatly enriched in the lectin column eluate, we concluded that the glyco-heavy form observed using reduced protein gels was a constituent of the product-associated variant having a retention time of 15.5 minutes.

The fraction of the total mass contained in the expected peak and glyco-heavy peak in **FIG. 12** and **FIG. 13** are shown in Table 4 below. Comparison of the antibody preparations prior to lectin purification ("Load" column) provides a quantitative assessment of antibody purity and prevalence of the glyco heavy form. The relative abundance of the glyco heavy form in the H4xL5 preparation (2.7%) was less than half of what it was in the H4xL3 preparation (6%).

**Table 4. Quantitative assessment of Ab-B purity in preparations from H4xL3 and H4xL5 strains by HPLC. Percentage of total protein mass contained in the "glyco form" (about 15.5 minutes retention time) and "main peak" (16.7 minutes retention time, corresponding to the expected antibody) is shown for preparations from each strain in the antibody preparation prior to ("Load") and after ("Lectin Eluate") purification with a lectin column.**

| | **Load** | **Lectin Eluate** |
|---|---|---|
| Ab-B H4xL3 glyco form | 6% | 58.6% |
| Ab-B H4xL3 main peak | 90.1% | 24.5% |
| Ab-B H4xL5 glyco form | 2.7% | 41.1% |
| Ab-B H4xL5 main peak | 89.6% | 36.1% |

Taken together, these results indicate that the proportion of glycosylated heavy chain can be altered by varying the heavy and light chain gene copy numbers. Thus, manipulation of the light and heavy gene copy numbers can effect a reduction in glycosylated antibody production when desired, and this can be accomplished by direct mating of selected haploid strains.

### EXAMPLE 4

### Methods for Generation of a Cell Panel Containing Varying Copy Numbers of Antibody Genes

This example describes methods used for the production of a panel of transformed yeast cells comprising variable numbers of copies of genes encoding each subunit of a heterologous multi-subunit protein, and mating of transformed cells to produce a panel of diploid cells that express the multi-subunit protein from varying numbers of gene copies. An overview of the strain generation methodology is shown in **FIG. 1****.**

### Construction of Pichia pastoris expression vectors for heavy and light chain.

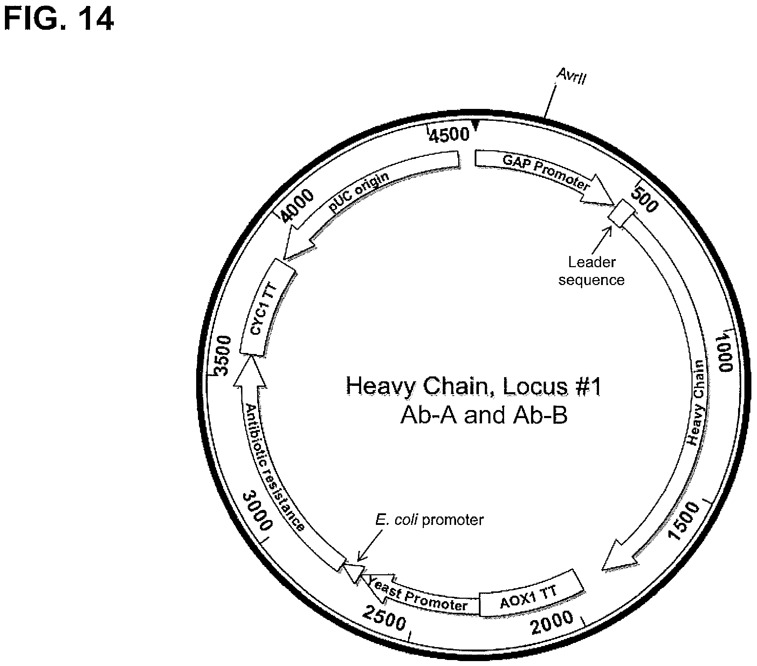

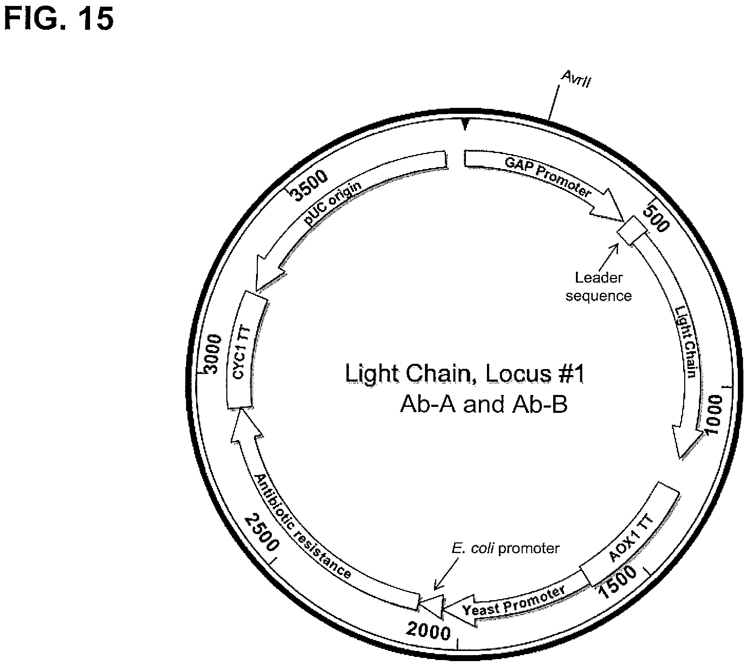

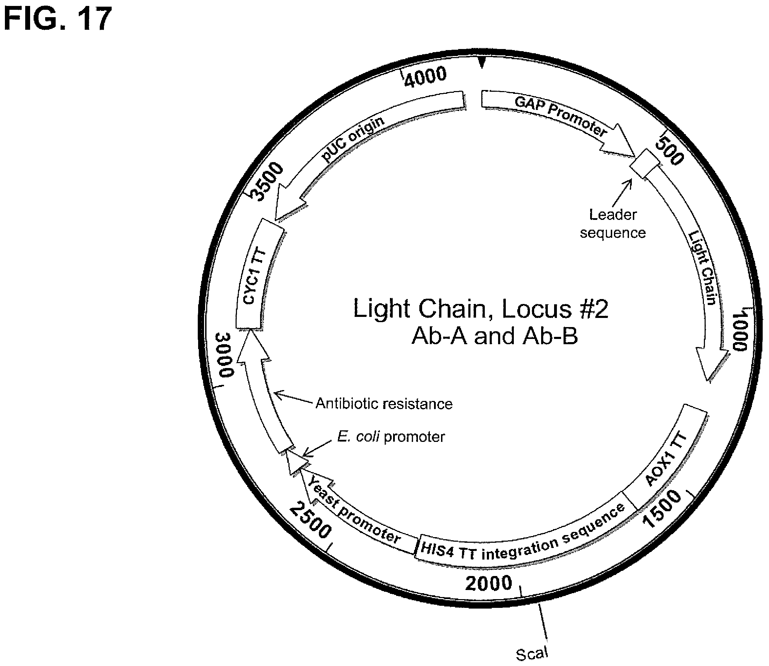

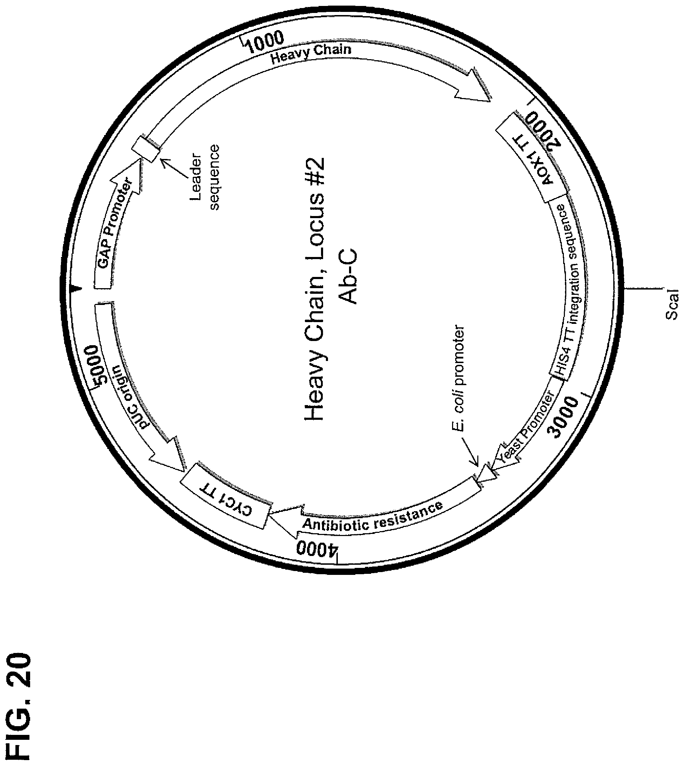

The light and heavy chain fragments were commercially synthesized and subcloned into a pGAP expression vector (**FIGS. 14****,** **15****,** **18****, and** **20**). The pGAP expression vector uses the GAP promoter to drive expression of the immunoglobulin chain. In addition, this vector contains common elements such as a bacterial origin of replication and an expression cassette for antibiotic resistance. For Ab-A and Ab-B, the GAP promoter sequence (about 500 basepairs in length) was used for targeting integration into this locus. The vector includes a copy of the kanamycin resistance gene which confers resistance to the antibiotic G418 in *P. pastoris.* For Ab-C, the *AOX1* transcription terminator sequence (about 350 basepairs in length) was used for targeting integration into this locus. The vector includes a copy of the *Sh ble* gene, which confers resistance to the antibiotic Zeocin™ (phleomycin). G418 and Zeocin™ provide a means of selection for strains that contain the desired expression vector integrated into their genome. Finally, for Ab-A, Ab-B and Ab-C, the vector used for the second round of integration includes 660 basepairs of *P. pastoris* genomic sequence surrounding the *HIS4* transcription terminator used for targeting integration into this locus (**FIGS. 13****,****14****,****16****, and** **17**). For Ab-A and Ab-B, the vector used for the second round of transformation includes a copy of the *Sh ble* gene. For Ab-C, the vector used for the second round of transformation includes a copy of the kanamycin resistance gene.

### Transformation of expression vectors into haploid met1 and lys3 host strains of Pichia pastoris

*P. pastoris* cells were transformed by electroporation following a modified protocol from Pichia Protocols, Second Edition (Methods in Molecular Biology, Cregg, JM, Ed. 2007. Humana Press, Totowa, NJ). The transformed strains were derived from JC231 (Lys⁻) or JC239 (Met⁻). A 3-mL YPD (1% yeast extract, 2% peptone, 2% dextrose) culture was inoculated with a *P. pastoris* colony for each host strain and allowed to grow overnight with shaking at 30°C. These cultures were then used to inoculate 400-mL YPD cultures in 2L Thomson shake flasks at a starting OD₆₀₀ of 0.01. Cells were harvested when OD₆₀₀ reached 1.0-2.0 and resuspended in 100 mL YPD medium containing 0.2M HEPES (pH 8.0) and 0.025M DTT. Cells were incubated at 30°C for 30 minutes, and the volume was then brought up to 400 mL using 1M cold sorbitol. Cells were washed once in 400 mL 1M cold sorbitol, followed by three times in 30 mL cold sorbitol before resuspending in a final volume of 1 mL of 1M cold sorbitol.

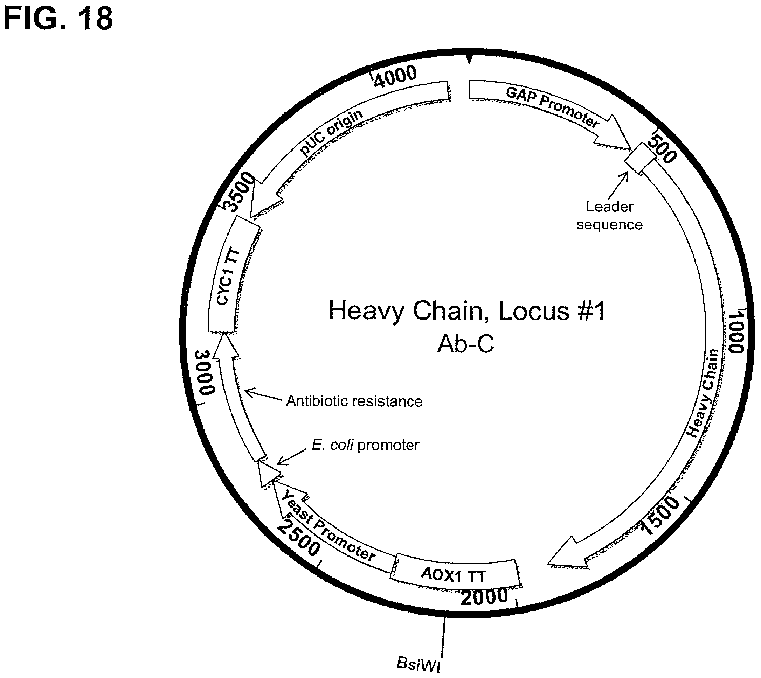

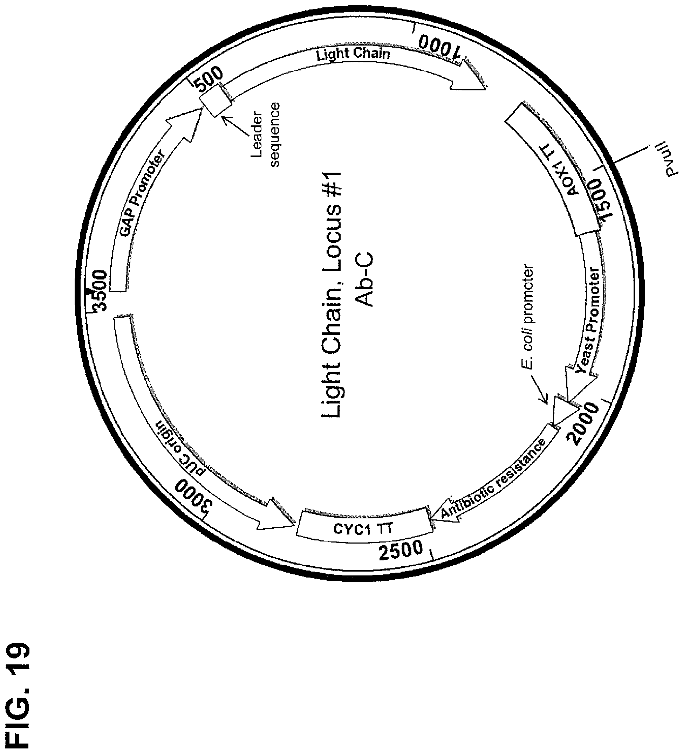

For preferred integration of Ab-A or Ab-B into the GAP promoter, prior to transformation each vector (**FIGS. 14-15**) was linearized within the GAP promoter sequences using AvrII restriction endonuclease to direct the integration of the vector into the GAP promoter locus of the *P. pastoris* genome. For preferred integration of Ab-C into the *AOX1* transcription terminator, each vector (**FIGS. 18-19**) was linearized within the 3'AOX TT sequence using either BsiWI (for heavy chain) or PvuII (for light chain). For Ab-A and Ab-B, successful transformants were selected on YPDS (1% yeast extract, 2% peptone, 2% dextrose, 2% agar, 1M sorbitol) agar plates containing G418. For Ab-C, successful transformants were selected on YPDS agar plates containing Zeocin™. This is referred to as the first locus integration. For Ab-A and Ab-C, fluorescence activated cell sorting (FACS) was used to enrich for clones containing higher copies of heavy or light chain. Briefly, transformation plates were scraped in approximately 5 mL PBS. Cells were stained with fluorescence detection antibodies specific for either heavy or light chain. Positive cells were detectable by FACS even though the genes of interest were fused to secretion signals, which was apparently due to at least transient retention of some protein at the cell surface. The top 20-40% of the stained cells were sorted and used to inoculate 25 mL BYED (3% yeast extract, 4% anhydrous dextrose, 1.34% yeast nitrogen base, 0.004% Biotin, and 100 mM potassium phosphate) shake flask cultures. After growing overnight at 30°C with shaking, cells were harvested, stained, and enriched a second time using FACS. The top 10-20% of the stained cells were streaked to single colony on YPD plates containing G418 (for Ab-A) or Zeocin™ (for Ab-C). The FACS enrichment assay was omitted for Ab-B.

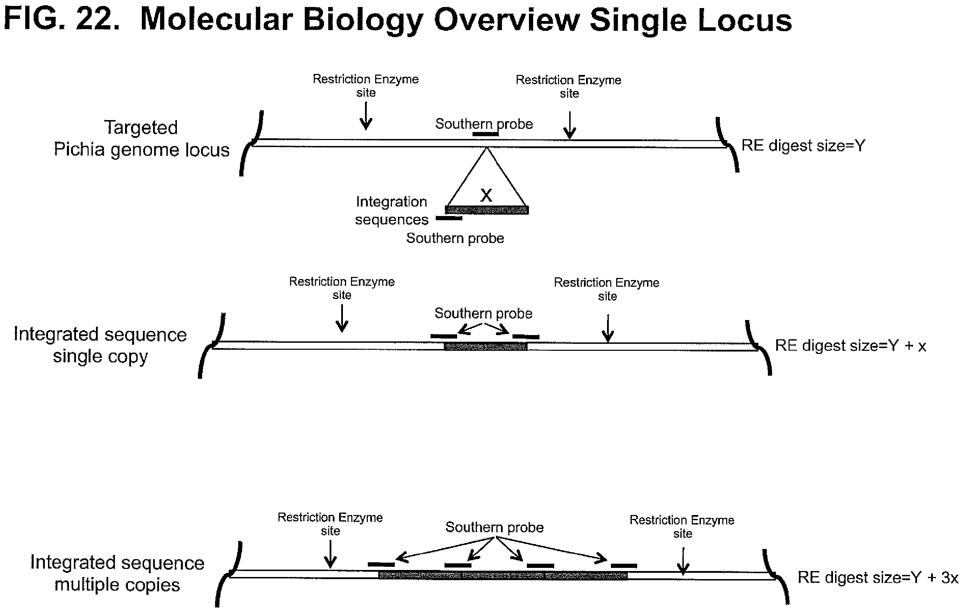

Varying numbers of copies of the construct integrated in tandem into the targeted locus (illustrated in **FIG. 22**). Copy numbers of heavy and light chain genes were determined for haploid strains by Southern blot analysis. In brief, genomic DNA was digested with a restriction enzyme that cleaves sequences flanking the integration site, resolved by agarose gel electrophoresis, transferred to a membrane, and hybridized to a probe consisting of the integration sequence. The size of the restriction fragment increases linearly with the number of integrated copies (see **FIG. 22**). If the size of the genomic restriction fragment prior to integration is Y and the length of the integrated sequence is X, then the fragment size after integration of N copies will be Y + NX. Using this relationship, the number of copies per transformant was determined from the length of the detected fragment.

Haploid strains with desired copy numbers were then mated and selected for their ability to grow in the absence of both auxotroph markers (i.e., Lys and Met) on BYNB (1.34% yeast nitrogen base, 2.5% agar, 2% dextrose, 0.1M potassium phosphate pH 6.0) agar plates. Resulting diploid clones were then analyzed by Southern blot to confirm copy numbers of heavy and light chain genes. Diploid clones expressing full length monoclonal antibodies were further characterized by 48hr growth in a deep-well plate containing 1mL of BSM (basal salt media, 10 g/L sodium citrate dihydrate, 36.4 g/L ammonium phosphate monobasic, 18.2 g/L potassium sulfate, 12.8 g/L potassium phosphate monobasic, 3.7 g/L magnesium sulfate heptahydrate, 40 g/L dextrose, 40 g/L yeast extract, 4.35 mL/L PTM1 solution, pH 6.0) containing 4% yeast extract. Antibody concentrations in the supernatants were then quantified using biolayer interferometry Protein A biosensors (Octet, ForteBio).

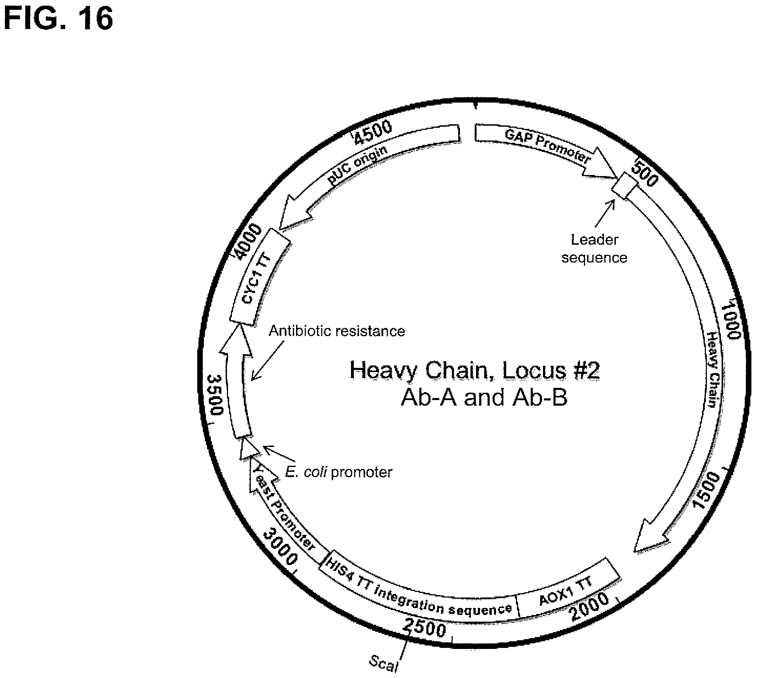

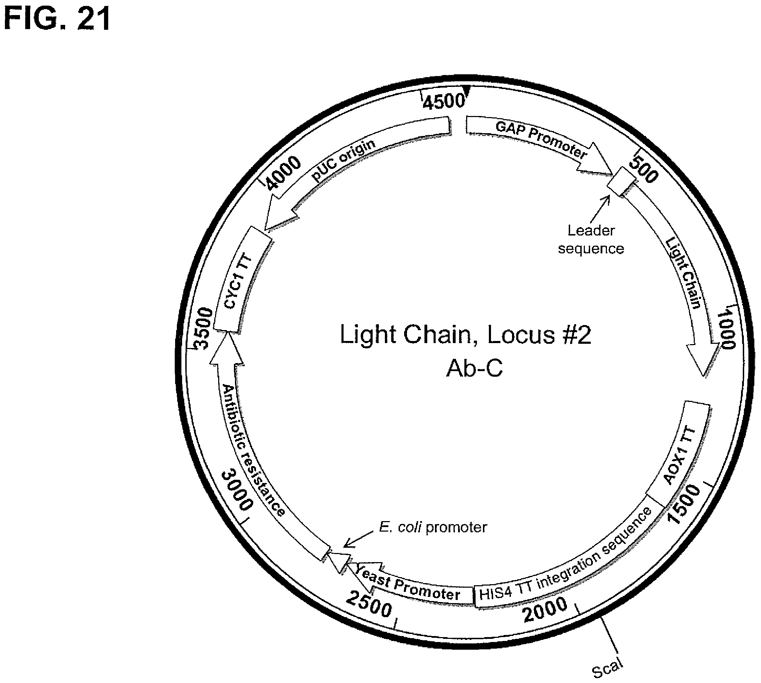

For integration into a second genomic locus, competent cells for the transformation were prepared using haploid strains containing a pre-determined copy number of heavy chain or light chain as per the protocol described above. For preferred integration into the HIS4 TT locus, each expression vector (**FIGS. 16-17** **and** **20-21**) was linearized within the HIS4 TT integration sequence using Sca1 restriction endonuclease to direct integration into that locus. For Ab-A and Ab-B, successful transformants were selected on YPDS agar plates containing Zeocin™. For Ab-C, successful transformants were selected on YPDS agar plates containing G418. Copy numbers of heavy and light chain genes integrated at the *HIS4* TT locus were determined using Southern blot. Haploid strains were mated, and diploid strains were selected as described above. A final Southern blot was done to confirm copy numbers of heavy and light chain genes at each of the integration loci. A clone was selected using Protein A biosensors to monitor expression (Octet, ForteBio).

### Southern blotting

Copy numbers of heavy and light chain genes were determined for haploid strains by Southern blot analysis. Single colonies were selected from YPD agar plates and used to inoculate 3-mL YPD cultures. Cultures were incubated overnight at 30°C with shaking until saturated. Genomic DNA was extracted from 1.8 mL of each culture using the MasterPure Yeast DNA Purification Kit (Epicentre) following the manufacturer's protocol. For the pGAP locus, one microgram of DNA was digested with Cla I and separated on a 0.8% TAE agarose gel. Following electrophoresis, the gel was treated with denaturing buffer (0.5M NaOH; 1.5M NaCl) for 45 minutes and neutralization buffer (0.5M Tris-HCl, pH 7.2; 1.5M NaCl; ImM EDTA) for 30 minutes. DNA was then transferred to a positively charged nylon membrane (BioRad) by capillary action and fixed using a UV crosslinker. The membrane was hybridized overnight at 41°C using a digoxigenin (DIG)-labeled DNA probe corresponding to the pGAP sequence. The membrane was washed under high stringency conditions and detected using the DIG High Prime Labeling and Detection Kit (Roche Applied Science). Hybridized bands were visualized by exposing the membrane to X-ray film.

Copy numbers of heavy and light chain genes integrated at the 3'AOX TT or HIS4 TT loci were determined using Southern blot following the steps described above, with some modifications. For the *AOX1* transcription terminator locus, HindIII restriction endonuclease was used for the genomic DNA digestion, and a DIG-labeled probe corresponding to the 3'AOX TT sequence was used for hybridization. For the HIS4 TT locus, SspI restriction endonuclease was used for the genomic DNA digestion, and a DIG-labeled probe corresponding to the HIS4 TT sequence was used for hybridization.

Using the foregoing methodology, a panel of transformants containing varying numbers of copies of the individual subunits are obtained, for example, strains labeled H3, H4, H5 and H6 containing three, four, five and six copies, respectively, of an antibody heavy chain, and strains labeled L3 through L7 containing three through seven copies, respectively, of an antibody light chain. These transformants are then mated to obtain diploids containing varying numbers of copies of the light and heavy chain genes, and gene copy numbers are optionally reconfirmed by Southern blot. Diploid cells containing known, varying copy numbers of the light and heavy chain genes are thereby produced. Optionally, a clone expressing the antibody of interest was selected using biolayer interferometry Protein-A biosensors to monitor expression (Octet, ForteBio). Frozen stocks of the resulting haploid and/or diploid strains are typically produced, and transformants are propagated for evaluation of yield, production rate, and purity of the mature antibody.

### Generation of a Panel of P. Pastoris Strains Containing a Varying Number of Copies of The Light and Heavy Chain Genes Encoding Ab-A

A panel of *P. pastoris* strains for expression of Ab-A containing variable numbers of copies of the light and heavy chain genes was produced using the methods described above. A total of thirteen diploid strains were produced containing between two and five copies of the heavy chain gene and between two and seven copies of the light chain gene. Initially, haploid strains containing defined numbers of copies of the light chain and heavy chain genes were produced (each haploid strain being either Lys⁻ or Met⁻), then mating between these haploid strains was used to produce diploid prototroph strains containing a known number of copies of the light and heavy chain genes. Each expression cassette was composed of the glyceraldehyde dehydrogenase gene (GAP gene) promoter, fused to a sequences encoding a secretion signal, followed by the sequence of the gene to be expressed, followed by sequences encoding a *P. pastoris* transcriptional termination signal from the *P. pastoris* alcohol oxidase I gene (AOX1) alone or in combination with the HIS4 TT sequence (illustrated in **FIGS. 14-21**).

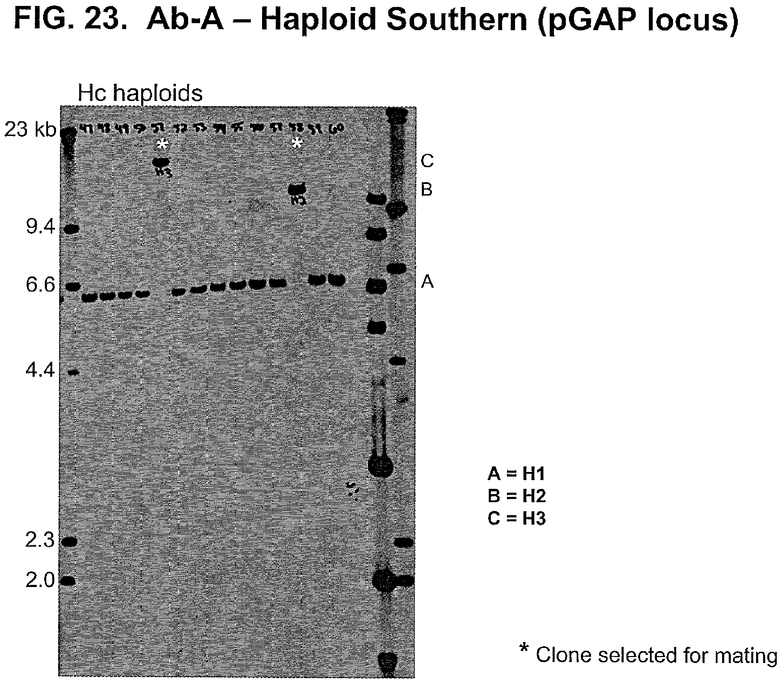

Transformants containing a gene encoding the Ab-A heavy chain integrated into the pGAP locus were assigned the identifiers Hc47 through Hc60. Purified genomic DNA was digested with a restriction enzyme that cleaved sites flanking the pGAP locus, and Southern blotting (using a labeled pGAP sequence) was used to determine the number of integrated copies in each strain as described above (**FIG. 23**). Lane 1 and the two rightmost lanes contain labeled DNA ladders (fragment sizes are written at the left edge of the blot). Lanes 2 through 15 correspond to strains Hc47 through Hc60, respectively. The labels A through C at the right edge of the blot indicate the expected size of the fragment from a strain containing one through three copies, respectively, of the integrated sequence. The fragment detected in strains Hc58 and Hc51 indicated that these strains contained two and three copies, respectively, of the heavy chain gene. Strains Hc58 and Hc51 were selected for mating.

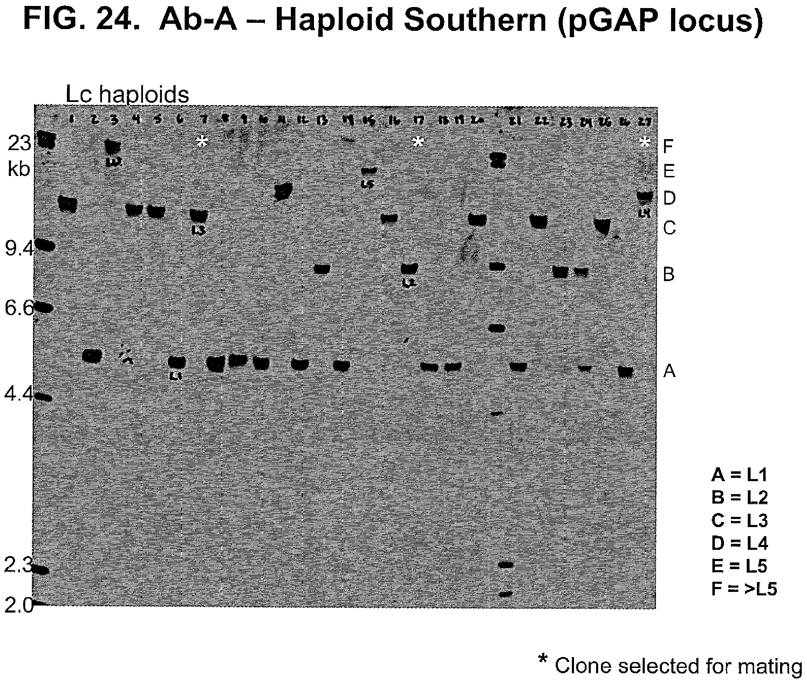

Similarly, a panel of haploid *P. pastoris* strains were produced that contained varying numbers of genomic copies of a gene encoding Ab-A light chain, also integrated into the pGAP locus. The strains were assigned the identifiers Lc1 through Lc27. Purified genomic DNA was digested with a restriction enzyme that cleaved sites flanking the pGAP locus, and Southern blotting (using a labeled pGAP sequence) was used to determine the number of integrated copies in each strain (**FIG. 24**). Lanes 1 and 22 contain a labeled DNA ladder (fragment sizes are written at the left edge of the blot). Lanes 2 through 21 and 23 through 29 correspond to strains Lc1 to Lc27, respectively. The labels A through F at the right edge of the blot indicate the expected size of the fragment from a strain containing one through five copies and more than 5 copies, respectively, of the integrated sequence. The fragment detected in strains Lc17, Lc7, and Lc27 indicated that these strains contained two, three, and four copies, respectively, of the light chain gene. Strains Lc17, Lc7, and Lc27 were selected for mating.

To further increase the number of copies of the heavy and light chain genes available in the panel, additional gene copies were integrated into a second locus. The total copy number of a specific integrated gene is therefore calculated by adding the number of copies from both integration loci. Specifically, additional copies of a gene encoding Ab-A heavy chain were integrated into the HIS4 TT locus in a strain already containing 3 copies of the Ab-A heavy chain gene integrated in the pGAP locus, thereby introducing one or two additional copies of the heavy chain gene, for a total of four or five copies. Similarly, additional copies of a gene encoding the Ab-A light chain were integrated into the HIS4 TT locus in a strain already containing 3 copies of the Ab-A light chain gene integrated in the pGAP locus, thereby introducing one to four additional copies of the light chain gene, for a total of four to seven copies.

Diploid strains were then produced by mating strains containing heavy and light chain genes. A total of thirteen different strains were produced containing between two and five copies of the heavy chain gene and between two and seven copies of the light chain gene. For each diploid strain, the number of copies of the heavy and light chain genes is indicated by the strain identifier (e.g., Ab-B-H4xL5 indicates 4 copies of the heavy chain gene and five copies of the light chain gene) and the loci from which the genes are expressed are identified in Table 1, above.

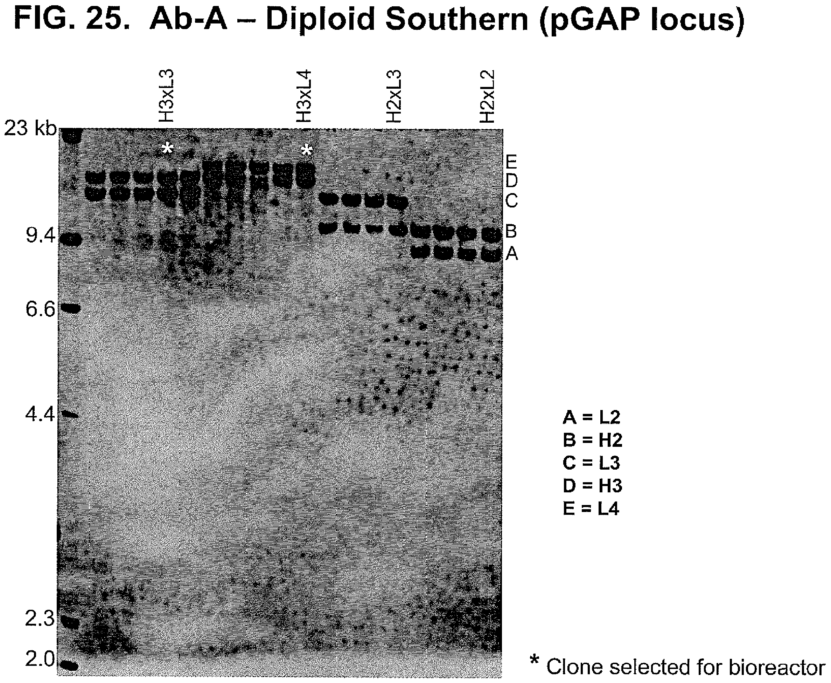

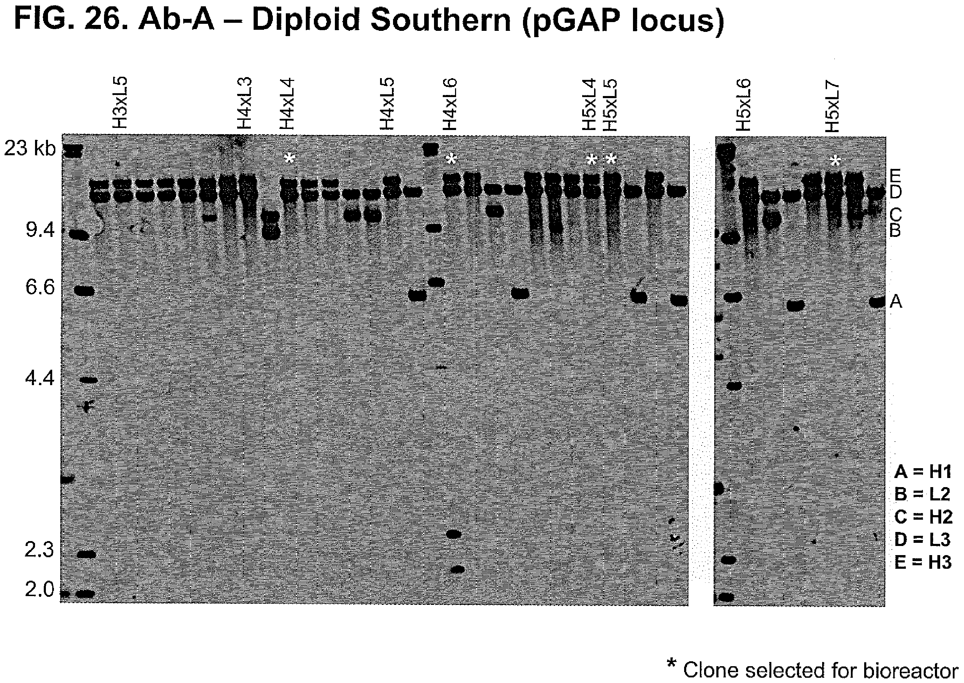

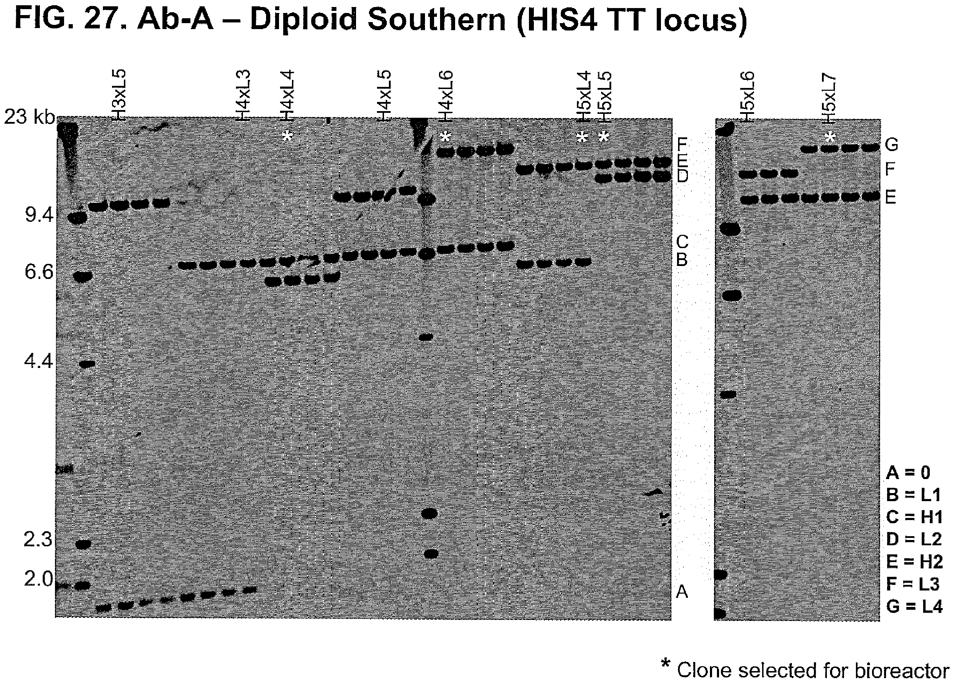

Multiple isolates of each diploid strains were generated by mating, and copy numbers were verified by Southern blotting before selecting particular isolates for further analysis. **FIG. 25** shows Southern blots of the pGAP locus to verify gene copy numbers for candidates of the Ab-A-H3xL3, Ab-A-H3xL4, Ab-A-H2xL3, and Ab-A-H2xL2 strains (lanes 2-6, 7-11, 12-15, and 16-19, respectively), and asterisks indicate candidates that were selected for further use. Lane 1 contains a labeled DNA ladder (fragment sizes are written at the left edge of the blot). The labels A, C, and E at the right edge of the blot indicate the expected size of the fragment from a strain containing two through four copies, respectively, of the light chain gene, and the labels B and D, indicate the expected size of the fragment from a strain containing two or three copies, respectively, of the heavy chain gene. **FIG. 26** shows Southern blots for verification of the gene copy numbers at the pGAP locus for isolates of the strains shown in Table 1, and asterisks indicate the isolates that were selected for further use. The labels A, C, and E at the right edge of the blot indicate the expected size of the fragment from a strain containing one through three copies, respectively, of the heavy chain gene, and the labels B and D, indicate the expected size of the fragment from a strain containing two or three copies, respectively, of the light chain gene. Similarly, **FIG. 27** shows Southern blots for verification of the gene copy numbers at the HIS4 TT locus for those strains expected to contain antibody genes integrated at that locus (identified in Table 1). The label A at the right edge of the blot indicates the expected size of a fragment containing an endogenous HIS4 TT locus, the labels B, D, F, and G indicate the expected size of the fragment from a strain containing one through four copies, respectively, of the light chain gene, and the labels C and E, indicate the expected size of the fragment from a strain containing one or two copies, respectively, of the heavy chain gene.

### Generation of a Panel of P. Pastoris Strains Containing a Varying Number of Copies of The Light and Heavy Chain Genes Encoding Ab-B

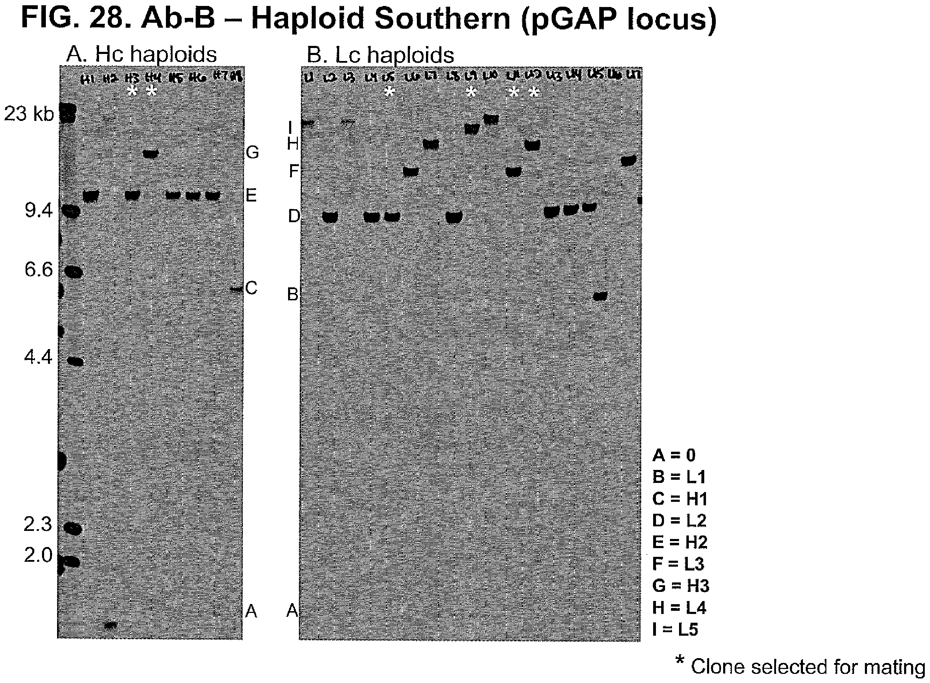

Haploid strains containing genes encoding the heavy and light chains of Ab-B (targeted to the pGAP locus) were generated using essentially the same methods described for Ab-A. Southern blots (using a pGAP sequence probe) identified strains Hc3 and Hc4 containing 2 and 3 copies, respectively, of the Ab-B heavy chain gene (**FIG. 28A**), and strains Lc5, Lc11, Lc12, and Lc9 containing 2 through 5 copies, respectively, of the Ab-B light chain gene (**FIG. 28B**). In **FIG. 28A****,** the labels A, C, E, and G at the right side of the blot indicate the expected size of the fragment containing zero to three copies, respectively, of the Ab-B heavy chain gene integrated into the pGAP locus, and in **FIG. 28B****,** the labels A, B, D, F, H, and I at the left side of the blot indicate the expected size of the fragment containing zero to five copies, respectively, of the Ab-B light chain gene integrated into the pGAP locus. Asterisks indicate haploid strains selected for further mating.

To further increase the number of copies of the heavy chain gene available in the panel, additional copies of a gene encoding Ab-B heavy chain were integrated into the HIS4 TT locus in a strain already containing 3 copies of the Ab-B heavy chain gene integrated in the pGAP locus, thereby introducing one or two additional copies of the heavy chain gene, for a total of four or five copies. Similarly, additional copies of a gene encoding the Ab-B light chain were integrated in to the HIS4 TT locus in a strain already containing 3 copies of the Ab-B light chain gene integrated in the pGAP locus, thereby introducing one to four additional copies of the light chain gene, for a total of four to seven copies.

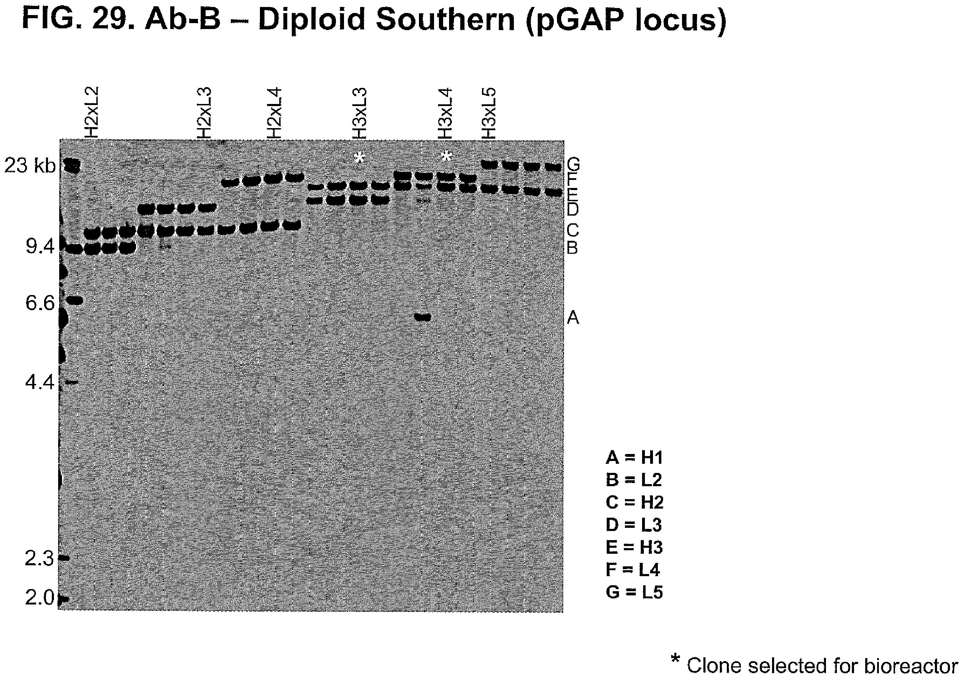

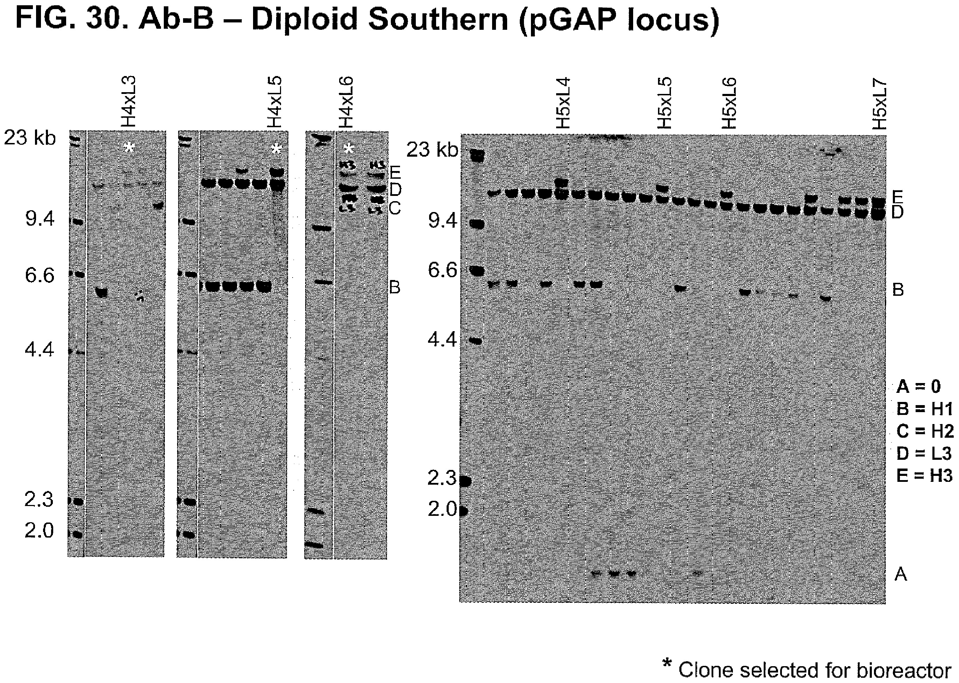

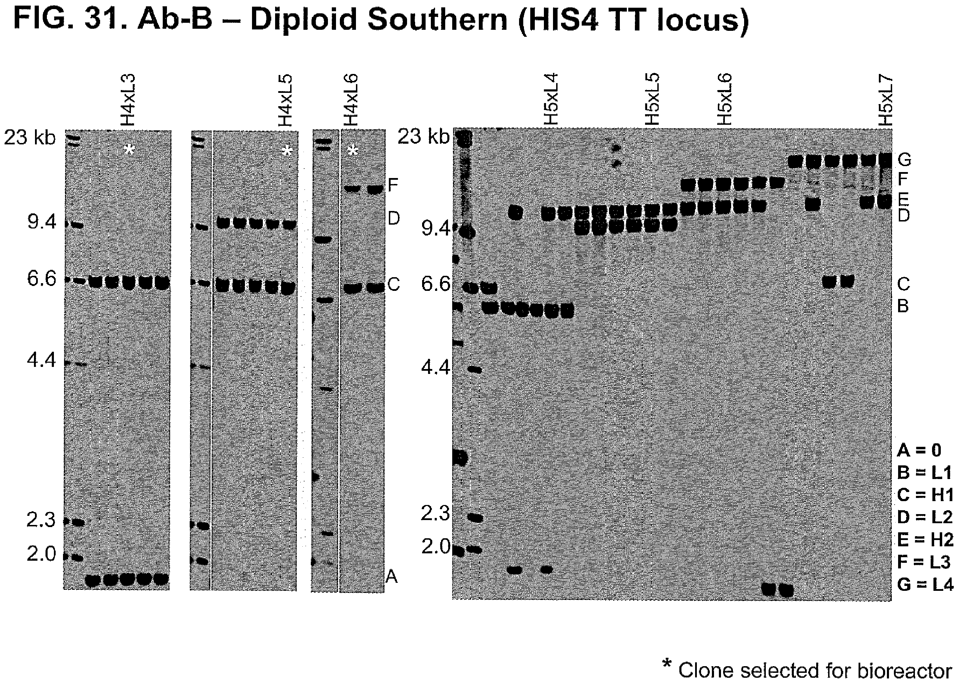

Diploid strains were then produced by mating strains containing heavy and light chain genes. A total of fourteen different strains were produced containing between two and five copies of the heavy chain gene and between two and seven copies of the light chain gene. For each diploid strain, the number of copies of the heavy and light chain genes is indicated by the strain identifier (e.g., H4xL5 indicates 4 copies of the heavy chain gene and five copies of the light chain gene) and the loci from which the genes are expressed are identified in Table 2, above. Gene copy numbers in the diploid strains were reconfirmed by Southern blots shown in **FIGS. 29-30** (pGAP probe) and **FIG. 31** (HIS4 TT probe). Multiple isolates of each diploid strain were generated, and asterisks in **FIGS. 29-31** indicate isolates selected for further use. In **FIG. 29****,** the labels A, C, and E at the right side of the blot indicate the expected size of the fragment containing one to three copies, respectively, of the Ab-B heavy chain gene integrated into the pGAP locus, and the labels B, D, F, and G indicate the expected size of the fragment containing two to five copies, respectively, of the Ab-B light chain gene integrated into the pGAP locus. In **FIG. 30**, the labels A, B, C, and E at the right side of the blot indicate the expected size of the fragment containing zero to three copies, respectively, of the Ab-B heavy chain gene integrated into the pGAP locus, and the labels A and D indicate the expected size of the fragment containing zero and three copies, respectively, of the Ab-B light chain gene integrated into the pGAP locus. In **FIG. 31****,** the labels A, C, and E indicate the expected size of the fragment containing zero to two copies, respectively, of the Ab-B heavy chain gene integrated into the HIS4 TT locus, and the labels A, B, D, F, and G indicate the expected size of the fragment containing zero to four copies, respectively, of the Ab-B light chain gene integrated into the HIS4 TT locus.

### Generation of a Panel of P. pastoris Strains Containing a Varying Number of Copies of The Light and Heavy Chain Genes Encoding Ab-C

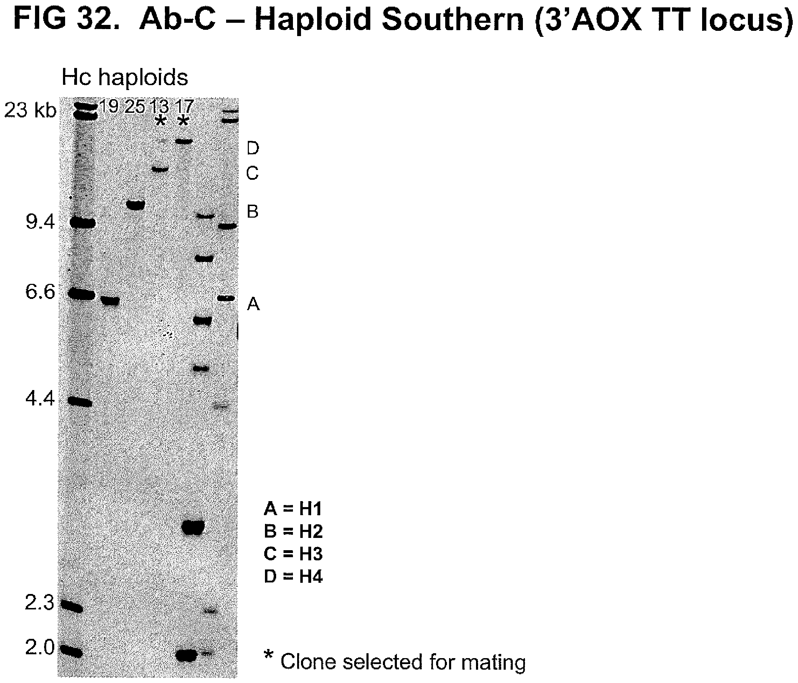

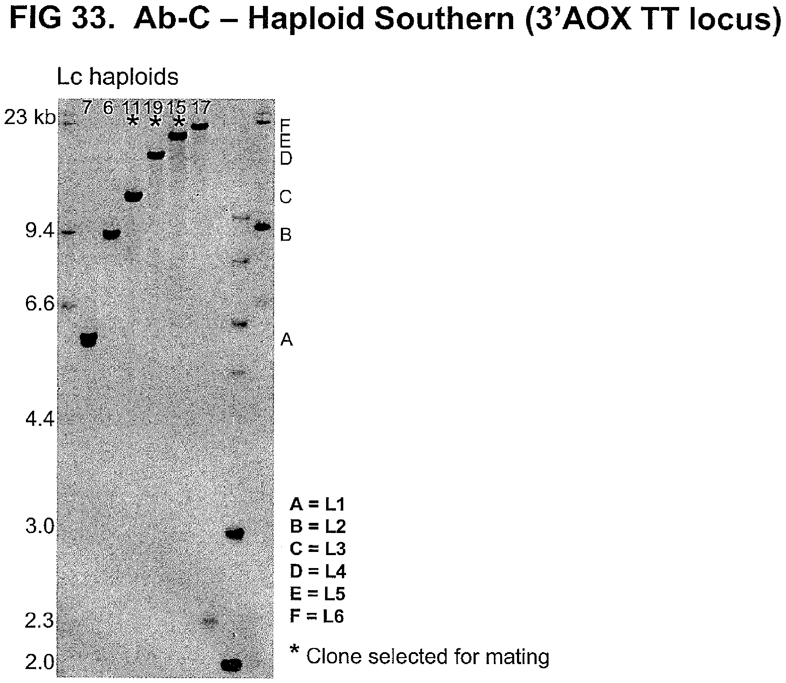

A panel of *P. pastoris* strains for expression of Ab-C containing variable numbers of copies of the light and heavy chain genes was produced using essentially the same methods for Ab-A and Ab-B. Southern blots (using a 3'AOX TT sequence probe) identified haploid strains Hc19, Hc25, Hc13, and Hc17 containing 1, 2, 3, and 4 copies, respectively, of the Ab-C heavy chain gene (FIG. 32), and strains Lc7, Lc6, Lc11, Lc19, Lc15, and Lc17 containing 1 through 6 copies, respectively, of the Ab-C light chain gene (FIG. 33). In FIG. 32, the labels A, B, C, and D on the right side of the blot indicate the expected size of the fragment containing one to four copies, respectively, of the Ab-C heavy chain gene integrated into the *AOX1* transcription terminator locus. In FIG. 33, the labels A, B, C, D, E, and F at the right side of the blot indicated the expected fragment size containing one to six copies, respectively, of the Ab-C light chain gene integrated at the *AOX1* transcription terminator locus. Asterisks indicated haploids selected for further mating.

To further increase the number of copies of the heavy and light chain genes available in the panel, additional gene copies were integrated into a second locus. The total copy number of a specific integrated gene is therefore calculated by adding the number of copies from both integration loci. Specifically, additional copies of a gene encoding Ab-C heavy chain were integrated into the HIS4 TT locus in a strain already containing 3 or 4 copies of the Ab-C heavy chain gene integrated in the *AOX1* transcription terminator locus, thereby introducing one or two additional copies of the heavy chain gene, for a total of five or six copies. Similarly, additional copies of a gene encoding the Ab-C light chain were integrated into the HIS4 TT locus in a strain already containing 3 or 4 copies of the Ab-C light chain gene integrated in the *AOX1* transcription terminator locus, thereby introducing two additional copies of the light chain gene, for a total of five or six copies.

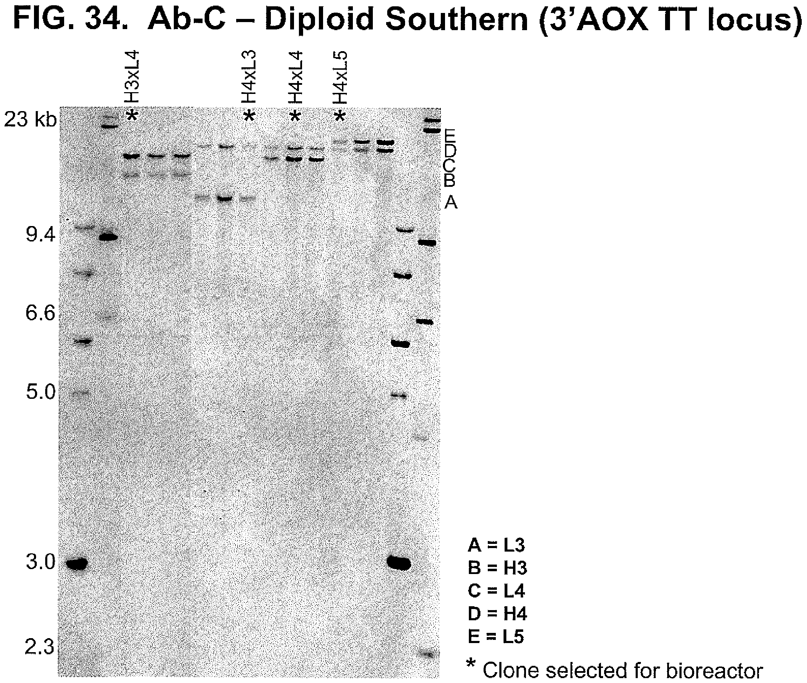

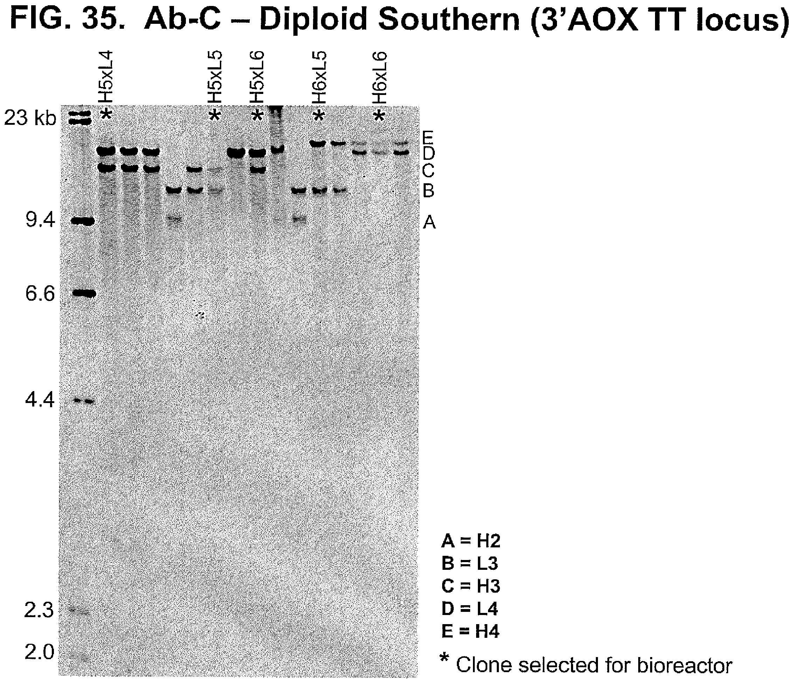

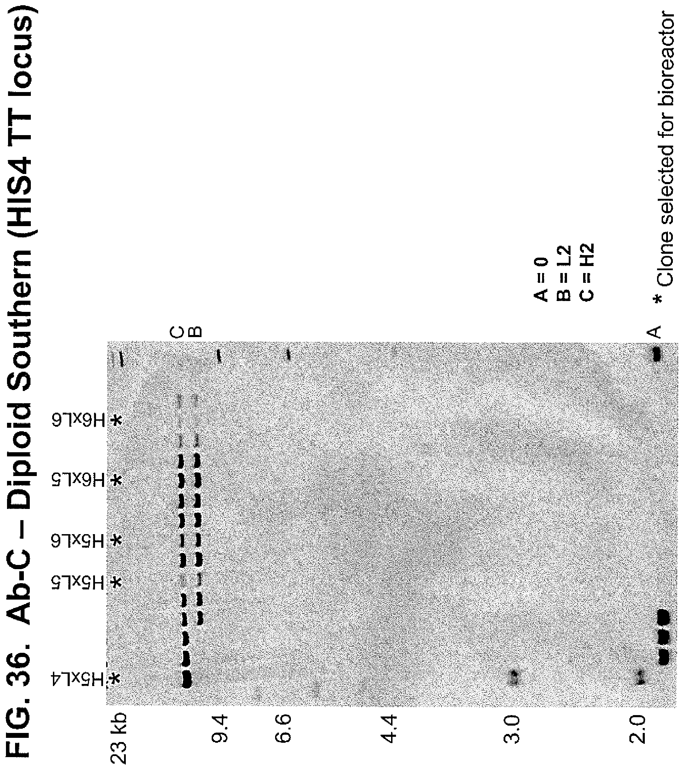

Diploid strains were then produced by mating haploids containing heavy and light chain genes. A total of nine different strains were produced containing between three and six copies of the heavy chain gene and between three and six copies of the light chain gene. For each diploid strain, the number of copies of the heavy and light chain genes is indicated by the strain identifier (e.g., Ab-C-H4xL5 indicates 4 copies of the heavy chain gene and five copies of the light chain gene) and the loci from which the genes are expressed are identified in Table 3, above. Gene copy numbers in the diploid strains were reconfirmed by Southern blots shown in **FIGS. 34-35** (3'AOX TT probe) and **FIG. 36** (HIS4 TT probe). Multiple isolates of each diploid strain were generated, and asterisks in **FIGS. 34-36** indicate isolates selected for further use. In **FIG. 34****,** the labels B and D at the right side of the blot indicate the expected size of the fragment containing three or four copies, respectively, of the Ab-C heavy chain gene integrated into the *AOX1* transcription terminator locus, and the labels A, C, and E indicate the expected size of the fragment containing three to five copies, respectively, of the Ab-C light chain gene integrated into the *AOX1* transcription terminator locus. In **FIG. 35****,** the labels A, C, and E at the right side of the blot indicate the expected size of the fragment containing two to four copies, respectively, of the Ab-C heavy chain gene integrated into the *AOX1* transcription terminator locus, and the labels B and D indicate the expected size of the fragment containing three and four copies, respectively, of the Ab-C light chain gene integrated into the *AOX1* transcription terminator locus. In **FIG. 36** the label A indicates the expected size of the fragment containing zero copies of the Ab-C heavy or light chain gene integrated into the HIS4 TT locus, the label B indicates the expected size of the fragment containing two copies of the Ab-C light chain gene integrated into the HIS4 TT locus, and label C indicates the expected size of the fragment containing two copies of the Ab-C heavy chain gene integrated into the HIS4 TT locus.

The Ab-C-H3xL3 expression strain was constructed using a slightly different method. Prior to transformation, each expression vector (**FIGS. 18-19**) was linearized in the pGAP sequence using AvrII to direct integration of the vectors into the GAP promoter locus. Haploid *P. pastoris* JC231 (Lys⁻) or JC239 (Met⁻) cells were then transformed individually with the linearized heavy or light chain vectors, respectively, by electroporation following a modified protocol from Pichia Protocols, Second Edition (Methods in Molecular Biology, Cregg, JM, Ed. 2007. Humana Press, Totowa, NJ). Successful transformants were selected on YPDS agar plates containing Zeocin™. To generate a library of diploid clones that express full-length Ab-C, the haploid transformant colonies were pooled, mixed together, and spread on mating medium agar plates. These were incubated for 24 hours at 30°C. Cells were then scraped from the mating plates and streaked onto BYNB agar plates to select for diploid clones. The ability of each diploid clone to express full-length antibody was assessed by a colony lift/immunoblot method (Wung et al. Biotechniques 21 808-812 (1996)). Briefly, secreted antibodies produced by the clones were transferred to a nitrocellulose membrane by touching the membrane to the plates. The filters were processed using a Western blot protocol employing a Goat anti-Human F(ab')2 HRP (horseradish peroxidase) detection antibody. Using chemiluminescence detection, colonies that expressed elevated levels of Ab-C were visualized on film. A large fraction of these colonies were picked into 96-deep well plates containing 300 µL of BYPD (1% yeast nitrogen base, 2% peptone, 2% glucose, 0.1M potassium phosphate pH 6, and 50 µg/mL Zeocin™). Cultures were grown for 60 hours at 30°C with constant shaking. The resulting supernatants were assayed by a standard enzyme-linked immunosorbent assay (ELISA). From this, a single diploid clone was selected as having high expression of Ab-C. Southern blot analysis to determine copy numbers of heavy and light chain genes was subsequently performed on the final clone following the methods described above.

### EXAMPLE 5

### Methods for expression of antibodies in a bioreactor.

This example describes methods for production of antibodies in a bioreactor for further characterization or use.

First, an inoculum was expanded using the research cell bank using medium comprised of the following nutrients (%w/v): yeast extract 3%, anhydrous dextrose 4%, YNB 1.34%, Biotin 0.004% and 100 mM potassium phosphate. To generate the inoculum for the fermenters, the cell bank was expanded for approximately 24 hours in a shaking incubator at 30°C and 300 rpm. A 10% inoculum was then added to Labfors 2.5L working volume vessels containing 1 L sterile growth medium. The growth medium was comprised of the following nutrients: potassium sulfate 18.2 g/L, ammonium phosphate monobasic 36.4 g/L, potassium phosphate dibasic 12.8 g/L, magnesium sulfate heptahydrate 3.72 g/L, sodium citrate dihydrate 10 g/L, glycerol 40 g/L, yeast extract 30 g/L, PTM1 trace metals 4.35 mL/L, and antifoam 204 1.67 mL/L. The PTM1 trace metal solution was comprised of the following components: cupric sulfate pentahydrate 6 g/L, sodium iodide 0.08 g/L, manganese sulfate hydrate 3 g/L, sodium molybdate dihyrate 0.2 g/L, boric acid 0.02 g/L, cobalt chloride 0.5 g/L, zinc chloride 20 g/L, ferrous sulfate heptahydrate 65 g/L, biotin 0.2 g/L, and sulfuric acid 5 mL/L.

The bioreactor process control parameters were set as follows: Agitation 1000 rpm, airflow 1.35 standard liter per minute, temperature 28°C and pH was controlled (at 6) using ammonium hydroxide. No oxygen supplementation was provided.

Fermentation cultures were grown for approximately 12 to 16 hours until the initial glycerol was consumed as denoted by a dissolved oxygen spike. The cultures were starved for approximately three hours after the dissolved oxygen spike. After this starvation period, a bolus addition of ethanol was added to the reactor to reach 1% ethanol (w/v). The fermentation cultures were allowed to equilibrate for 15 to 30 minutes. Feed addition was initiated 30 minutes post-ethanol bolus and set at a constant rate of 1 mL/min for 40 minutes, then the feed pump was controlled by an ethanol sensor keeping the concentration of ethanol at 1% for the remainder of the run using an ethanol sensing probe (Raven Biotech). The feed was comprised of the following components: yeast extract 50 g/L, dextrose monohydrate 500 g/L, magnesium sulfate heptahydrate 3 g/L, and PTM1 trace metals 12 mL/L. Optionally, sodium citrate dihydrate (0.5g/L) was also added to the feed. The total fermentation time was approximately 90 hours (T90).

Antibody yield was then determined using analytic high pressure liquid chromatography (HPLC) and fitted with a Protein A affinity column. A purified antibody sample was used to determine a standard curve by integrating the optical absorbance at 280nm (A₂₈₀) in the HPLC peak.

### EXAMPLE 6

### Methods for determination of antibody yield and purity by HPLC

To analyze the purity of protein-A purified antibody preparations, size exclusion high-pressure liquid chromatography (SE-HPLC) was used. Briefly, an Agilent (Santa Clara, CA) 1200 Series HPLC with UV detection instrument was used. For sample separation, a TSKgel GS3000SWx1 7.8x300 mM column connected with a TSKgel Guard SWxl 6x40 mM from Tosoh Bioscience (King of Prussia, PA) was used. A 100 mM sodium phosphate, 200 mM sodium chloride pH 6.5 was used as mobile phase with a flow rate of 0.5 mL/min in isocratic mode and absorbance at UV 215nm was monitored. Before injection of samples the column was equilibrated until a stable baseline was achieved. Samples were diluted to a concentration of 1 mg/mL using mobile phase and a 30 µL volume was injected. To monitor column performance, BioRad (Hercules, CA) gel filtration standards were used.

### EXAMPLE 7

### Methods for antibody purification by Protein-A affinity

For characterization of *Pichia pastoris* expressed antibodies, protein A purification was performed. Briefly, approximately 20 mL of 0.2µ clarified supernatants from harvested fermentation broth were diluted with the same volume of equilibration buffer (20 mM Histidine pH6). From this diluted broth, 20 mL were then loaded onto a pre-equilibrated 1 mL HiTrap MabSelect Sure column (GE, Piscataway, NJ). The column was subsequently washed using 30 column volumes of equilibration buffer. The antibody bound onto the column was eluted using a step gradient into 100% elution buffer (100 mM Citric Acid pH 3.0). One mL fractions were collected and immediately neutralized using 100µL of 2M Tris buffer pH 8.0. Protein containing fractions were determined by measuring absorbance at 280nM and protein-containing fractions were pooled.

### EXAMPLE 8

### Methods for characterization of impurities by SDS-PAGE, Western Blotting, and Lectin column purification

SE-HPLC allowed us to quantitate the product-associated variant being modulated by the ratio of light chain to heavy chain expression in the different strains. To characterize the nature of this product-associated variant we did western blot analysis and purification using an affinity lectin column. Briefly, for western blot analysis, 5µg samples were prepared in LDS sample loading buffer with NuPAGE reducing agent (Invitrogen, Carlsbad, CA), heated at 70C for 10 minutes. The equivalent of four micrograms of sample were then loaded onto a 4-12% BisTris gradient gel and separated by electrophoresis using MES running buffer (Invitrogen, Carlsbad, CA). The proteins separated in the gel were then blotted onto a nitrocellulose membrane using an I-Blot (Invitrogen, Carlsbad, CA) and blocked for 60 minutes using a blocking solution (10% powdered milk solution in DPBS-T [DPBS solution containing 0.1% Tween-20 (Invitrogen, Carlsbad, CA)]). The blocked membrane was then probed for 30 minutes with a goat-anti-human FC peroxidase conjugated antibody (Jackson Immunoresearch Laboratories, Inc, West Grove, PA), using a 1:10,000 dilution in blocking solution. The blot was then washed for five minutes in a DPBS 0.1% Tween solution for a total of four times. For development the ECL advance chemiluminescent reagent (Amersham/GE, Piscataway, NJ) was used and the image was captured using a CCD camara (Alpha Innotech/Cell Biosciences, Santa Clara, CA).

To further characterize the product-associated variant, we used agarose-bound *Galanthus nivalis* lectin (Vector Laboratories, Inc, Burlingame, CA). *Galantus nivalis* lectin is a small molecular weight protein that binds to mannose-containing proteins and does not require Ca⁺⁺ or Mn⁺⁺ for binding. For binding, 2 mL of resin was washed with 14 mL of DBS four times by resuspension-centrifugation. After wash, the beads were resuspended to a final 50% slurry concentration in DPBS and 400µL were added to Protein-A purified antibody samples containing 1.5 to 4mgs of protein. The agarose-bound lectin was then incubated with the antibody at room temperature for 2.5 hours with continuous mixing. At the end of this incubation, the sample was spun, the supernatant collected and labeled as "flow-thru". The beads were then transferred onto an empty Bio-Rad drip column (Hercules, CA) and washed by gravity using DPBS. A total of 6 mL were used for washing using 0.5 mL at the time and monitoring 280nm absorbance. Bound protein was eluted using 0.2M methyl-alpha-D-mannopyranoside. Samples, load and eluted proteins, were then analyzed by SDS-PAGE, and Western blot using an anti-Human Fc specific reagent, and SE-HPLC. SDS-PAGE was carried out using precast polyacrylamide gels (NuPAGE® Bis-Tris Gels) containing a 4%-12% polyacrylamide gradient, using NuPAGE® MES SDS running buffer and NuPAGE® LDS Sample Buffer (all from Invitrogen, Carlsbad, Ca.) in accord with the manufacturer's instructions. Prior to loading, samples were reduced using the NuPAGE® Sample Reducing Agent (Invitrogen, Carlsbad, Ca.) in accord with the manufacturer's instructions.

The above description of various illustrated embodiments of the invention is not intended to be exhaustive and the teachings provided herein of the invention can be applied to other purposes, other than the examples described above.

The invention may be practiced in ways other than those particularly described in the foregoing description and examples. Numerous modifications and variations of the invention are possible in light of the above teachings and, therefore, are within the scope of the appended claims.

Certain teachings related to humanization of rabbit-derived monoclonal antibodies and preferred sequence modifications to maintain antigen binding affinity were disclosed in International Application No. PCT/US2008/064421, corresponding to International Publication No. WO/2008/144757, entitled "Novel Rabbit Antibody Humanization Methods and Humanized Rabbit Antibodies", filed May 21, 2008.

Certain teachings related to producing antibodies or fragments thereof using mating competent yeast and corresponding methods were disclosed in U.S. Patent application no. 11/429,053, filed May 8, 2006, (U.S. Patent Application Publication No. US2006/0270045).

## Claims

1. A method of identifying a diploid *Pichia pastoris* host cell that produces a desired antibody with a greater purity than another diploid *Pichia pastoris* host cell, said method comprising:
(a) providing a panel of diploid *Pichia pastoris* host cells, said panel comprising at least two diploid *Pichia pastoris* host cells that each comprise differing numbers of copies of one or more of the genes that provide for the expression of the subunits of the desired antibody, wherein said genes that provide for the expression of the subunits of the desired antibody comprise genes encoding the light chain and the heavy chain of the desired antibody;
(b) culturing each of said diploid *Pichia pastoris* host cells to express the desired antibody;
(c) measuring the purity of the desired antibody produced by each of said diploid *Pichia pastoris* host cells; and
(d) identifying a diploid *Pichia pastoris* host cell that produces said desired antibody with a greater purity than another diploid *Pichia pastoris* host cell in said panel of diploid *Pichia pastoris* host cells that produce the desired antibody.

2. The method of claim 1, wherein said panel of *Pichia pastoris* host cells comprises a heterogenous population of diploid *Pichia pastoris* cells, wherein said heterogenous population of diploid *Pichia pastoris* cells comprises cells comprising different copy number combinations of said genes encoding subunits of said desired antibody relative to one another and *Pichia pastoris* cells comprising the same copy number combinations of said genes encoding subunits of said desired antibody relative to one another.

3. The method of claim 1 or 2, wherein the respective number of copies of the gene encoding the heavy chain of said desired antibody and the number of copies of the gene encoding the light chain of said desired antibody in said host cells are; 2 and 1, 3 and 1, 4 and 1, 5 and 1, 6 and 1, 7 and 1, 8 and 1, 9 and 1 , 10 and 1, 1 and 2, 2 and 2, 3 and 2, 4 and 2, 5 and 2, 6 and 2, 7 and 2, 8 and 2, 9 and 2, 10 and 2, 1 and 3, 2 and 3, 3 and 3, 4 and 3, 5 and 3, 6 and 3, 7 and 3, 8 and 3, 9 and 3, 10 and 3, 1 and 4, 2 and 4, 3 and 4, 4 and 4, 5 and 4, 6 and 4, 7 and 4, 8 and 4, 9 and 4, 10 and 4, 1 and 5, 2 and 5, 3 and 5, 4 and 5, 5 and 5, 6 and 5, 7 and 5, 8 and 5, 9 and 5, 10 and 5, 1 and 6, 2 and 6, 3 and 6, 4 and 6, 5 and 6, 6 and 6, 7 and 6, 8 and 6, 9 and 6, 10 and 6, 1 and 7, 2 and 7, 3 and 7, 4 and 7, 5 and 7, 6 and 7, 7 and 7, 8 and 7, 9 and 7, 10 and 7, 1 and 8, 2 and 8, 3 and 8, 4 and 8, 5 and 8, 6 and 8, 7 and 8, 8 and 8, 9 and 8, 10 and 8, 1 and 9, 2 and 9, 3 and 9, 4 and 9, 5 and 9, 6 and 9, 7 and 9, 8 and 9, 9 and 9, 10 and 9, 1 and 10, 2 and 10, 3 and 10, 4 and 10, 5 and 10, 6 and 10, 7 and 10, 8 and 10, 9 and 10, 10 and 10.

4. The method of any one of claims 1-3, wherein:
(i) the panel of diploid *Pichia pastoris* host cells includes cells that express up to 15 copies of each gene that encode the desired antibody, and represent substantially all possible copy number combinations of each subunit relative to the other subunit(s);
(ii) the panel of diploid *Pichia pastoris* host cells includes cells that express up to 12 copies of each gene that encode the desired antibody, and represent substantially all possible copy number combinations of each subunit relative to the other subunit(s);
(iii) the panel of diploid *Pichia pastoris* host cells includes cells that express up to 10 copies of each gene that encode the desired antibody, and represent substantially all possible copy number combinations of each subunit relative to the other subunit(s);
(iv) the panel of diploid *Pichia pastoris* host cells includes cells that express up to 8 copies of each gene that encode the desired antibody, and represent substantially all possible copy number combinations of each subunit relative to the other subunit(s); or
(v) the panel of diploid *Pichia pastoris* host cells includes cells that express up to 6 copies of each gene that encode the desired antibody, and represent substantially all possible copy number combinations of each subunit relative to the other subunit(s).

5. The method of any one of claims 1-4, wherein said purity is determined by measuring the relative proportion of one or more glycosylated antibody chains and optionally wherein one or more of said glycosylated antibody chains include a glycosylated antibody heavy chain variant.

6. The method of any one of claims 1-5, wherein:
(i) said panel of diploid *Pichia pastoris* host cells is produced by a mating or fusion of haploid *Pichia pastoris* cells containing differing numbers of copies of the genes encoding one or more subunits of said desired antibody, whereby said mating produces diploid *Pichia pastoris* cells containing differing numbers of copies of the genes encoding the subunits of said desired antibody; or
(ii) said panel of diploid *Pichia pastoris* host cells is produced by a mating or fusion of haploid *Pichia pastoris* cells containing differing numbers of copies of the genes encoding one or more subunits of said desired antibody, whereby said mating produces diploid *Pichia pastoris* cells containing differing numbers of copies of the genes encoding the subunits of said desired antibody and wherein said genes that provide for the expression of the subunits of the desired antibody are integrated into the genome of said haploid *Pichia pastoris* cells.

7. A method according to any of claims 1-6, wherein said identified diploid *Pichia pastoris* host cell is used to develop a production culture.

8. A method according to any of claims 1-7, wherein said genes that provide for the expression of the subunits of the desired antibody are integrated into the genome of said diploid *Pichia pastoris* host cells.

9. The method of claim 8, wherein the genes that provide for the expression of the subunits of the desired antibody are integrated into one or more genomic loci selected from the group consisting of the pGAP, 3' AOX TT, PpURA5, OCH1, AOX1, HIS4, GAP, ARG, and HIS4 TT loci.

10. A method according to any one of claims 1-9, wherein:
(i) at least one of said genes encoding said subunits of the desired antibody are expressed under the control of an inducible or constitutive promoter;
(ii) at least one of said genes encoding said subunits of the desired antibody are expressed under the control of an inducible or constitutive promoter, wherein said inducible promoter is selected from the group consisting of the GAP, CUP1, AOX1, and FLD1 promoters;
(iii) at least one of said genes encoding said subunits of the desired antibody are expressed under the control of an inducible or constitutive promoter, wherein at least one of said genes encoding said subunits of the desired antibody are expressed under the control of a promoter selected from the group consisting of: the AOX1, ICL1, glyceraldehyde-3-phosphate dehydrogenase (GAP), FLD1 , ADH1, alcohol dehydrogenase II, GAL4, PHO3, PHO5, and Pyk promoters, chimeric promoters derived therefrom, yeast promoters, mammalian promoters, insect promoters, plant promoters, reptile promoters, amphibian promoters, viral promoters, and avian promoters;
(iv) the desired antibody is purified from said diploid *Pichia pastoris* host cells;
(v) the desired antibody is purified from an intracellular component, cytoplasm, nucleoplasm, or a membrane of said diploid *Pichia pastoris* host cells;
(vi) the diploid *Pichia pastoris* host cell secretes said desired antibody into the culture medium;
(vii) the desired antibody is a monospecific or bispecific antibody;
(viii) at least two diploid *Pichia pastoris* host cells comprise differing numbers of copies of a gene encoding a subunit of said desired antibody;
(ix) at least one diploid *Pichia pastoris* host cell in said panel comprises a gene encoding at least one subunit of the desired antibody whose expression is driven by a different promoter than the promoter that drives the expression of the corresponding gene in a different diploid *Pichia pastoris* host cell in said panel;
(x) at least one diploid *Pichia pastoris* host cell in said panel comprises a polycistronic gene comprising more than one sequence encoding a subunit of the desired antibody;
(xi) said desired antibody specifically binds to IL-6, TNF-alpha, CGRP, PCSK9, or NGF;
(xii) said desired antibody comprises a human antibody or a humanized antibody or fragment thereof;
(xiii) said desired antibody comprises a humanized antibody or fragment thereof and said humanized antibody is of mouse, rat, rabbit, goat, sheep, or cow origin, preferably rabbit origin;
(xiv) said desired antibody comprises a monovalent, bivalent, or multivalent antibody;
(xv) at least one of said genes that provide for expression of a subunit of said desired antibody in at least one of said diploid *Pichia pastoris* host cells in said panel is optimized for expression in said diploid *Pichia pastoris* host cell; or
(xvi) said desired antibody is glycosylated and the purity of said desired antibody is assessed by measuring the fraction of the desired antibody produced by said diploid *Pichia pastoris* host cell that is non-glycosylated, is contained in antibody complexes having the expected apparent hydrodynamic radius, and /or specifically binds a target of said desired antibody.

11. The method of any one of claims 1-10, wherein:
(i) the copy number of the genes encoding the desired antibody is stable for 20 generations;
(ii) the copy number of the genes encoding the desired antibody is stable for 50 generations;
(iii) the copy number of the genes encoding the desired antibody is stable for 100 generations;
(iv) the copy number of the genes encoding the desired antibody is stable for 500 generations;
(v) the copy number of the genes encoding the desired antibody is stable for 1000 generations;
(vi) the gene copies encoding at least one of the subunits of said desired antibody are integrated into two or more loci;
(vii) the gene copies encoding at least one of the subunits of said desired antibody are integrated into three or more loci;
(viii) the gene copies encoding at least one of the subunits of said desired antibody are integrated into two, three or more loci wherein each locus contains no more than 5 copies of a given subunit;
(ix) the gene copies encoding at least one of the subunits of said desired antibody are integrated into two, three or more loci wherein each locus contains no more than 4 copies of a given subunit; or
(x) the gene copies encoding at least one of the subunits of said desired antibody are integrated into two, three or more loci wherein each locus contains no more than 3 copies of a given subunit.

12. The method of any one of claims 1-11, further comprising recombinantly producing said desired antibody from said diploid *Pichia pastoris* host cell identified as a diploid *Pichia pastoris* host cell that produces a desired antibody with a greater purity, wherein the diploid *Pichia pastoris* host cell (i) comprises one or more genes encoding each subunit of the desired antibody; or (ii) comprises between two and ten copies of a gene encoding each subunit of said desired antibody,
wherein the method comprises:
(a) providing the diploid *Pichia pastoris* host cell; and
(b) culturing said diploid *Pichia pastoris* host cell to express said desired antibody.

13. The method of claim 12, wherein the diploid *Pichia pastoris* host cell comprises one or more genes encoding each subunit of the desired antibody and is identified by the method of any one of claims 1-11 as a diploid *Pichia pastoris* host cell that produces a desired antibody with a greater purity, and wherein:
(i) the method further comprises the purification of said desired antibody; or
(ii) said diploid *Pichia pastoris* host cell produces an antibody titer of at least 100 mg / L, at least 150 mg / L, at least 200 mg / L, at least 250 mg / L, at least 300 mg / L, between 100 and 300 mg / L, between 100 and 500 mg / L, between 100 and 1000 mg / L, at least 1000 mg / L, at least 1250 mg/liter, at least 1500 mg/liter, 1750 mg/liter, 2000 mg liter, or more.

14. The method of claim 12, wherein said diploid *Pichia pastoris* host cell is produced by mating or fusion of two parental cells, wherein each parental cell comprises one or more copies of at least one gene encoding a subunit of said desired antibody; optionally wherein at least one copy of each of two different genes each encoding a subunit of said desired antibody are integrated into the same locus of two homologous chromosomes of said diploid *Pichia pastoris* host cell.

15. The method of claim 14, wherein:
(i) at least one of the genes that provide for expression of said desired antibody are integrated into genome of said diploid *Pichia pastoris* host cell;
(ii) at least one of the genes that provide for expression of said desired antibody is contained on one or more extrachromosomal elements, plasmids, or artificial chromosomes;
(iii) said diploid *Pichia pastoris* host cell comprises more copies of the gene that provide for the expression of the light chain of said desired antibody than copies of the gene that provide for expression of the heavy chain of said desired antibody;
(iv) said diploid *Pichia pastoris* host cell comprises more copies of the gene that provide for the expression of the heavy chain of said desired antibody than copies of the gene that provide for expression of the light chain of said desired antibody;
(v) said diploid *Pichia pastoris* host cell comprises a number of copies of the gene that provide for the expression of the light chain of said desired antibody that is equal to the number of copies of the gene that provide for expression of the heavy chain of said desired antibody; or
(vi) wherein the respective number of copies of the gene encoding the heavy chain of said desired antibody and the number of copies of the gene encoding the light chain of said desired antibody in said diploid *Pichia pastoris* host cell are: 2 and 2, 2 and 3, 3 and 3, 3 and 4, 3 and 5, 4 and 3, 4 and 4, 4 and 5, 4 and 6, 5 and 4, 5 and 5, 5 and 6, or 5 and 7.

16. The method according to claim 12, wherein the diploid *Pichia pastoris* host cell comprises between two and ten copies of a gene encoding each subunit of said desired antibody, wherein said host cell is produced by mating or fusion of two parental cells, wherein each parental cell comprises one or more copies of genes encoding one or more subunits of said multi-subunit complex and wherein the method of recombinantly producing a desired antibody further optionally includes one or more of the following:
(i) at least one copy of each of two different genes each encoding a subunit of said desired antibody are integrated into the same locus of two homologous chromosomes of said diploid *Pichia pastoris* host cell;
(ii) at least one of said genes that provide for expression of the subunits of said desired antibody are integrated into one or more genomic loci selected from the group consisting of the pGAP locus and the HIS4 TT locus;
(iii) at least one of said genes encoding said desired antibody are expressed under control of an inducible or constitutive promoter;
(iv) at least one of said genes encoding said desired antibody are expressed under control of an inducible or constitutive promoter, wherein said inducible promoter is selected from the group consisting of the GAP, AOX1, CUP1, and FLD1 promoters;
(v) at least one of said genes encoding the desired antibody light chain and / or heavy chain are expressed under control of a promoter selected from the group consisting of: the AOX1, ICL1, glyceraldehyde-3-phosphate dehydrogenase (GAP), FLD1, ADH1, alcohol dehydrogenase II, GAL4, PHO3, PHO5, and Pyk promoters, chimeric promoters derived therefrom, yeast promoters, mammalian promoters, insect promoters, plant promoters, reptile promoters, amphibian promoters, viral promoters, and avian promoters;
(vi) the diploid *Pichia pastoris* host cell secretes said desired antibody into the culture medium;
(vii) the desired antibody produced by the diploid *Pichia pastoris* host cell is a monospecific or bispecific antibody;
(viii) the desired antibody produced by the diploid *Pichia pastoris* host cell specifically binds to IL-6, TNF-alpha, CGRP, PCSK9, or NGF;
(ix) the desired antibody produced by the diploid *Pichia pastoris* host cell is a humanized antibody or fragment thereof which optionally specifically binds to IL-6, TNF-alpha, CGRP, PCSK9, or NGF;
(x) the desired antibody produced by the diploid *Pichia pastoris* host cell is a humanized antibody of rabbit origin;
(xi) the desired antibody produced by the diploid *Pichia pastoris* host cell is a monovalent, bivalent, or multivalent antibody;
(xii) at least one of said copies of a gene encoding a subunit of said desired antibody is optimized for expression in said diploid *Pichia pastoris* host cell;
(xiii) the number of copies of a gene encoding the light chain of said desired antibody is greater than or equal to said number of copies of a gene encoding the heavy chain of said desired antibody;
(xiv) the method produces a greater purity of said desired antibody than a method using a strain containing a single copy of each gene encoding a subunit of said desired antibody;
(xv) the method produces a greater purity of said desired antibody than a method using a strain containing a single copy of each gene encoding a subunit of said desired antibody, wherein said purity is determined by measuring the mass of glycosylated heavy and/or light chain polypeptides as a percentage of total mass of heavy and/or light chain polypeptides; or
(xvi) at least one subunit of said desired antibody comprises a secretion signal, optionally wherein the secretion signal comprises a polypeptide selected from the group consisting of: chicken lysozyme (CLY) signal peptide; CLY-L8; S. cerevisiae invertase (SUC2) signal peptide; MF-alpha (Prepro); MF-alpha (Pre)-apv; MF-alpha (Pre)-apv-SLEKR; MF-alpha (Prepro)-(EA)3; aF signal peptide; KILM1 signal peptide; repressible acid phosphatase (PHOI) signal peptide; A. niger GOX signal peptide; Schwanniomyces occidentalis glucoamylase gene (GAM1) signal peptide; human serum albumin (HSA) signal peptide with pro-sequence; human serum albumin (HSA) signal peptide without pro-sequence; ISN signal peptide; IFN signal peptide; HGH signal peptide; phytohemagglutinin (PHA); Silkworm lysozyme; Human lysozyme (LYZI); activin receptor type-1; activin type II receptor; P. pastoris immunoglobulin binding protein (PpBiP); human antibody 3D6 light chain leader; and any combination thereof.

## Patentansprüche

1. Verfahren zum Identifizieren einer diploiden *Pichia pastoris* Wirtszelle, die einen gewünschten Antikörper mit einer höheren Reinheit als eine andere diploide *Pichia pastoris* Wirtszelle produziert, wobei das genannte Verfahren Folgendes beinhaltet:
(a) Bereitstellen eines Panel von diploiden *Pichia pastoris* Wirtszellen, wobei das genannte Panel wenigstens zwei diploide *Pichia pastoris* Wirtszellen umfasst, die jeweils unterschiedliche Anzahlen von Kopien von einem oder mehreren der Gene umfassen, die die Expression der Untereinheiten des gewünschten Antikörpers ermöglichen, wobei die genannten Gene, die die Expression der Untereinheiten des gewünschten Antikörpers ermöglichen, Gene umfassen, die die leichte Kette und die schwere Kette des gewünschten Antikörpers codieren;
(b) Kultivieren jeder der genannten diploiden *Pichia pastoris* Wirtszellen zum Exprimieren des gewünschten Antikörpers;
(c) Messen der Reinheit des von jeder der genannten diploiden *Pichia pastoris* Wirtszellen produzierten gewünschten Antikörpers; und
(d) Identifizieren einer diploiden *Pichia pastoris* Wirtszelle, die den genannten gewünschten Antikörper mit einer höheren Reinheit als eine andere diploide *Pichia pastoris* Wirtszelle in dem genannten Panel von diploiden *Pichia pastoris* Wirtszellen produziert, die den gewünschten Antikörper produzieren.

2. Verfahren nach Anspruch 1, wobei das genannte Panel von *Pichia pastoris* Wirtszellen eine heterogene Population von diploiden *Pichia pastoris* Zellen umfasst, wobei die genannte heterogene Population von diploiden *Pichia pastoris* Zellen Zellen umfasst, die unterschiedliche Kopienzahlkombinationen der genannten Gene umfassen, die Untereinheiten des genannten gewünschten Antikörpers relativ zueinander codieren, und *Pichia pastoris* Zellen, die dieselben Kopienzahlkombinationen der genannten Gene umfassen, die Untereinheiten des genannten gewünschten Antikörpers relativ zueinander codieren.

3. Verfahren nach Anspruch 1 oder 2, wobei die jeweilige Anzahl von Kopien des Gens, das die schwere Kette des gewünschten Antikörpers codiert, und die Anzahl von Kopien des Gens, das die leichte Kette des gewünschten Antikörpers in den genannten Wirtszellen codiert, wie folgt sind: 2 und 1, 3 und 1, 4 und 1, 5 und 1, 6 und 1, 7 und 1, 8 und 1, 9 und 1, 10 und 1, 1 und 2, 2 und 2, 3 und 2, 4 und 2, 5 und 2, 6 und 2, 7 und 2, 8 und 2, 9 und 2, 10 und 2, 1 und 3, 2 und 3, 3 und 3, 4 und 3, 5 und 3, 6 und 3, 7 und 3, 8 und 3, 9 und 3, 10 und 3, 1 und 4, 2 und 4, 3 und 4, 4 und 4, 5 und 4, 6 und 4, 7 und 4, 8 und 4, 9 und 4, 10 und 4, 1 und 5, 2 und 5, 3 und 5, 4 und 5, 5 und 5, 6 und 5, 7 und 5, 8 und 5, 9 und 5, 10 und 5, 1 und 6, 2 und 6, 3 und 6, 4 und 6, 5 und 6, 6 und 6, 7 und 6, 8 und 6, 9 und 6, 10 und 6, 1 und 7, 2 und 7, 3 und 7, 4 und 7, 5 und 7, 6 und 7, 7 und 7, 8 und 7, 9 und 7, 10 und 7, 1 und 8, 2 und 8, 3 und 8, 4 und 8, 5 und 8, 6 und 8, 7 und 8, 8 und 8, 9 und 8, 10 und 8, 1 und 9, 2 und 9, 3 und 9, 4 und 9, 5 und 9, 6 und 9, 7 und 9, 8 und 9, 9 und 9, 10 und 9, 1 und 10, 2 und 10, 3 und 10, 4 und 10, 5 und 10, 6 und 10, 7 und 10, 8 und 10, 9 und 10, 10 und 10.

4. Verfahren nach einem der Ansprüche 1-3, wobei:
(i) das Panel von diploiden *Pichia pastoris* Wirtszellen Zellen beinhaltet, die bis zu 15 Kopien jedes Gens exprimieren, das den gewünschten Antkörper codiert, und im Wesentlichen alle möglichen Kopienzahlkombinationen jeder Untereinheit relativ zu den ein oder mehreren anderen Untereinheiten repräsentieren;
(ii) das Panel von diploiden *Pichia pastoris* Wirtszellen Zellen beinhaltet, die bis zu 12 Kopien jedes Gens exprimieren, das den gewünschten Antkörper codiert, und im Wesentlichen alle möglichen Kopienzahlkombinationen jeder Untereinheit relativ zu den ein oder mehreren anderen Untereinheiten repräsentieren;
(iii) das Panel von diploiden *Pichia pastoris* Wirtszellen Zellen beinhaltet, die bis zu 10 Kopien jedes Gens exprimieren, das den gewünschten Antkörper codiert, und im Wesentlichen alle möglichen Kopienzahlkombinationen jeder Untereinheit relativ zu den ein oder mehreren anderen Untereinheiten repräsentieren;
(iv) das Panel von diploiden *Pichia pastoris* Wirtszellen Zellen beinhaltet, die bis zu 8 Kopien jedes Gens exprimieren, das den gewünschten Antkörper codiert, und im Wesentlichen alle möglichen Kopienzahlkombinationen jeder Untereinheit relativ zu den ein oder mehreren anderen Untereinheiten repräsentieren; oder
(v) das Panel von diploiden *Pichia pastoris* Wirtszellen Zellen beinhaltet, die bis zu 6 Kopien jedes Gens exprimieren, das den gewünschten Antkörper codiert, und im Wesentlichen alle möglichen Kopienzahlkombinationen jeder Untereinheit relativ zu den ein oder mehreren anderen Untereinheiten repräsentieren.

5. Verfahren nach einem der Ansprüche 1-4, wobei die genannte Reinheit durch Messen des relativen Anteils von einer oder mehreren glykosylierten Antikörperketten bestimmt wird und wobei optional eine oder mehrere der genannten glykosylierten Antikörperketten eine glykosylierte Antikörper-Schwerkettenvariante beinhaltet.

6. Verfahren nach einem der Ansprüche 1-5, wobei:
(i) das genannte Panel von diploiden *Pichia pastoris* Wirtszellen durch eine Paarung oder Fusion von haploiden *Pichia pastoris* Zellen produziert wird, die unterschiedliche Anzahlen von Kopien der Gene enthalten, die eine oder mehrere Untereinheiten des genannten gewünschten Antikörpers codieren, so dass die genannte Paarung diploide *Pichia pastoris* Zellen produziert, die unterschiedliche Anzahlen von Kopien der Gene enthalten, die die Untereinheiten des genannten gewünschten Antikörpers codieren; oder
(ii) das genannte Panel von diploiden *Pichia pastoris* Wirtszellen durch eine Paarung oder Fusion von haploiden *Pichia pastoris* Zellen produziert wird, die unterschiedliche Anzahlen von Kopien der Gene enthalten, die eine oder mehrere Untereinheiten des genannten gewünschten Antikörpers codieren, so dass die genannte Paarung diploide *Pichia pastoris* Zellen produziert, die unterschiedliche Anzahlen von Kopien der Gene enthalten, die die Untereinheiten des genannten gewünschten Antikörpers codieren, und wobei die genannten Gene, die die Expression der Untereinheiten des gewünschten Antikörpers ermöglichen, in das Genom der genannten haploiden *Pichia pastoris* Zellen integriert sind.

7. Verfahren nach einem der Ansprüche 1-6, wobei die genannte identifizierte diploide *Pichia pastoris* Wirtselle zum Entwickeln einer Produktionskultur benutzt wird.

8. Verfahren nach einem der Ansprüche 1-7, wobei die genannten Gene, die die Expression der Untereinheiten des gewünschten Antikörpers ermöglichen, in das Genom der genannten diploiden *Pichia pastoris* Wirtszellen integriert sind.

9. Verfahren nach Anspruch 8, wobei die Gene, die die Expression der Untereinheiten des gewünschten Antikörpers ermöglichen, in ein oder mehrere genomische Loci integriert sind, ausgewählt aus der Gruppe bestehend aus den Loci pGAP, 3' AOX TT, PpURA5, OCH1, AOX1, HIS4, GAP, ARG und HIS4 TT.

10. Verfahren nach einem der Ansprüche 1-9, wobei:
(i) wenigstens eines der genannten Gene, die die genannten Untereinheiten des gewünschten Antikörpers codieren, unter der Kontrolle eines induzierbaren oder konstitutiven Promotors exprimiert wird;
(ii) wenigstens eines der genannten Gene, die die genannten Untereinheiten des gewünschten Antikörpers codieren, unter der Kontrolle eines induzierbaren oder konstitutiven Promotors exprimiert wird, wobei der genannte induzierbare Promotor ausgewählt ist aus der Gruppe bestehend aus den Promotoren GAP, CUP1, AOX1 und FLD1;
(iii) wenigstens eines der genannten Gene, die die genannten Untereinheiten des gewünschten Antikörpers codieren, unter der Kontrolle eines induzierbaren oder konstitutiven Promotors exprimiert wird, wobei wenigstens eines der genannten Gene, die die genannten Untereinheiten des gewünschten Antikörpers codieren, unter der Kontrolle eines Promotors exprimiert wird, ausgewählt aus der Gruppe bestehend aus: den Promotoren AOX1, ICL1, Glyceraldehyd-3-phosphat-dehydrogenase (GAP), FLD1, ADH1, Alkoholdehydrogenase II, GAL4, PHO3, PHO5 und Pyk, davon abgeleiteten chimeren Promotoren, Hefepromotoren, Säugetierpromotoren, Insektenpromotoren, Pflanzenpromotoren, Reptilienpromotoren, Amphibienpromotoren, Viruspromotoren und Vogelpromotoren;
(iv) der gewünschte Antikörper von den genannten diploiden *Pichia pastoris* Wirtszellen gereinigt wird;
(v) der gewünschte Antikörper von einer intrazellulären Komponente, Zytoplasma, Nukleoplasma oder einer Membran der genannten diploiden *Pichia pastoris* Wirtszellen gereinigt wird;
(vi) die diploide *Pichia pastoris* Wirtszelle den genannten gewünschten Antikörper in das Kulturmedium sekretiert;
(vii) der gewünschte Antikörper ein monospezifischer oder bispezifischer Antikörper ist;
(viii) wenigstens zwei diploide *Pichia pastoris* Wirtszellen unterschiedliche Anzahlen von Kopien eines Gens umfassen, das eine Untereinheit des genannten gewünschten Antikörpers codiert;
(ix) wenigstens eine diploide *Pichia pastoris* Wirtszelle in dem genannten Panel ein Gen umfasst, das wenigstens eine Untereinheit des gewünschten Antikörpers codiert, dessen Expression durch einen anderen Promotor als den Promotor angetrieben wird, der die Expression des entsprechenden Gens in einer anderen diploiden *Pichia pastoris* Wirtszelle in dem genannten Panel antreibt;
(x) wenigstens eine diploide *Pichia pastoris* Wirtszelle in dem genannten Panel ein polycistronisches Gen umfasst, das mehr als eine Sequenz umfasst, die eine Untereinheit des gewünschten Antkörpers codiert;
(xi) der genannte gewünschte Antikörper sich speziell an IL-6, TNF-alpha, CGRP, PCSK9 oder NGF bindet;
(xii) der genannte gewünschte Antikörper einen humanen Antikörper oder einen humanisierten Antikörper oder ein Fragment davon umfasst;
(xiii) der genannte gewünschte Antikörper einen humanisierten Antikörper oder ein Fragment davon umfasst und der genannte humanisierte Antikörper von Maus, Ratte, Kaninchen, Ziege, Schaf oder Kuh, vorzugsweise von Kaninchen abstammt;
(xiv) der genannte gewünschte Antikörper einen einwertigen, zweiwertigen oder mehrwertigen Antikörper umfasst;
(xv) wenigstens eines der genannten Gene, die die Expression einer Untereinheit des genannten gewünschten Antikörpers in wenigstens einer der genannten diploiden *Pichia pastoris* Wirtszellen in dem genannten Panel ermöglichen, für eine Expression in der genannten diploiden *Pichia pastoris* Wirtszelle optimiert wird; oder
(xvi) der genannte gewünschte Antikörper glykosyliert ist und die Reinheit des genannten gewünschten Antikörpers durch Messen der Fraktion des gewünschten Antikörpers beurteilt wird, produziert von der genannten diploiden *Pichia pastoris* Wirtszelle, die nicht glykosyliert ist, in Antikörperkomplexen mit dem erwarteten scheinbaren hydrodynamischen Radius enthalten ist und/oder speziell ein Ziel des genannten gewünschten Antikörpers bindet.

11. Verfahren nach einem der Ansprüche 1-10, wobei:
(i) die Kopienzahl der den gewünschten Antikörper codierenden Gene für 20 Generationen stabil ist;
(ii) die Kopienzahl der den gewünschten Antikörper codierenden Gene für 50 Generationen stabil ist;
(iii) die Kopienzahl der den gewünschten Antikörper codierenden Gene für 100 Generationen stabil ist;
(iv) die Kopienzahl der den gewünschten Antikörper codierenden Gene für 500 Generationen stabil ist;
(v) die Kopienzahl der den gewünschten Antikörper codierenden Gene für 1000 Generationen stabil ist;
(vi) die wenigstens eine der Untereinheiten des genannten gewünschten Antikörpers codierenden Genkopien in zwei oder mehr Loci integriert sind;
(vii) die wenigstens eine der Untereinheiten des genannten gewünschten Antikörpers codierenden Genkopien in drei oder mehr Loci integriert sind;
(viii) die wenigstens eine der Untereinheiten des genannten gewünschten Antikörpers codierenden Genkopien in zwei, drei oder mehr Loci integriert sind, wobei jeder Locus maximal 5 Kopien einer gegebenen Untereinheit enthält;
(ix) die wenigstens eine der Untereinheiten des genannten gewünschten Antikörpers codierenden Genkopien in zwei, drei oder mehr Loci integriert sind, wobei jeder Locus maximal 4 Kopien einer gegebenen Untereinheit enthält; oder
(x) die wenigstens eine der Untereinheiten des genannten gewünschten Antikörpers codierenden Genkopien in zwei, drei oder mehr Loci integriert sind, wobei jeder Locus maximal 3 Kopien einer gegebenen Untereinheit enthält.

12. Verfahren nach einem der Ansprüche 1-11, das ferner das rekombinante Produzieren des genannten gewünschten Antikörpers von der genannten diploiden *Pichia pastoris* Wirtszelle beinhaltet, die als eine diploide *Pichia pastoris* Wirtszelle identifiziert wird, die einen gewünschten Antikörper mit einer höheren Reinheit produziert, wobei die diploide *Pichia pastoris* Wirtszelle (i) ein oder mehrere jede Untereinheit des gewünschten Antikörpers codierende Gene umfasst; oder (ii) zwischen zwei und zehn Kopien eines jede Untereinheit des genannten gewünschten Antikörpers codierenden Gens umfasst,
wobei das Verfahren Folgendes beinhaltet:
(a) Bereitstellen der diploiden *Pichia pastoris* Wirtszelle; und
(b) Kultivieren der genannten diploiden *Pichia pastoris* Wirtszelle zum Exprimieren des genannten gewünschten Antikörpers.

13. Verfahren nach Anspruch 12, wobei die diploide *Pichia pastoris* Wirtszelle ein oder mehrere jede Untereinheit des gewünschten Antikörpers codierende Gene umfasst und mit dem Verfahren nach einem der Ansprüche 1-11 als eine diploide *Pichia pastoris* Wirtszelle identifiziert wird, die einen gewünschten Antikörper mit einer höheren Reinheit produziert, und wobei:
(i) das Verfahren ferner das Reinigen des genannten gewünschten Antikörpers beinhaltet; oder
(ii) die genannte diploide *Pichia pastoris* Wirtszelle einen Antikörpertiter von wenigstens 100 mg/l, wenigstens 150 mg/l, wenigstens 200 mg/l, wenigstens 250 mg/l, wenigstens 300 mg/l, zwischen 100 und 300 mg/l, zwischen 100 und 500 mg/l, zwischen 100 und 1000 mg/l, wenigstens 1000 mg/l, wenigstens 1250 mg/l, wenigstens 1500 mg/l, 1750 mg/l, 2000 mg/l oder mehr produziert.

14. Verfahren nach Anspruch 12, wobei die genannte diploide *Pichia pastoris* Wirtszelle durch Paarung oder Fusion von zwei Elternzellen produziert wird, wobei jede Elternzelle eine oder mehrere Kopien von wenigstens einem eine Untereinheit des genannten gewünschten Antikörpers codierenden Gen umfasst; wobei optional wenigstens eine Kopie jedes von zwei unterschiedlichen Genen, die jeweils eine Untereinheit des genannten gewünschten Antikörpers codieren, in denselben Locus von zwei homologen Chromosomen der genannten diploiden *Pichia pastoris* Wirtszelle integriert sind.

15. Verfahren nach Anspruch 14, wobei:
(i) wenigstens eines der Gene, die die Expression des genannten gewünschten Antikörpers ermöglichen, in das Genom der genannten diploiden *Pichia pastoris* Wirtszelle integriert ist;
(ii) wenigstens eines der Gene, die die Expression des genannten gewünschten Antikörpers ermöglichen, auf einem oder mehreren extrachromosomalen Elementen, Plasmiden oder künstlichen Chromosomen enthalten ist;
(iii) die genannte diploide *Pichia pastoris* Wirtszelle mehr Kopien des Gens, das die Expression der leichten Kette des genannten gewünschten Antikörpers ermöglicht, umfasst als Kopien des Gens, das die Expression der schweren Kette des genannten gewünschten Antikörpers ermöglicht;
(iv) die genannte diploide *Pichia pastoris* Wirtszelle mehr Kopien des Gens, das die Expression der schweren Kette des genannten gewünschten Antikörpers ermöglicht, umfasst als Kopien des Gens, das die Expression der leichten Kette des genannten gewünschten Antikörpers ermöglicht;
(v) die genannte diploide *Pichia pastoris* Wirtszelle eine Anzahl von Kopien des Gens umfasst, das die Expression der leichten Kette des genannten gewünschten Antikörpers ermöglicht, die gleich der Anzahl von Kopien des Gens ist, das die Expression der schweren Kette des genannten gewünschten Antikörpers ermöglicht; oder
(vi) wobei die jeweilige Anzahl von Kopien des Gens, das die schwere Kette des genannten gewünschten Antikörpers codiert, und die Anzahl von Kopien des Gens, das die leichte Kette des gewünschten Antikörpers in der genannten diploiden *Pichia pastoris* Wirtszelle codiert, wie folgt sind: 2 und 2, 2 und 3, 3 und 3, 3 und 4, 3 und 5, 4 und 3, 4 und 4, 4 und 5, 4 und 6, 5 und 4, 5 und 5, 5 und 6 oder 5 und 7.

16. Verfahren nach Anspruch 12, wobei die diploide *Pichia pastoris* Wirtszelle zwischen zwei und zehn Kopien eines Gens umfasst, das jede Untereinheit des genannten gewünschten Antikörpers codiert, wobei die genannte Wirtszelle durch Paarung oder Fusion von zwei Elternzellen produziert wird, wobei jede Elternzelle eine oder mehrere Kopien von Genen umfasst, die eine oder mehrere Untereinheiten des genannten Multi-Untereinheitskomplexes codiert, und wobei das Verfahren des rekombinanten Produzierens eines gewünschten Antikörpers ferner eines oder mehrere der Folgenden optional beinhaltet:
(i) wenigstens eine Kopie von jeweils zwei unterschiedlichen Genen, die jeweils eine Untereinheit des gewünschten Antikörpers codieren, ist in denselben Locus von zwei homologen Chromosomen der genannten diploiden *Pichia pastoris* Wirtszelle integriert;
(ii) wenigstens eines der genannten Gene, die die Expression der Untereinheiten des genannten gewünschten Antikörpers ermöglichen, ist in ein oder zwei genomische Loci integriert, ausgewählt aus der Gruppe bestehend aus dem pGAP Locus und dem HIS4 TT Locus;
(iii) wenigstens eines der genannten Gene, die den genannten gewünschten Antikörper codieren, ist unter der Kontrolle eines induzierbaren oder konstitutiven Promotors exprimiert;
(iv) wenigstens eines der genannten Gene, die den genannten gewünschten Antikörper codieren, ist unter der Kontrolle eines induzierbaren oder konstitutiven Promotors exprimiert, wobei der genannte induzierbare Promotor ausgewählt ist aus der Gruppe bestehend aus den Promotoren GAP, AOX1, CUP1 und FLD1;
(v) wenigstens eines der genannten Gene, die die genannte gewünschte Antikörperleichtkette und/oderschwerkette codieren, wird unter der Kontrolle eines Promotors exprimiert, ausgewählt aus der Gruppe bestehend aus: den Promotoren AOX1, ICL1, Glyceraldehyd-3-phosphatdehydrogenase (GAP), FLD1, ADH1, Alkoholdehydrogenase II, GAL4, PHO3, PHO5 und Pyk, davon abgeleiteten chimeren Promotoren, Hefepromotoren, Säugetierpromotoren, Insektenpromotoren, Pflanzenpromotoren, Reptilienpromotoren, Amphibienpromotoren, Viruspromotoren und Vogelpromotoren;
(vi) die diploide *Pichia pastoris* Wirtszelle sekretiert den genannten gewünschten Antikörper in das Kulturmedium;
(vii) der von der diploiden *Pichia pastoris* Wirtszelle produzierte gewünschte Antikörper ist ein monospezifischer oder bispezifischer Antikörper;
(viii) der von der diploiden *Pichia pastoris* Wirtszelle produzierte gewünschte Antikörper bindet sich spezifisch an IL-6, TNF-alpha, CGRP, PCSK9 oder NGF;
(ix) der von der diploiden *Pichia pastoris* Wirtszelle produzierte gewünschte Antikörper ist ein humanisierter Antikörper oder ein Fragment davon, der/das sich optional spezifisch an IL-6, TNF-alpha, CGRP, PCSK9 oder NGF bindet;
(x) der von der diploiden *Pichia pastoris* Wirtszelle produzierte gewünschte Antikörper ist ein humanisierter Antikörper von Kaninchenursprung;
(xi) der von der diploiden *Pichia pastoris* Wirtszelle produzierte gewünschte Antikörper ist ein einwertiger, zweiwertiger oder mehrwertiger Antikörper;
(xii) wenigstens eine der genannten Kopien eines eine Untereinheit des genannten gewünschten Antikörpers codierenden Gens ist für eine Expression in der genannten diploiden *Pichia pastoris* Wirtszelle optimiert;
(xiii) die Anzahl von Kopien eines die leichte Kette des genannten gewünschten Antikörpers codierenden Gens ist gleich oder größer als die genannte Anzahl von Kopien eines die schwere Kette des genannten gewünschten Antikörpers codierenden Gens;
(xiv) das Verfahren produziert eine höhere Reinheit des genannten gewünschten Antikörpers als ein Verfahren unter Verwendung eines Stamms, der eine einzige Kopie jedes eine Untereinheit des genannten gewünschten Antikörpers codierenden Gens enthält;
(xv) das Verfahren produziert eine höhere Reinheit des genannten gewünschten Antikörpers als ein Verfahren unter Verwendung eines Stamms, der eine einzige Kopie jedes eine Untereinheit des genannten gewünschten Antikörpers codierenden Gens enthält, wobei die genannte Reinheit durch Messen der Masse von glykosylierten Schwer- und/oder Leichtkettenpolypeptiden als Prozentanteil der Gesamtmenge von Schwer- und/oder Leichtkettenpolypeptiden bestimmt wird; oder
(xvi) wenigstens eine Untereinheit des genannten gewünschten Antikörpers umfasst ein Sekretionssignal, wobei optional das Sekretionssignal ein Polypeptid umfasst, ausgewählt aus der Gruppe bestehend aus: Hühner-Lysozym (CLY)-Signalpeptid; CLY-L8; S. cerevisiae invertase (SUC2) Signalpeptid; MF-Alpha (Prepro); MF-Alpha (Pre)-apv; MF-Alpha-(Pre)-apv-SLEKR; MF-Alpha-(Prepro)-(EA)3; aF-Signalpeptid; KILM1-Signalpeptid; reprimierbares Säurephosphatase-(PHO1)-Signalpeptid; A. niger GOX Signalpeptid; Schwanniomyces occidentalis Glucoamylasegen (GAM1) Signalpeptid; humanes Serum albumin (HSA) Signalpeptid mit Pro-Sequenz; humanes Serum albumin (HSA) Signalpeptid ohne Pro-Sequenz; ISN-Signalpeptid; IFN-Signalpeptid; HGH-Signalpeptid; Phytohämagglutinin (PHA); Seidenraupenlysozym; humanes Lysozym (LYZ1); Aktivinrezeptor Typ-1; Activin Typ II Rezeptor; P. pastoris Immunglobulinbindungsprotein (PpBiP); humaner Antikörper-3D6-Leichtketten-Leader; und eine beliebige Kombination davon.

## Revendications

1. Procédé pour identifier une cellule hôte *Pichia pastoris* diploïde qui produit un anticorps désiré ayant une pureté supérieure à celle d'une autre cellule hôte *Pichia pastoris* diploïde, ledit procédé consistant à :
(a) fournir un panneau de cellules hôtes *Pichia pastoris* diploïdes, ledit panneau comportant au moins deux cellules hôtes *Pichia pastoris* diploïdes qui, chacune, comprennent des nombres différents de copies d'un ou de plusieurs gènes qui déclenchent l'expression des sous-unités de l'anticorps désiré, dans lequel lesdits gènes qui déclenchent l'expression des sous-unités comprennent des gènes qui codent la chaîne légère et la chaîne lourde de l'anticorps désiré ;
(b) cultiver chacune desdites cellules hôtes *Pichia pastoris* diploïdes pour exprimer l'anticorps désiré ;
(c) mesurer la pureté de l'anticorps désiré produite par chacune desdites cellules hôtes *Pichia pastoris* diploïdes; et
(d) identifier une cellule hôte *Pichia pastoris* diploïde qui produit ledit anticorps désiré ayant une pureté supérieure à une autre cellule hôte *Pichia pastoris* diploïde dans ledit panneau de cellules hôtes *Pichia pastoris* diploïdes qui produisent l'anticorps désiré.

2. Procédé selon la revendication 1, dans lequel ledit panneau des cellules hôtes *Pichia pastoris* comporte une population hétérogène de cellules hôtes *Pichia pastoris* diploïdes, dans lequel ladite population hétérogène de cellules hôtes *Pichia pastoris* diploïdes comprend des cellules comportant diverses combinaisons de nombres de copies desdits gènes qui codent des sous-unités dudit anticorps désiré les unes par rapport aux autres et des cellules *Pichia pastoris* comportant les mêmes combinaisons de nombres de copies desdits gènes qui codent des sous-unités dudit anticorps désiré les unes par rapport aux autres.

3. Procédé selon, soit la revendication 1, soit la revendication 2, dans lequel le nombre respectif de copies du gène qui code la chaîne lourde dudit anticorps et le nombre des copies du gène qui code la chaîne légère dudit anticorps désiré dans lesdites cellules hôtes sont : 2 et 1, 3 et 1, 4 et 1, 5 et 1, 6 et 1, 7 et 1, 8 et 1, 9 et 1, 10 et 1, 1 et 2, 2 et 2, 3 et 2, 4 et 2, 5 et 2, 6 et 2, 7 et 2, 8 et 2, 9 et 2, 10 et 2, 1 et 3, 2 et 3, 3 et 3, 4 et 3, 5 et 3, 6 et 3, 7 et 3, 8 et 3, 9 et 3, 10 et 3, 1 et 4, 2 et 4, 3 et 4, 4 et 4, 5 et 4, 6 et 4, 7 et 4, 8 et 4, 9 et 4, 10 et 4, 1 et 5, 2 et 5, 3 et 5, 4 et 5, 5 et 5, 6 et 5, 7 et 5, 8 et 5, 9 et 5, 10 et 5, 1 et 6, 2 et 6, 3 et 6, 4 et 6, 5 et 6, 6 et 6, 7 et 6, 8 et 6, 9 et 6, 10 et 6, 1 et 7, 2 et 7, 3 et 7, 4 et 7, 5 et 7, 6 et 7, 7 et 7, 8 et 7, 9 et 7, 10 et 7, 1 et 8, 2 et 8, 3 et 8, 4 et 8, 5 et 8, 6 et 8, 7 et 8, 8 et 8, 9 et 8, 10 et 8, 1 et 9, 2 et 9, 3 et 9, 4 et 9, 5 et 9, 6 et 9, 7 et 9, 8 et 9, 9 et 8, 10 et 9, 1 et 10, 2 et 10, 3 et 10, 4 et 10, 5 et 10, 6 et 10, 7 et 10, 8 et 10, 9 et 10, 10 et 10.

4. Procédé selon l'une quelconque des revendications 1 à 3, dans lequel :
(i) le panneau de cellules hôtes *Pichia pastoris* diploïdes comporte des cellules qui expriment jusqu'à 15 copies de chaque gène qui code l'anticorps désiré, et qui représente largement toutes les combinaisons de nombres de copies possibles de chaque sous-unité par rapport à l'autre/aux autres sous-unité(s) ;
(ii) le panneau de cellules hôtes *Pichia pastoris* diploïdes comporte des cellules qui expriment jusqu'à 12 copies de chaque gène qui code l'anticorps désiré, et qui représente largement toutes les combinaisons de nombres de copies possibles de chaque sous-unité par rapport à l'autre/aux autres sous-unité(s) ;
(iii) le panneau de cellules hôtes *Pichia pastoris* diploïdes comporte des cellules qui expriment jusqu'à 10 copies de chaque gène qui code l'anticorps désiré, et qui représente largement toutes les combinaisons de nombres de copies possibles de chaque sous-unité par rapport à l'autre/aux autres sous-unité(s) ;
(iv) le panneau de cellules hôtes *Pichia pastoris* diploïdes comporte des cellules qui expriment jusqu'à 8 copies de chaque gène qui code l'anticorps désiré, et qui représente largement toutes les combinaisons de nombres de copies possibles de chaque sous-unité par rapport à l'autre/aux autres sous-unité(s) ;
(v) le panneau de cellules hôtes *Pichia pastoris* diploïdes comporte des cellules qui expriment jusqu'à 6 copies de chaque gène qui code l'anticorps désiré, et qui représente largement toutes les combinaisons de nombres de copies possibles de chaque sous-unité par rapport à l'autre/aux autres sous-unité(s).

5. Procédé selon l'une quelconque des revendications 1 à 4, dans lequel ladite pureté est déterminée en mesurant la proportion relative de l'une ou de plusieurs chaînes d'anticorps glycosylé et éventuellement dans lequel une ou plusieurs desdites chaînes d'anticorps glycosylé comportent un variant de chaîne lourde d'anticorps glycosylé.

6. Procédé selon l'une quelconque des revendications 1 à 5, dans lequel :
(i) ledit panneau de cellules hôtes *Pichia pastoris* diploïdes est produit par liaison ou par fusion de cellules *Pichia pastoris* haploïdes contenant différents nombres de copies de gènes qui codent une ou plusieurs sous-unités dudit anticorps désiré, en vertu duquel ladite liaison produit des cellules *Pichia pastoris* diploïdes contenant différents nombres de copies de gènes qui codent les sous-unités dudit anticorps désiré ; ou
(ii) ledit panneau de cellules hôtes *Pichia pastoris* diploïdes est produit par liaison ou par fusion de cellules *Pichia pastoris* haploïdes contenant différents nombres de copies de gènes qui codent une ou plusieurs sous-unités dudit anticorps désiré, en vertu duquel ladite liaison produit des cellules *Pichia pastoris* diploïdes contenant différents nombres de copies de gènes qui codent les sous-unités dudit anticorps désiré et dans lequel lesdits gènes qui fournissent l'expression des sous-unités de l'anticorps désiré sont intégrés au génome desdites cellules *Pichia pastoris* haploïdes.

7. Procédé selon l'une quelconque des revendications 1 à 6, dans lequel la cellule *Pichia pastoris* diploïde identifiée est utilisée pour développer une culture de production.

8. Procédé selon l'une quelconque des revendications 1 à 7, dans lequel lesdits gènes qui déclenchent l'expression des sous-unités de l'anticorps désiré sont intégrés au génome desdites cellules hôtes *Pichia pastoris* diploïdes.

9. Procédé selon la revendication 8, dans lequel les gènes qui déclenchent l'expression des sous-unités de l'anticorps désiré sont intégrés dans l'un ou plusieurs loci génomiques sélectionnés dans le groupe consistant en loci pGAP, 3'AOX TT, PpURAS5, OCH1, AOX1, HIS4, GAP, ARG et HIS4 TT.

10. Procédé selon l'une quelconque des revendications 1 à 9, dans lequel :
(i) au moins l'un desdits gènes qui codent lesdites sous-unités de l'anticorps désiré est déclenché sous le contrôle d'un promoteur inductible ou constitutif ;
(ii) au moins l'un desdits gènes qui codent lesdites sous-unités de l'anticorps désiré est déclenché sous le contrôle d'un promoteur inductible ou constitutif, dans lequel ledit promoteur inductible est sélectionné dans le groupe consistant en promoteurs GAP, CUPI, AOX1 et fLD1 ;
(iii) au moins l'un desdits gènes qui codent lesdites sous-unités de l'anticorps désiré est déclenché sous le contrôle d'un promoteur inductible ou constitutif, dans lequel au moins l'un desdits gènes qui codent lesdites sous-unités de l'anticorps désiré est déclenché sous le contrôle d'un promoteur sélectionné dans le groupe consistant en : promoteurs AOX1, ICLI, glycéraldéhyde-3-phosphate déshydrogénase (GAP), FLDI, ADHI, alcool déshydrogénase II, GAL4, PHO3, PHO5 et Pyk, promoteurs chimériques qui en sont dérivés, promoteurs de levure, promoteurs mammaliens, promoteurs pour les insectes, promoteurs pour les plantes, promoteurs pour les reptiles, promoteurs amphibiens, promoteurs viraux et promoteurs aviaires ;
(iv) l'anticorps désiré est purifié par lesdites cellules hôtes *Pichia pastoris* diploïdes;
(v) l'anticorps désiré est purifié par un composant intracellulaire, du cytoplasme, du nucléoplasme ou une membrane desdites cellules hôtes *Pichia pastoris* diploïdes;
(vi) la cellule hôte *Pichia pastoris* diploïde sécrète ledit anticorps désiré dans le milieu de culture ;
(vii) l'anticorps désiré est un anticorps monospécifique ou bispécifique ;
(viii)au moins deux cellules hôtes *Pichia pastoris* diploïdes comprennent des nombres différents de copies d'un gène qui code une sous-unité dudit anticorps désiré ;
(ix) au moins une cellule hôte *Pichia pastoris* diploïde dans ledit panneau comprend un gène qui code au moins une sous-unité de l'anticorps désiré, dont l'expression est dirigée par un promoteur autre que le promoteur qui dirige l'expression du gène correspondant dans une cellule hôte *Pichia pastoris* diploïde différente dans ledit panneau ;
(x) au moins une cellule hôte *Pichia pastoris* diploïde dans ledit panneau comprend un gène polycistronique comportant plus d'une séquence qui code une sous-unité de l'anticorps désiré ;
(xi) ledit anticorps désiré se lie spécifiquement aux IL-6, TNF-alpha, CGRP, PCSK9 ou NGF ;
(xii) ledit anticorps désiré comprend un anticorps humain ou un anticorps humanisé ou un fragment de ceux-ci ;
(xiii) ledit anticorps désiré comprend un anticorps humanisé ou un fragment de celui-ci et ledit anticorps humanisé provient à l'origine de la souris, du rat, du lapin, de la chèvre, du mouton ou de la vache, de préférence du lapin ;
(xiv) ledit anticorps désiré comprend un anticorps monovalent, bivalent ou multivalent ;
(xv) au moins l'un desdits gènes qui déclenchent l'expression d'une sous-unité dudit anticorps désiré dans au moins l'une des dites cellules hôtes *Pichia pastoris* diploïdes dans ledit panneau est optimisé pour de l'expression dans ladite cellule hôte *Pichia pastoris* diploïde; ou
(xvi) ledit anticorps désiré est glycosylé et la pureté dudit anticorps désiré est évaluée en mesurant la fraction de l'anticorps désiré que produit la cellule hôte *Pichia pastoris* diploïde qui n'est pas glycosylée, est contenu dans des complexes anticorps comportant le rayon hydrodynamique apparent attendu, et/ou se lie spécifiquement à une cible dudit anticorps désiré.

11. Procédé selon l'une quelconque des revendications 1 à 10, dans lequel :
(i) le nombre de copies de gènes qui codent l'anticorps désiré est stable pendant 20 générations ;
(ii) le nombre de copies de gènes qui codent l'anticorps désiré est stable pendant 50 générations ;
(iii) le nombre de copies de gènes qui codent l'anticorps désiré est stable pendant 100 générations ;
(iv) le nombre de copies de gènes qui codent l'anticorps désiré est stable pendant 500 générations ;
(v) le nombre de copies de gènes qui codent l'anticorps désiré est stable pendant 1000 générations ;
(vi) les copies de gènes qui codent au moins l'une des sous-unités dudit anticorps désiré sont intégrées dans deux loci ou davantage ;
(vii) les copies de gènes qui codent au moins l'une des sous-unités dudit anticorps désiré sont intégrées dans trois loci ou davantage ;
(viii) les copies de gènes qui codent au moins l'une des sous-unités dudit anticorps désiré sont intégrées dans deux, trois loci ou davantage, dans lequel chaque locus ne contient pas plus de 5 copies d'une sous-unité donnée ;
(ix) les copies de gènes qui codent au moins l'une des sous-unités dudit anticorps désiré sont intégrées dans deux, trois loci ou davantage, dans lequel chaque locus ne contient pas plus de 4 copies d'une sous-unité donnée ; ou
(x) les copies de gènes qui codent au moins l'une des sous-unités dudit anticorps désiré sont intégrées dans deux, trois loci ou davantage, dans lequel chaque locus ne contient pas plus de 3 copies d'une sous-unité donnée.

12. Procédé selon l'une quelconque des revendications 1 à 11, comprenant en outre la production par recombinaison dudit anticorps désiré par ladite cellule hôte *Pichia pastoris* diploïde identifiée comme une cellule hôte *Pichia pastoris* diploïde qui produit un anticorps désiré ayant une plus grande pureté, dans lequel la cellule hôte *Pichia pastoris* diploïde (i) comporte un ou plusieurs gènes qui codent chaque sous-unité de l'anticorps désiré ; ou (ii) comporte entre deux et dix copies d'un gène qui code chaque sous-unité dudit anticorps désiré,
dans lequel le procédé consiste à :
(a) fournir la cellule hôte *Pichia pastoris* diploïde; et
(b) cultiver ladite cellule hôte *Pichia pastoris* diploïde pour déclencher ledit anticorps désiré.

13. Procédé selon la revendication 12, dans lequel la cellule hôte *Pichia pastoris* diploïde comprend un ou plusieurs gènes qui codent chaque sous-unité de l'anticorps désiré et est identifiée par le procédé selon l'une quelconque des revendications 1 à 11 en tant que cellule hôte *Pichia pastoris* diploïde qui produit un anticorps désiré ayant une plus grande pureté, et dans lequel :
(i) le procédé comprend en outre la purification dudit anticorps désiré ; ou
(ii) ladite cellule hôte *Pichia pastoris* diploïde produit un titre d'anticorps d'au moins 100 mg/L, d'au moins 150 mg/L, d'au moins 200 mg/L, d'au moins 250 mg/L, d'au moins 300 mg/L, d'entre 100 et 300 mg/L, d'entre 100 et 500 mg/L, d'entre 100 et 1000 mg/L, d'au moins 1000 mg/L, d'au moins 1250 mg/litre, d'au moins 1500 mg/litre, 1750 mg/litre, 2000 mg/litre, ou davantage.

14. Procédé selon la revendication 12, dans lequel ladite cellule hôte *Pichia pastoris* diploïde est produite par liaison ou fusion de deux cellules parentales, dans lequel chaque cellule parentale comprend une ou plusieurs copies d'au moins un gène qui code une sous-unité dudit anticorps désiré ; éventuellement dans lequel au moins une copie de chacun des deux gènes différents qui codent une sous-unité dudit anticorps désiré est intégrée dans le même locus de deux chromosomes homologues de ladite cellule hôte *Pichia pastoris* diploïde.

15. Procédé selon la revendication 14, dans lequel :
(i) au moins l'un des gènes qui déclenchent l'expression dudit anticorps désiré est intégré dans le génome de ladite cellule hôte *Pichia pastoris* diploïde;
(ii) au moins l'un des gènes qui déclenchent l'expression dudit anticorps désiré est contenu dans un ou plusieurs éléments extrachromosomiques, plasmides, ou chromosomes artificiels ;
(iii) ladite cellule hôte *Pichia pastoris* diploïde comprend plus de copies du gène qui déclenchent l'expression de la chaîne légère dudit anticorps désiré que de copies du gène qui déclenchent l'expression de la chaîne lourde dudit anticorps désiré ;
(iv) ladite cellule hôte *Pichia pastoris* diploïde comprend plus de copies du gène qui déclenchent l'expression de la chaîne lourde dudit anticorps désiré que de copies du gène qui déclenchent l'expression de la chaîne légère dudit anticorps désiré ;
(v) ladite cellule hôte *Pichia pastoris* diploïde comprend un nombre de copies du gène qui déclenchent l'expression de la chaîne légère dudit anticorps désiré qui est égal au nombre de copies du gène qui déclenchent l'expression de la chaîne lourde dudit anticorps désiré ; ou
(vi) dans lequel le nombre respectif de copies du gène qui code la chaîne lourde dudit anticorps désiré et le nombre de copies du gène qui code la chaîne légère dudit anticorps désiré dans ladite cellule hôte *Pichia pastoris* diploïde sont : 2 et 2, 2 et 3, 3 et 3, 3 et 4, 3 et 5, 4 et 3, 4 et 4, 4 et 5, 4 et 6, 5 et 4, 5 et 5, 5 et 6, ou 5 et 7.

16. Procédé selon la revendication 12, dans lequel la cellule hôte *Pichia pastoris* diploïde comprend entre deux et dix copies d'un gène qui code chaque sous-unité dudit anticorps désiré, dans lequel ladite cellule hôte est produite par liaison ou fusion de deux cellules parentales, dans lequel chaque cellule parentale comporte une ou plusieurs copies de gènes qui codent une ou plusieurs sous-unités dudit complexe multi-sous-unités et dans lequel le procédé consistant à produire de façon recombinante un anticorps désiré inclut en outre éventuellement un ou plusieurs des éléments suivants :
(i) au moins une copie de chacun des deux différents gènes qui chacun codent une sous-unité dudit anticorps désiré est intégrée dans le même locus de deux chromosomes homologues de ladite cellule hôte *Pichia pastoris* diploïde;
(ii) au moins l'un desdits gènes qui déclenchent l'expression des sous-unités dudit anticorps désiré est intégrée dans un ou plusieurs loci génomiques sélectionnés dans le groupe composé du locus pGAP et du locus HIS4 TT ;
(iii) au moins l'un desdits gènes qui codent ledit anticorps désiré est déclenché sous le contrôle d'un promoteur inductible ou constitutif ;
(iv) au moins l'un desdits gènes qui codent ledit anticorps désiré est déclenché sous le contrôle d'un promoteur inductible ou constitutif, dans lequel ledit promoteur inductible est sélectionné dans le groupe composé de GAP, AOX1, CUP1 et FLD1 ;
(v) au moins l'un desdits gènes qui codent la chaîne légère et/ou la chaîne lourde de l'anticorps désiré est déclenché sous le contrôle d'un promoteur sélectionné dans le groupe composé des promoteurs : AOX1, ICLI, glycéraldéhyde-3-phosphate déshydrogénase (GAP), FLDI, ADHI, alcool déshydrogénase II, promoteurs GAL4, PHO3, PHO5 et Pyk, promoteurs chimériques qui en sont dérivés, promoteurs de levure, promoteurs mammaliens, promoteurs pour les insectes, promoteurs pour les plantes, promoteurs pour les reptiles, promoteurs amphibiens, promoteurs viraux et promoteurs aviaires ;
(vi) la cellule hôte *Pichia pastoris* diploïde sécrète ledit anticorps désiré dans le milieu de culture ;
(vii) l'anticorps désiré produit par la cellule hôte *Pichia pastoris* diploïde est un anticorps monospécifique ou bispécifique ;
(viii) l'anticorps désiré produit par la cellule hôte *Pichia pastoris* diploïde se lie spécifiquement aux IL-6, TNF-alpha, CGRP, PCSK9 ou NGF ;
(ix) ledit anticorps désiré produit par la cellule hôte *Pichia pastoris* diploïde est un anticorps humanisé ou un fragment de celui-ci qui spécifiquement se lie éventuellement aux IL-6, TNF-Alpha, CGRP, PCSK9 ou NGF ;
(x) l'anticorps désiré produit par la cellule hôte *Pichia pastoris* diploïde est un anticorps humanisé provenant à l'origine du lapin ;
(xi) l'anticorps désiré produit par la cellule hôte *Pichia pastoris* diploïde est un anticorps monovalent, bivalent ou multivalent ;
(xii) au moins l'une desdites copies d'un gène qui code une sous-unité dudit anticorps désiré est optimisé pour l'expression dans ladite cellule hôte *Pichia pastoris* diploïde;
(xiii) le nombre de copies d'un gène qui code la chaîne légère dudit anticorps désiré est supérieur ou égal audit nombre de copies d'un gène qui code la chaîne lourde dudit anticorps désiré ;
(xiv) le procédé produit une pureté supérieure dudit anticorps désiré qu'un procédé utilisant une souche contenant une simple copie de chaque gène qui code une sous-unité dudit anticorps désiré ;
(xv) le procédé produit une pureté supérieure dudit anticorps désiré qu'un procédé utilisant une souche contenant une simple copie de chaque gène qui code une sous-unité dudit anticorps désiré, dans lequel ladite pureté est déterminée en mesurant la masse des polypeptides de la chaîne lourde et/ou de la chaîne légère glycosylée(s) en pourcentage de la masse totale des polypeptides de la chaîne lourde et/ou de la chaîne légère ; ou
(xvi) au moins une sous-unité dudit anticorps désiré comprend un signal de sécrétion, éventuellement dans lequel le signal de sécrétion comporte un polypeptide sélectionné dans le groupe composé de : peptide signal de lysozyme de poulet (CLY) ; CLY-L8 ; peptide signal d'invertase de S.cerevisiae (SUC2) ; MF-Alpha (Prepo) ; MF-Alpha (Pre)-apv ; MF-alpha (Pre)-apv-SLEKR ; MF-alpha (Prepo)-(EA)3 ; peptide signal aF ; peptide signal KILM1 ; peptide signal de phosphatase acide répressible (PHO1) ; peptide signal GOX A.niger ; peptide signal de gène de glucoamylase Schwanniomyces occidentalis (GAM1) ; peptide signal de sérum albumine humain (HSA) avec proséquence ; peptide signal de sérum albumine humain (HSA) sans proséquence ; peptide signal ISN ; peptide signal IFN ; Peptide signal HGH ; phytohémagglutinine (PHA) ; lysozyme de ver à soie ; lysozyme humain (LYZI) ; récepteur d'activine de type-1 ; récepteur d'activine de type II ; protéine de liaison à une immunoglobuline pastoris (PpBiP) ; leader de chaîne légère 3D6 d'anticorps humain ; et toute combinaison de ceux-ci.


### SEQUENCE LISTING

<110> ALDERBIO HOLDINGS LLC
   Mitchell, Danielle M
   Garcia-Martinez, Leon F
   Ojala, Ethan W
   Inan, Mehmet
   McNeill, Patricia D
   Latham, John
<120> MULTI-COPY STRATEGY FOR HIGH-TITER AND HIGH-PURITY PRODUCTION OF MULTI-SUBUNIT PROTEINS SUCH AS ANTIBODIES IN TRANSFORMED MICROBES SUCH AS PICHIA PASTORIS
<130> 67858.730202
<140> tba
   <141> 2012-08-20
<150> 61/525,307
   <151> 2011-08-19
<150> 13/466,795
   <151> 2012-05-08
<160> 54
<170> PatentIn version 3.5
<210> 1
   <211> 441
   <212> PRT
   <213> Artificial
<220>
   <223> Humanized antibody sequence
<400> 1
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Leu Asp Leu Ser Ser Tyr
               20                  25                  30
   Tyr Met Gln Trp Val Arg Gln Ala Pro Gly Lys Gly Leu Glu Trp Val
           35                  40                  45
   Gly Val Ile Gly Ile Asn Asp Asn Thr Tyr Tyr Ala Ser Trp Ala Lys
       50                  55                  60
   Gly Arg Phe Thr Ile Ser Arg Asp Asn Ser Lys Thr Thr Val Tyr Leu
   65                  70                  75                  80
   Gln Met Asn Ser Leu Arg Ala Glu Asp Thr Ala Val Tyr Phe Cys Ala
                   85                  90                  95
   Arg Gly Asp Ile Trp Gly Gln Gly Thr Leu Val Thr Val Ser Ser Ala
   100 105 110
   Ser Thr Lys Gly Pro Ser Val Phe Pro Leu Ala Pro Ser Ser Lys Ser
   115 120 125
   Thr Ser Gly Gly Thr Ala Ala Leu Gly Cys Leu Val Lys Asp Tyr Phe
   130 135 140
   Pro Glu Pro Val Thr Val Ser Trp Asn Ser Gly Ala Leu Thr Ser Gly
   145 150 155 160
   Val His Thr Phe Pro Ala Val Leu Gln Ser Ser Gly Leu Tyr Ser Leu
   165 170 175
   Ser Ser Val Val Thr Val Pro Ser Ser Ser Leu Gly Thr Gln Thr Tyr
   180 185 190
   Ile Cys Asn Val Asn His Lys Pro Ser Asn Thr Lys Val Asp Ala Arg
   195 200 205
   Val Glu Pro Lys Ser Cys Asp Lys Thr His Thr Cys Pro Pro Cys Pro
   210 215 220
   Ala Pro Glu Leu Leu Gly Gly Pro Ser Val Phe Leu Phe Pro Pro Lys
   225 230 235 240
   Pro Lys Asp Thr Leu Met Ile Ser Arg Thr Pro Glu Val Thr Cys Val
   245 250 255
   Val Val Asp Val Ser His Glu Asp Pro Glu Val Lys Phe Asn Trp Tyr
   260 265 270
   Val Asp Gly Val Glu Val His Asn Ala Lys Thr Lys Pro Arg Glu Glu
   275 280 285
   Gln Tyr Ala Ser Thr Tyr Arg Val Val Ser Val Leu Thr Val Leu His
   290 295 300
   Gln Asp Trp Leu Asn Gly Lys Glu Tyr Lys Cys Lys Val Ser Asn Lys
   305 310 315 320
   Ala Leu Pro Ala Pro Ile Glu Lys Thr Ile Ser Lys Ala Lys Gly Gln
   325 330 335
   Pro Arg Glu Pro Gln Val Tyr Thr Leu Pro Pro Ser Arg Glu Glu Met
   340 345 350
   Thr Lys Asn Gln Val Ser Leu Thr Cys Leu Val Lys Gly Phe Tyr Pro
           355                 360                 365
   Ser Asp Ile Ala Val Glu Trp Glu Ser Asn Gly Gln Pro Glu Asn Asn
       370                 375                 380
   Tyr Lys Thr Thr Pro Pro Val Leu Asp Ser Asp Gly Ser Phe Phe Leu
   385                 390                 395                 400
   Tyr Ser Lys Leu Thr Val Asp Lys Ser Arg Trp Gln Gln Gly Asn Val
                   405                 410                 415
   Phe Ser Cys Ser Val Met His Glu Ala Leu His Asn His Tyr Thr Gln
               420                 425                 430
   Lys Ser Leu Ser Leu Ser Pro Gly Lys
           435                 440
<210> 2
   <211> 111
   <212> PRT
   <213> Artificial
<220>
   <223> Humanized antibody sequence
<400> 2
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Leu Asp Leu Ser Ser Tyr
               20                  25                  30
   Tyr Met Gln Trp Val Arg Gln Ala Pro Gly Lys Gly Leu Glu Trp Val
           35                  40                  45
   Gly Val Ile Gly Ile Asn Asp Asn Thr Tyr Tyr Ala Ser Trp Ala Lys
       50                  55                  60
   Gly Arg Phe Thr Ile Ser Arg Asp Asn Ser Lys Thr Thr Val Tyr Leu
   65                  70                  75                  80
   Gln Met Asn Ser Leu Arg Ala Glu Asp Thr Ala Val Tyr Phe Cys Ala
                   85                  90                  95
   Arg Gly Asp Ile Trp Gly Gln Gly Thr Leu Val Thr Val Ser Ser
               100                 105                 110
<210> 3
   <211> 5
   <212> PRT
   <213> Artificial
<220>
   <223> Humanized antibody sequence
<400> 3
   Ser Tyr Tyr Met Gln
   1               5
<210> 4
   <211> 16
   <212> PRT
   <213> Artificial
<220>
   <223> Humanized antibody sequence
<400> 4
   Val Ile Gly Ile Asn Asp Asn Thr Tyr Tyr Ala Ser Trp Ala Lys Gly
   1               5                   10                  15
<210> 5
   <211> 333
   <212> DNA
   <213> Artificial
<220>
   <223> Humanized antibody sequence
<400> 5
   gaggtgcagc ttgtggagtc tgggggaggc ttggtccagc ctggggggtc cctgagactc     60
   tcctgtgcag tctctggact cgacctcagt agctactaca tgcaatgggt ccgtcaggct    120
   ccagggaagg ggctggagtg ggtcggagtc attggtatca atgataacac atactacgcg    180
   agctgggcga aaggccgatt caccatctcc agagacaatt ccaagaccac ggtgtatctt    240
   caaatgaaca gcctgagagc tgaggacact gctgtgtatt tctgtgctag aggggacatc    300
   tggggccaag ggaccctcgt caccgtctcg agc                                 333
<210> 6
   <211> 1326
   <212> DNA
   <213> Artificial
<220>
   <223> Humanized antibody sequence
<400> 6
   gaggtgcagc ttgtggagtc tgggggaggc ttggtccagc ctggggggtc cctgagactc 60
   tcctgtgcag tctctggact cgacctcagt agctactaca tgcaatgggt ccgtcaggct 120
   ccagggaagg ggctggagtg ggtcggagtc attggtatca atgataacac atactacgcg 180
   agctgggcga aaggccgatt caccatctcc agagacaatt ccaagaccac ggtgtatctt 240
   caaatgaaca gcctgagagc tgaggacact gctgtgtatt tctgtgctag aggggacatc 300
   tggggccaag ggaccctcgt caccgtctcg agcgcctcca ccaagggccc atcggtcttc 360
   cccctggcac cctcctccaa gagcacctct gggggcacag cggccctggg ctgcctggtc 420
   aaggactact tccccgaacc ggtgacggtg tcgtggaact caggcgccct gaccagcggc 480
   gtgcacacct tcccggctgt cctacagtcc tcaggactct actccctcag cagcgtggtg 540
   accgtgccct ccagcagctt gggcacccag acctacatct gcaacgtgaa tcacaagccc 600
   agcaacacca aggtggacgc gagagttgag cccaaatctt gtgacaaaac tcacacatgc 660
   ccaccgtgcc cagcacctga actcctgggg ggaccgtcag tcttcctctt ccccccaaaa 720
   cccaaggaca ccctcatgat ctcccggacc cctgaggtca catgcgtggt ggtggacgtg 780
   agccacgaag accctgaggt caagttcaac tggtacgtgg acggcgtgga ggtgcataat 840
   gccaagacaa agccgcggga ggagcagtac gccagcacgt accgtgtggt cagcgtcctc 900
   accgtcctgc accaggactg gctgaatggc aaggagtaca agtgcaaggt ctccaacaaa 960
   gccctcccag cccccatcga gaaaaccatc tccaaagcca aagggcagcc ccgagaacca 1020
   caggtgtaca ccctgccccc atcccgggag gagatgacca agaaccaggt cagcctgacc 1080
   tgcctggtca aaggcttcta tcccagcgac atcgccgtgg agtgggagag caatgggcag 1140
   ccggagaaca actacaagac cacgcctccc gtgctggact ccgacggctc cttcttcctc 1200
   tacagcaagc tcaccgtgga caagagcagg tggcagcagg ggaacgtctt ctcatgctcc 1260
   gtgatgcatg aggctctgca caaccactac acgcagaaga gcctctccct gtctccgggt 1320
   aaatga 1326
<210> 7
   <211> 219
   <212> PRT
   <213> Artificial
<220>
   <223> Humanized antibody sequence
<400> 7
   Gln Val Leu Thr Gln Ser Pro Ser Ser Leu Ser Ala Ser Val Gly Asp
   1               5                   10                  15
   Arg Val Thr Ile Asn Cys Gln Ala Ser Gln Ser Val Tyr Asp Asn Asn
               20                  25                  30
   Tyr Leu Ala Trp Tyr Gln Gln Lys Pro Gly Lys Val Pro Lys Gln Leu
           35                  40                  45
   Ile Tyr Ser Thr Ser Thr Leu Ala Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Val Ala Thr Tyr Tyr Cys Leu Gly Ser Tyr Asp Cys Ser
                   85                  90                  95
   Ser Gly Asp Cys Phe Val Phe Gly Gly Gly Thr Lys Val Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 8
   <211> 113
   <212> PRT
   <213> Artificial
<220>
   <223> Humanized antibody sequence
<400> 8
   Gln Val Leu Thr Gln Ser Pro Ser Ser Leu Ser Ala Ser Val Gly Asp
   1               5                   10                  15
   Arg Val Thr Ile Asn Cys Gln Ala Ser Gln Ser Val Tyr Asp Asn Asn
               20                  25                  30
   Tyr Leu Ala Trp Tyr Gln Gln Lys Pro Gly Lys Val Pro Lys Gln Leu
           35                  40                  45
   Ile Tyr Ser Thr Ser Thr Leu Ala Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Val Ala Thr Tyr Tyr Cys Leu Gly Ser Tyr Asp Cys Ser
                   85                  90                  95
   Ser Gly Asp Cys Phe Val Phe Gly Gly Gly Thr Lys Val Glu Ile Lys
               100                 105                 110
   Arg
<210> 9
   <211> 13
   <212> PRT
   <213> Artificial
<220>
   <223> Humanized antibody sequence
<400> 9
   Gln Ala Ser Gln Ser Val Tyr Asp Asn Asn Tyr Leu Ala
   1               5                   10
<210> 10
   <211> 7
   <212> PRT
   <213> Artificial
<220>
   <223> Humanized antibody sequence
<400> 10
   Ser Thr Ser Thr Leu Ala Ser
   1               5
<210> 11
   <211> 13
   <212> PRT
   <213> Artificial
<220>
   <223> Humanized antibody sequence
<400> 11
   Leu Gly Ser Tyr Asp Cys Ser Ser Gly Asp Cys Phe Val
   1               5                   10
<210> 12
   <211> 339
   <212> DNA
   <213> Artificial
<220>
   <223> Humanized antibody sequence
<400> 12
   caagtgctga cccagtctcc atcctccctg tctgcatctg taggagacag agtcaccatc      60
   aattgccagg ccagtcagag tgtttatgat aacaactacc tagcctggta tcagcagaaa     120
   ccagggaaag ttcctaagca actgatctat tctacatcca ctctggcatc tggggtccca     180
   tctcgtttca gtggcagtgg atctgggaca gatttcactc tcaccatcag cagcctgcag     240
   cctgaagatg ttgcaactta ttactgtcta ggcagttatg attgtagtag tggtgattgt     300
   tttgttttcg gcggaggaac caaggtggaa atcaaacgt                            339
<210> 13
   <211> 660
   <212> DNA
   <213> Artificial
<220>
   <223> Humanized antibody sequence
<400> 13
   caagtgctga cccagtctcc atcctccctg tctgcatctg taggagacag agtcaccatc      60
   aattgccagg ccagtcagag tgtttatgat aacaactacc tagcctggta tcagcagaaa     120
   ccagggaaag ttcctaagca actgatctat tctacatcca ctctggcatc tggggtccca     180
   tctcgtttca gtggcagtgg atctgggaca gatttcactc tcaccatcag cagcctgcag     240
   cctgaagatg ttgcaactta ttactgtcta ggcagttatg attgtagtag tggtgattgt     300
   tttgttttcg gcggaggaac caaggtggaa atcaaacgta cggtggctgc accatctgtc     360
   ttcatcttcc cgccatctga tgagcagttg aaatctggaa ctgcctctgt tgtgtgcctg     420
   ctgaataact tctatcccag agaggccaaa gtacagtgga aggtggataa cgccctccaa     480
   tcgggtaact cccaggagag tgtcacagag caggacagca aggacagcac ctacagcctc     540
   agcagcaccc tgacgctgag caaagcagac tacgagaaac acaaagtcta cgcctgcgaa     600
   gtcacccatc agggcctgag ctcgcccgtc acaaagagct tcaacagggg agagtgttag     660
<210> 14
   <211> 441
   <212> PRT
   <213> Artificial
<220>
   <223> Humanized antibody sequence
<400> 14
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Ile Asp Leu Ser Gly Tyr
               20                  25                  30
   Tyr Met Asn Trp Val Arg Gln Ala Pro Gly Lys Gly Leu Glu Trp Val
           35                  40                  45
   Gly Val Ile Gly Ile Asn Gly Ala Thr Tyr Tyr Ala Ser Trp Ala Lys
       50                  55                  60
   Gly Arg Phe Thr Ile Ser Arg Asp Asn Ser Lys Thr Thr Val Tyr Leu
   65                  70                  75                  80
   Gln Met Asn Ser Leu Arg Ala Glu Asp Thr Ala Val Tyr Phe Cys Ala
                   85                  90                  95
   Arg Gly Asp Ile Trp Gly Gln Gly Thr Leu Val Thr Val Ser Ser Ala
               100                 105                 110
   Ser Thr Lys Gly Pro Ser Val Phe Pro Leu Ala Pro Ser Ser Lys Ser
           115                 120                 125
   Thr Ser Gly Gly Thr Ala Ala Leu Gly Cys Leu Val Lys Asp Tyr Phe
       130                 135                 140
   Pro Glu Pro Val Thr Val Ser Trp Asn Ser Gly Ala Leu Thr Ser Gly
   145                 150                 155                 160
   Val His Thr Phe Pro Ala Val Leu Gln Ser Ser Gly Leu Tyr Ser Leu
                   165                 170                 175
   Ser Ser Val Val Thr Val Pro Ser Ser Ser Leu Gly Thr Gln Thr Tyr
               180                 185                 190
   Ile Cys Asn Val Asn His Lys Pro Ser Asn Thr Lys Val Asp Ala Arg
           195                 200                 205
   Val Glu Pro Lys Ser Cys Asp Lys Thr His Thr Cys Pro Pro Cys Pro
       210                 215                 220
   Ala Pro Glu Leu Leu Gly Gly Pro Ser Val Phe Leu Phe Pro Pro Lys
   225                 230                 235                 240
   Pro Lys Asp Thr Leu Met Ile Ser Arg Thr Pro Glu Val Thr Cys Val
   245 250 255
   Val Val Asp Val Ser His Glu Asp Pro Glu Val Lys Phe Asn Trp Tyr
   260 265 270
   Val Asp Gly Val Glu Val His Asn Ala Lys Thr Lys Pro Arg Glu Glu
   275 280 285
   Gln Tyr Ala Ser Thr Tyr Arg Val Val Ser Val Leu Thr Val Leu His
   290 295 300
   Gln Asp Trp Leu Asn Gly Lys Glu Tyr Lys Cys Lys Val Ser Asn Lys
   305 310 315 320
   Ala Leu Pro Ala Pro Ile Glu Lys Thr Ile Ser Lys Ala Lys Gly Gln
   325 330 335
   Pro Arg Glu Pro Gln Val Tyr Thr Leu Pro Pro Ser Arg Glu Glu Met
   340 345 350
   Thr Lys Asn Gln Val Ser Leu Thr Cys Leu Val Lys Gly Phe Tyr Pro
   355 360 365
   Ser Asp Ile Ala Val Glu Trp Glu Ser Asn Gly Gln Pro Glu Asn Asn
   370 375 380
   Tyr Lys Thr Thr Pro Pro Val Leu Asp Ser Asp Gly Ser Phe Phe Leu
   385 390 395 400
   Tyr Ser Lys Leu Thr Val Asp Lys Ser Arg Trp Gln Gln Gly Asn Val
   405 410 415
   Phe Ser Cys Ser Val Met His Glu Ala Leu His Asn His Tyr Thr Gln
   420 425 430
   Lys Ser Leu Ser Leu Ser Pro Gly Lys
   435 440
<210> 15
   <211> 111
   <212> PRT
   <213> Artificial
<220>
   <223> Humanized antibody sequence
<400> 15
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Ile Asp Leu Ser Gly Tyr
               20                  25                  30
   Tyr Met Asn Trp Val Arg Gln Ala Pro Gly Lys Gly Leu Glu Trp Val
           35                  40                  45
   Gly Val Ile Gly Ile Asn Gly Ala Thr Tyr Tyr Ala Ser Trp Ala Lys
       50                  55                  60
   Gly Arg Phe Thr Ile Ser Arg Asp Asn Ser Lys Thr Thr Val Tyr Leu
   65                  70                  75                  80
   Gln Met Asn Ser Leu Arg Ala Glu Asp Thr Ala Val Tyr Phe Cys Ala
                   85                  90                  95
   Arg Gly Asp Ile Trp Gly Gln Gly Thr Leu Val Thr Val Ser Ser
               100                 105                 110
<210> 16
   <211> 5
   <212> PRT
   <213> Artificial
<220>
   <223> Humanized antibody sequence
<400> 16
   Gly Tyr Tyr Met Asn
   1               5
<210> 17
   <211> 15
   <212> PRT
   <213> Artificial
<220>
   <223> Humanized antibody sequence
<400> 17
   Ile Gly Ile Asn Gly Ala Thr Tyr Tyr Ala Ser Trp Ala Lys Gly
   1               5                   10                  15
<210> 18
   <211> 333
   <212> DNA
   <213> Artificial
<220>
   <223> Humanized antibody sequence
<400> 18
   gaggtgcagc ttgtggagtc tgggggaggc ttggtccagc ctggggggtc cctgagactc       60
   tcctgtgcag tctctggaat cgacctcagt ggctactaca tgaactgggt ccgtcaggct      120
   ccagggaagg ggctggagtg ggtcggagtc attggtatta atggtgccac atactacgcg      180
   agctgggcga aaggccgatt caccatctcc agagacaatt ccaagaccac ggtgtatctt      240
   caaatgaaca gcctgagagc tgaggacact gctgtgtatt tctgtgctag aggggacatc      300
   tggggccaag ggaccctcgt caccgtctcg agc                                   333
<210> 19
   <211> 1326
   <212> DNA
   <213> Artificial
<220>
   <223> Humanized antibody sequence
<400> 19
   gaggtgcagc ttgtggagtc tgggggaggc ttggtccagc ctggggggtc cctgagactc      60
   tcctgtgcag tctctggaat cgacctcagt ggctactaca tgaactgggt ccgtcaggct     120
   ccagggaagg ggctggagtg ggtcggagtc attggtatta atggtgccac atactacgcg     180
   agctgggcga aaggccgatt caccatctcc agagacaatt ccaagaccac ggtgtatctt     240
   caaatgaaca gcctgagagc tgaggacact gctgtgtatt tctgtgctag aggggacatc     300
   tggggccaag ggaccctcgt caccgtctcg agcgcctcca ccaagggccc atcggtcttc     360
   cccctggcac cctcctccaa gagcacctct gggggcacag cggccctggg ctgcctggtc     420
   aaggactact tccccgaacc ggtgacggtg tcgtggaact caggcgccct gaccagcggc     480
   gtgcacacct tcccggctgt cctacagtcc tcaggactct actccctcag cagcgtggtg     540
   accgtgccct ccagcagctt gggcacccag acctacatct gcaacgtgaa tcacaagccc     600
   agcaacacca aggtggacgc gagagttgag cccaaatctt gtgacaaaac tcacacatgc     660
   ccaccgtgcc cagcacctga actcctgggg ggaccgtcag tcttcctctt ccccccaaaa     720
   cccaaggaca ccctcatgat ctcccggacc cctgaggtca catgcgtggt ggtggacgtg     780
   agccacgaag accctgaggt caagttcaac tggtacgtgg acggcgtgga ggtgcataat     840
   gccaagacaa agccgcggga ggagcagtac gccagcacgt accgtgtggt cagcgtcctc     900
   accgtcctgc accaggactg gctgaatggc aaggagtaca agtgcaaggt ctccaacaaa     960
   gccctcccag cccccatcga gaaaaccatc tccaaagcca aagggcagcc ccgagaacca    1020
   caggtgtaca ccctgccccc atcccgggag gagatgacca agaaccaggt cagcctgacc    1080
   tgcctggtca aaggcttcta tcccagcgac atcgccgtgg agtgggagag caatgggcag    1140
   ccggagaaca actacaagac cacgcctccc gtgctggact ccgacggctc cttcttcctc    1200
   tacagcaagc tcaccgtgga caagagcagg tggcagcagg ggaacgtctt ctcatgctcc    1260
   gtgatgcatg aggctctgca caaccactac acgcagaaga gcctctccct gtctccgggt    1320
   aaatga                                                                1326
<210> 20
   <211> 219
   <212> PRT
   <213> Artificial
<220>
   <223> Humanized antibody sequence
<400> 20
   Gln Val Leu Thr Gln Ser Pro Ser Ser Leu Ser Ala Ser Val Gly Asp
   1               5                   10                  15
   Arg Val Thr Ile Asn Cys Gln Ala Ser Gln Ser Val Tyr His Asn Thr
               20                  25                  30
   Tyr Leu Ala Trp Tyr Gln Gln Lys Pro Gly Lys Val Pro Lys Gln Leu
           35                  40                  45
   Ile Tyr Asp Ala Ser Thr Leu Ala Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Val Ala Thr Tyr Tyr Cys Leu Gly Ser Tyr Asp Cys Thr
                   85                  90                  95
   Asn Gly Asp Cys Phe Val Phe Gly Gly Gly Thr Lys Val Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 21
   <211> 113
   <212> PRT
   <213> Artificial
<220>
   <223> Humanized antibody sequence
<400> 21
   Gln Val Leu Thr Gln Ser Pro Ser Ser Leu Ser Ala Ser Val Gly Asp
   1               5                   10                  15
   Arg Val Thr Ile Asn Cys Gln Ala Ser Gln Ser Val Tyr His Asn Thr
               20                  25                  30
   Tyr Leu Ala Trp Tyr Gln Gln Lys Pro Gly Lys Val Pro Lys Gln Leu
           35                  40                  45
   Ile Tyr Asp Ala Ser Thr Leu Ala Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Val Ala Thr Tyr Tyr Cys Leu Gly Ser Tyr Asp Cys Thr
                   85                  90                  95
   Asn Gly Asp Cys Phe Val Phe Gly Gly Gly Thr Lys Val Glu Ile Lys
               100                 105                 110
   Arg
<210> 22
   <211> 13
   <212> PRT
   <213> Artificial
<220>
   <223> Humanized antibody sequence
<400> 22
   Gln Ala Ser Gln Ser Val Tyr His Asn Thr Tyr Leu Ala
   1               5                   10
<210> 23
   <211> 7
   <212> PRT
   <213> Artificial
<220>
   <223> Humanized antibody sequence
<400> 23
   Asp Ala Ser Thr Leu Ala Ser
   1               5
<210> 24
   <211> 13
   <212> PRT
   <213> Artificial
<220>
   <223> Humanized antibody sequence
<400> 24
   Leu Gly Ser Tyr Asp Cys Thr Asn Gly Asp Cys Phe Val
   1               5                   10
<210> 25
   <211> 339
   <212> DNA
   <213> Artificial
<220>
   <223> Humanized antibody sequence
<400> 25
   caagtgctga cccagtctcc atcctccctg tctgcatctg taggagacag agtcaccatc      60
   aattgccagg ccagtcagag tgtttatcat aacacctacc tggcctggta tcagcagaaa     120
   ccagggaaag ttcctaagca actgatctat gatgcatcca ctctggcatc tggggtccca     180
   tctcgtttca gtggcagtgg atctgggaca gatttcactc tcaccatcag cagcctgcag     240
   cctgaagatg ttgcaactta ttactgtctg ggcagttatg attgtactaa tggtgattgt     300
   tttgttttcg gcggaggaac caaggtggaa atcaaacgt                            339
<210> 26
   <211> 660
   <212> DNA
   <213> Artificial
<220>
   <223> Humanized antibody sequence
<400> 26
   caagtgctga cccagtctcc atcctccctg tctgcatctg taggagacag agtcaccatc      60
   aattgccagg ccagtcagag tgtttatcat aacacctacc tggcctggta tcagcagaaa     120
   ccagggaaag ttcctaagca actgatctat gatgcatcca ctctggcatc tggggtccca     180
   tctcgtttca gtggcagtgg atctgggaca gatttcactc tcaccatcag cagcctgcag     240
   cctgaagatg ttgcaactta ttactgtctg ggcagttatg attgtactaa tggtgattgt     300
   tttgttttcg gcggaggaac caaggtggaa atcaaacgta cggtggctgc accatctgtc     360
   ttcatcttcc cgccatctga tgagcagttg aaatctggaa ctgcctctgt tgtgtgcctg     420
   ctgaataact tctatcccag agaggccaaa gtacagtgga aggtggataa cgccctccaa     480
   tcgggtaact cccaggagag tgtcacagag caggacagca aggacagcac ctacagcctc     540
   agcagcaccc tgacgctgag caaagcagac tacgagaaac acaaagtcta cgcctgcgaa     600
   gtcacccatc agggcctgag ctcgcccgtc acaaagagct tcaacagggg agagtgttag     660
<210> 27
   <211> 1350
   <212> DNA
   <213> Artificial
<220>
   <223> Humanized antibody sequence
<400> 27
   gaggtgcagc tggtggagtc tgggggaggc ttggtccagc ctggggggtc cctgagactc 60
   tcctgtgcag cctctggatt ctccctcagt aactactacg tgacctgggt ccgtcaggct 120
   ccagggaagg ggctggagtg ggtcggcatc atctatggta gtgatgaaac cgcctacgct 180
   acctccgcta taggccgatt caccatctcc agagacaatt ccaagaacac cctgtatctt 240
   caaatgaaca gcctgagagc tgaggacact gctgtgtatt actgtgctag agatgatagt 300
   agtgactggg atgcaaagtt caacttgtgg ggccaaggga ccctcgtcac cgtctcgagc 360
   gcctccacca agggcccatc ggtcttcccc ctggcaccct cctccaagag cacctctggg 420
   ggcacagcgg ccctgggctg cctggtcaag gactacttcc ccgaaccggt gacggtgtcg 480
   tggaactcag gcgccctgac cagcggcgtg cacaccttcc cggctgtcct acagtcctca 540
   ggactctact ccctcagcag cgtggtgacc gtgccctcca gcagcttggg cacccagacc 600
   tacatctgca acgtgaatca caagcccagc aacaccaagg tggacaagag agttgagccc 660
   aaatcttgtg acaaaactca cacatgccca ccgtgcccag cacctgaact cctgggggga 720
   ccgtcagtct tcctcttccc cccaaaaccc aaggacaccc tcatgatctc ccggacccct 780
   gaggtcacat gcgtggtggt ggacgtgagc cacgaagacc ctgaggtcaa gttcaactgg 840
   tacgtggacg gcgtggaggt gcataatgcc aagacaaagc cgcgggagga gcagtacgcc 900
   agcacgtacc gtgtggtcag cgtcctcacc gtcctgcacc aggactggct gaatggcaag 960
   gagtacaagt gcaaggtctc caacaaagcc ctcccagccc ccatcgagaa aaccatctcc 1020
   aaagccaaag ggcagccccg agaaccacag gtgtacaccc tgcccccatc ccgggaggag 1080
   atgaccaaga accaggtcag cctgacctgc ctggtcaaag gcttctatcc cagcgacatc 1140
   gccgtggagt gggagagcaa tgggcagccg gagaacaact acaagaccac gcctcccgtg 1200
   ctggactccg acggctcctt cttcctctac agcaagctca ccgtggacaa gagcaggtgg 1260
   cagcagggga acgtcttctc atgctccgtg atgcatgagg ctctgcacaa ccactacacg 1320
   cagaagagcc tctccctgtc tccgggtaaa 1350
<210> 28
   <211> 450
   <212> PRT
   <213> Artificial
<220>
   <223> Humanized antibody sequence
<400> 28
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Ser Asn Tyr
               20                  25                  30
   Tyr Val Thr Trp Val Arg Gln Ala Pro Gly Lys Gly Leu Glu Trp Val
           35                  40                  45
   Gly Ile Ile Tyr Gly Ser Asp Glu Thr Ala Tyr Ala Thr Ser Ala Ile
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Asp Asp Ser Ser Asp Trp Asp Ala Lys Phe Asn Leu Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Arg Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Ala Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Glu Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
       450
<210> 29
   <211> 651
   <212> DNA
   <213> Artificial
<220>
   <223> Humanized antibody sequence
<400> 29
   gctatccaga tgacccagtc tccttcctcc ctgtctgcat ctgtaggaga cagagtcacc      60
   atcacttgcc aggccagtca gagcattaac aatgagttat cctggtatca gcagaaacca     120
   gggaaagccc ctaagctcct gatctatagg gcatccactc tggcatctgg ggtcccatca     180
   aggttcagcg gcagtggatc tgggacagac ttcactctca ccatcagcag cctgcagcct     240
   gatgattttg caacttatta ctgccaacag ggttatagtc tgaggaacat tgataatgct     300
   ttcggcggag ggaccaaggt ggaaatcaaa cgtacggtgg ctgcaccatc tgtcttcatc     360
   ttcccgccat ctgatgagca gttgaaatct ggaactgcct ctgttgtgtg cctgctgaat     420
   aacttctatc ccagagaggc caaagtacag tggaaggtgg ataacgccct ccaatcgggt     480
   aactcccagg agagtgtcac agagcaggac agcaaggaca gcacctacag cctcagcagc     540
   accctgacgc tgagcaaagc agactacgag aaacacaaag tctacgcctg cgaagtcacc     600
   catcagggcc tgagctcgcc cgtcacaaag agcttcaaca ggggagagtg t              651
<210> 30
   <211> 217
   <212> PRT
   <213> Artificial
<220>
   <223> Humanized antibody sequence
<400> 30
   Ala Ile Gln Met Thr Gln Ser Pro Ser Ser Leu Ser Ala Ser Val Gly
   1               5                   10                  15
   Asp Arg Val Thr Ile Thr Cys Gln Ala Ser Gln Ser Ile Asn Asn Glu
               20                  25                  30
   Leu Ser Trp Tyr Gln Gln Lys Pro Gly Lys Ala Pro Lys Leu Leu Ile
           35                  40                  45
   Tyr Arg Ala Ser Thr Leu Ala Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Asp Asp Phe Ala Thr Tyr Tyr Cys Gln Gln Gly Tyr Ser Leu Arg Asn
                   85                  90                  95
   Ile Asp Asn Ala Phe Gly Gly Gly Thr Lys Val Glu Ile Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Val Tyr Ala Cys Glu Val Thr His Gln Gly Leu Ser Ser Pro Val
           195                 200                 205
   Thr Lys Ser Phe Asn Arg Gly Glu Cys
       210                 215
<210> 31
   <211> 18
   <212> PRT
   <213> Artificial
<220>
   <223> Secretion signal peptide
<400> 31
   Met Arg Ser Leu Leu Ile Leu Val Leu Cys Phe Leu Pro Leu Ala Ala
   1               5                   10                  15
   Leu Gly
<210> 32
   <211> 16
   <212> PRT
   <213> Artificial
<220>
   <223> Secretion signal peptide
<400> 32
   Met Arg Leu Leu Leu Leu Leu Leu Leu Leu Pro Leu Ala Ala Leu Gly
   1               5                   10                  15
<210> 33
   <211> 19
   <212> PRT
   <213> Artificial
<220>
   <223> Secretion signal peptide
<400> 33
   Met Leu Leu Gln Ala Phe Leu Phe Leu Leu Ala Gly Phe Ala Ala Lys
   1               5                   10                  15
   Ile Ser Ala
<210> 34
   <211> 35
   <212> PRT
   <213> Artificial
<220>
   <223> Secretion signal peptide
<400> 34
   Met Arg Phe Pro Ser Ile Phe Thr Ala Val Leu Phe Ala Ala Ser Ser
   1               5                   10                  15
   Ala Leu Ala Ala Pro Val Asn Thr Thr Thr Glu Glu Gly Val Ser Leu
               20                  25                  30
   Glu Lys Arg
           35
<210> 35
   <211> 22
   <212> PRT
   <213> Artificial
<220>
   <223> Secretion signal peptide
<400> 35
   Met Arg Phe Pro Ser Ile Phe Thr Ala Val Leu Phe Ala Ala Ser Ser
   1               5                   10                  15
   Ala Leu Ala Ala Pro Val
               20
<210> 36
   <211> 27
   <212> PRT
   <213> Artificial
<220>
   <223> Secretion signal peptide
<400> 36
   Met Arg Phe Pro Ser Ile Phe Thr Ala Val Leu Phe Ala Ala Ser Ser
   1               5                   10                  15
   Ala Leu Ala Ala Pro Val Ser Leu Glu Lys Arg
               20                  25
<210> 37
   <211> 41
   <212> PRT
   <213> Artificial
<220>
   <223> Secretion signal peptide
<400> 37
   Met Arg Phe Pro Ser Ile Phe Thr Ala Val Leu Phe Ala Ala Ser Ser
   1 5 10 15
   Ala Leu Ala Ala Pro Val Asn Thr Thr Thr Glu Glu Gly Val Ser Leu
   20 25 30
   Glu Lys Arg Glu Ala Glu Ala Glu Ala
   35 40
<210> 38
   <211> 85
   <212> PRT
   <213> Artificial
<220>
   <223> Secretion signal peptide
<400> 38
   Met Arg Phe Pro Ser Ile Phe Thr Ala Val Leu Phe Ala Ala Ser Ser
   1 5 10 15
   Ala Leu Ala Ala Pro Val Asn Thr Thr Thr Glu Asp Glu Thr Ala Gln
   20 25 30
   Ile Pro Ala Glu Ala Val Ile Gly Tyr Ser Asp Leu Glu Gly Asp Phe
   35 40 45
   Asp Val Ala Val Leu Pro Phe Ser Asn Ser Thr Asn Asn Gly Leu Leu
   50 55 60
   Phe Ile Asn Thr Thr Ile Ala Ser Ile Ala Ala Lys Glu Glu Gly Val
   65 70 75 80
   Ser Leu Glu Lys Arg
   85
<210> 39
   <211> 44
   <212> PRT
   <213> Artificial
<220>
   <223> Secretion signal peptide
<400> 39
   Met Thr Lys Pro Thr Gln Val Leu Val Arg Ser Val Ser Ile Leu Phe
   1               5                   10                  15
   Phe Ile Thr Leu Leu His Leu Val Val Ala Leu Asn Asp Val Ala Gly
               20                  25                  30
   Pro Ala Glu Thr Ala Pro Val Ser Leu Leu Pro Arg
           35                  40
<210> 40
   <211> 22
   <212> PRT
   <213> Artificial
<220>
   <223> Secretion signal peptide
<400> 40
   Met Phe Ser Pro Ile Leu Ser Leu Glu Ile Ile Leu Ala Leu Ala Thr
   1               5                   10                  15
   Leu Gln Ser Val Phe Ala
               20
<210> 41
   <211> 22
   <212> PRT
   <213> Artificial
<220>
   <223> Secretion signal peptide
<400> 41
   Met Gln Thr Leu Leu Val Ser Ser Leu Val Val Ser Leu Ala Ala Ala
   1               5                   10                  15
   Leu Pro His Tyr Ile Arg
               20
<210> 42
   <211> 22
   <212> PRT
   <213> Artificial
<220>
   <223> Secretion signal peptide
<400> 42
   Met Ile Phe Leu Lys Leu Ile Lys Ser Ile Val Ile Gly Leu Gly Leu
   1               5                   10                  15
   Val Ser Ala Ile Gln Ala
               20
<210> 43
   <211> 24
   <212> PRT
   <213> Artificial
<220>
   <223> Secretion signal peptide
<400> 43
   Met Lys Trp Val Thr Phe Ile Ser Leu Leu Phe Leu Phe Ser Ser Ala
   1               5                   10                  15
   Tyr Ser Arg Gly Val Phe Arg Arg
               20
<210> 44
   <211> 18
   <212> PRT
   <213> Artificial
<220>
   <223> Secretion signal peptide
<400> 44
   Met Lys Trp Val Thr Phe Ile Ser Leu Leu Phe Leu Phe Ser Ser Ala
   1               5                   10                  15
   Tyr Ser
<210> 45
   <211> 24
   <212> PRT
   <213> Artificial
<220>
   <223> Secretion signal peptide
<400> 45
   Met Ala Leu Trp Met Arg Leu Leu Pro Leu Leu Ala Leu Leu Ala Leu
   1               5                   10                  15
   Trp Gly Pro Asp Pro Ala Ala Ala
               20
<210> 46
   <211> 24
   <212> PRT
   <213> Artificial
<220>
   <223> Secretion signal peptide
<400> 46
   Met Lys Tyr Thr Ser Tyr Ile Leu Ala Phe Gln Leu Cys Ile Val Leu
   1               5                   10                  15
   Gly Ser Leu Gly Cys Asp Leu Pro
               20
<210> 47
   <211> 26
   <212> PRT
   <213> Artificial
<220>
   <223> Secretion signal peptide
<400> 47
   Met Ala Ala Asp Ser Gln Thr Pro Trp Leu Leu Thr Phe Ser Leu Leu
   1               5                   10                  15
   Cys Leu Leu Trp Pro Gln Glu Pro Gly Ala
               20                  25
<210> 48
   <211> 27
   <212> PRT
   <213> Artificial
<220>
   <223> Secretion signal peptide
<400> 48
   Met Lys Lys Asn Arg Met Met Met Met Ile Trp Ser Val Gly Val Val
   1               5                   10                  15
   Trp Met Leu Leu Leu Val Gly Gly Ser Tyr Gly
               20                  25
<210> 49
   <211> 18
   <212> PRT
   <213> Artificial
<220>
   <223> Secretion signal peptide
<400> 49
   Met Gln Lys Leu Ile Ile Phe Ala Leu Val Val Leu Cys Val Gly Ser
   1               5                   10                  15
   Glu Ala
<210> 50
   <211> 18
   <212> PRT
   <213> Artificial
<220>
   <223> Secretion signal peptide
<400> 50
   Met Lys Ala Leu Ile Val Leu Gly Leu Val Leu Leu Ser Val Thr Val
   1               5                   10                  15
   Gln Gly
<210> 51
   <211> 20
   <212> PRT
   <213> Artificial
<220>
   <223> Secretion signal peptide
<400> 51
   Met Val Asp Gly Val Met Ile Leu Pro Val Leu Ile Met Ile Ala Leu
   1               5                   10                  15
   Pro Ser Pro Ser
               20
<210> 52
   <211> 19
   <212> PRT
   <213> Artificial
<220>
   <223> Secretion signal peptide
<400> 52
   Met Gly Ala Ala Ala Lys Leu Ala Phe Ala Val Phe Leu Ile Ser Cys
   1               5                   10                  15
   Ser Ser Gly
<210> 53
   <211> 31
   <212> PRT
   <213> Artificial
<220>
   <223> Secretion signal peptide
<400> 53
   Met Leu Ser Leu Lys Pro Ser Trp Leu Thr Leu Ala Ala Leu Met Tyr
   1               5                   10                  15
   Ala Met Leu Leu Val Val Val Pro Phe Ala Lys Pro Val Arg Ala
               20                  25                  30
<210> 54
   <211> 22
   <212> PRT
   <213> Artificial
<220>
   <223> Secretion signal peptide
<400> 54
   Met Asp Met Arg Val Pro Ala Gln Leu Leu Gly Leu Leu Leu Leu Trp
   1               5                   10                  15
   Leu Pro Gly Ala Lys Cys
               20